# EUROPEAN PATENT APPLICATION

(11) **EP 3 929 296 A1**
(43) Date of publication of application: **29.12.2021**
(21) Application number: 21175651.5
(22) Date of filing: 29.01.2016
(51) Int. Cl.: C12N 15/63, C12N 15/66, C12N 9/22, C12N 15/113, A61K 35/17

(54) **PROTEIN DELIVERY IN PRIMARY HEMATOPOIETIC CELLS**

(30) Priority: 30.01.2015 US 201562110187 P; 25.08.2015 US 201562209711 P
(62) Divisional of application: 16744244.1
(71) Applicant: The Regents of the University of California, Oakland, CA 94607 (US)
(72) Inventor: MARSON, Alexander, San Francisco, CA 94115 (US); DOUDNA, Jennifer, Berkeley, CA 94705 (US); BLUESTONE, Jeffrey, San Francisco, CA 94118 (US); SCHUMANN, Kathrin, San Francisco, CA 94122 (US); LIN, Steven, Nankang Taipei 115 (TW)
(74) Representative: Grünecker Patent- und Rechtsanwälte PartG mbB

(57) **Abstract**

Methods and compositions are provided for highly efficient delivery of Cas9 and Cas9 ribonucleoproteins to cells, including primary hematopoietic cells and primary hematopoietic stem cells.

SEQUENCE LISTING

<110>  The Regents of the University of California

<120>  PROTEIN DELIVERY IN PRIMARY HEMATOPOIETIC CELLS

<130>  EP113726-HVMGpau

<140>  not yet assigned
<141>  2016-01-29

<150>  EP16744244.1
<151>  2016-01-29

<150>  PCT/US2016/015836
<151>  2016-01-29

<150>  US 62/110,187
<151>  2015-01-30

<150>  US 62/209,711
<151>  2015-08-25

<150>  PCT/US2016/015836
<151>  2016-01-29

<160>  110

<170>  PatentIn version 3.5

<210>  1
<211>  5
<212>  PRT
<213>  Artificial Sequence

<220>
<223>  synthetic CXCR4 sequence positions 84-88

<400>  1

Asp Leu Leu Phe Val
1               5


<210>  2
<211>  5
<212>  PRT
<213>  Artificial Sequence

<220>
<223>  synthetic mutated CXCR4 sequence positions 84-88

<400>  2

Glu Ser Leu Asp Pro
1               5


<210>  3
<211>  82
<212>  DNA
<213>  Artificial Sequence

<220>
<223>  synthetic primer SLKS3

<400>  3
taatacgact cactatagga agcgtgatga caaagagggt tttagagcta tgctggaaac      60

agcatagcaa gttaaaataa gg                                               82


<210>  4
<211>  73
<212>  DNA
<213>  Artificial Sequence

<220>
<223>  synthetic primer SLKS1

<400>  4
gcaccgactc ggtgccactt tttcaagttg ataacggact agccttattt taacttgcta      60

tgctgtttcc agc                                                         73


<210>  5
<211>  18
<212>  DNA
<213>  Artificial Sequence

<220>
<223>  synthetic primer T25

<400>  5
taatacgact cactatag                                                    18


<210>  6
<211>  27
<212>  DNA
<213>  Artificial Sequence

<220>
<223>  synthetic primer SLKS1

<400>  6
gcaccgactc ggtgccactt tttcaag                                          27


<210>  7
<211>  82
<212>  DNA
<213>  Artificial Sequence

<220>
<223>  synthetic primer SLKS4

<400>  7
taatacgact cactatagag gagcctcgcc cagctggagt tttagagcta tgctggaaac      60

agcatagcaa gttaaaataa gg                                               82


<210>  8
<211>  192

<212>  DNA
<213>  Artificial Sequence

<220>
<223>  synthetic HDR template sequence

<400>  8
gggcaatgga ttggtcatcc tggtcatggg ttaccagaag aaactgagaa gcatgacgga      60

caagtacagg ctgcacctgt cagtggccga aagcttggat cccatcacgc ttcccttctg     120

ggcagttgat gccgtggcaa actggtactt tgggaacttc ctatgcaagg cagtccatgt     180

catctacaca gt                                                         192


<210>  9
<211>  32
<212>  DNA
<213>  Artificial Sequence

<220>
<223>  synthetic CXCR4 forward primer

<400>  9
agaggagtta gccaagatgt gactttgaaa cc                                    32


<210>  10
<211>  33
<212>  DNA
<213>  Artificial Sequence

<220>
<223>  synthetic CXCR4 reverse primer

<400>  10
ggacaggatg acaataccag gcaggataag gcc                                   33


<210>  11
<211>  25
<212>  DNA
<213>  Artificial Sequence

<220>
<223>  synthetic FOXP3 Target 1 forward primer

<400>  11
ttcaaatact ctgcactgca agccc                                            25


<210>  12
<211>  26
<212>  DNA
<213>  Artificial Sequence

<220>
<223>  synthetic FOXP3 Target 1 reverse primer

<400>  12
catgtacctg tgttcttggt gtgtgt                                           26

<210>  13
<211>  35
<212>  DNA
<213>  Artificial Sequence

<220>
<223>  synthetic FOXP3 Target 2 forward primer

<400>  13
gctgacattt tgactagctt tgtaaagctc tgtgg                                35


<210>  14
<211>  21
<212>  DNA
<213>  Artificial Sequence

<220>
<223>  synthetic FOXP3 Target 2 reverse primer

<400>  14
tctccccgac ctcccaatcc c                                               21


<210>  15
<211>  82
<212>  DNA
<213>  Artificial Sequence

<220>
<223>  synthetic PD-1 sgRNA

<400>  15
taatacgact cactatagcg actggccagg gcgcctgtgt tttagagcta tgctggaaac      60

agcatagcaa gttaaaataa gg                                              82


<210>  16
<211>  123
<212>  DNA
<213>  Artificial Sequence

<220>
<223>  synthetic ultramer sequence CXCR4_1

<400>  16
taatacgact cactatagga agcgtgatga caaagagggt tttagagcta tgctggaaac      60

agcatagcaa gttaaaataa ggctagtccg ttatcaactt gaaaaagtgg caccgagtcg     120

gtg                                                                  123


<210>  17
<211>  123
<212>  DNA
<213>  Artificial Sequence

<220>
<223>  synthetic ultramer sequence CXCR4_2

<400>  17
caccgactcg gtgccacttt ttcaagttga taacggacta gccttatttt aacttgctat      60

gctgtttcca gcatagctct aaaaccctct ttgtcatcac gcttcctata gtgagtcgta     120

tta                                                                   123


<210>  18
<211>  124
<212>  DNA
<213>  Artificial Sequence

<220>
<223>  synthetic ultramer sequence PD-1_1

<400>  18
taatacgact cactatagcg actggccagg gcgcctgtgt tttagagcta tgctggaaac      60

agcatagcaa gttaaaataa ggctagtccg ttatcaactt gaaaaagtgg caccgagtcg     120

gtgc                                                                  124


<210>  19
<211>  124
<212>  DNA
<213>  Artificial Sequence

<220>
<223>  synthetic ultramer sequence PD-1_2

<400>  19
gcaccgactc ggtgccactt tttcaagttg ataacggact agccttattt taacttgcta      60

tgctgtttcc agcatagctc taaaacacag gcgccctggc cagtcgctat agtgagtcgt     120

atta                                                                  124


<210>  20
<211>  191
<212>  DNA
<213>  Artificial Sequence

<220>
<223>  synthetic PD-1 HDR template sequence

<400>  20
aacctgacct gggacagttt cccttccgct cacctccgcc tgagcagtgg agaaggcggc      60

actctggtgg ggctgctcca ggcatgcaga taatgaaagc ttctggccag tcgtctgggc     120

ggtgctacaa ctgggctggc ggccaggatg gttcttaggt aggtggggtc ggcggtcagg     180

tgtcccagag c                                                          191


<210>  21
<211>  192
<212>  DNA
<213>  Artificial Sequence

<220>
<223>  synthetic CXCR4 HDR control template sequence

<400>  21
ttcaaaacta gcgtcagggg ctcgatttac tcgggacttg ctacaacatc gcagtcacgc      60

gcacgatcct tccaggattg gaggtggact tagataaagc ttccgtgtgc accgtataga     120

ttcgttgatg caggctattc ccgtgatccc acgcggaggt gatggagcgt caagcatagc     180

tagcacagat ga                                                         192


<210>  22
<211>  20
<212>  DNA
<213>  Artificial Sequence

<220>
<223>  synthetic PD-1 forward primer

<400>  22
ggggctcatc ccatccttag                                                  20


<210>  23
<211>  20
<212>  DNA
<213>  Artificial Sequence

<220>
<223>  synthetic PD-1 reverse primer

<400>  23
gccacagcag tgagcagaga                                                  20


<210>  24
<211>  76
<212>  DNA
<213>  Artificial Sequence

<220>
<223>  synthetic CXCR4 on-target primer


<220>
<221>  misc_feature
<222>  (34)..(38)
<223>  N is A, C, G or T

<400>  24
acactctttc cctacacgac gctcttccga tctnnnnnct tcctgcccac catctactcc      60

atcatcttct taactg                                                      76


<210>  25
<211>  78
<212>  DNA
<213>  Artificial Sequence

<220>

<223>  synthetic CXCR4 on-target primer

<220>
<221>  misc_feature
<222>  (35)..(39)
<223>  N is A, C, G or T

<400>  25
gtgactggag ttcagacgtg tgctcttccg atctnnnnnc aggtagcggt ccagactgat      60

gaaggccagg atgaggac                                                    78

<210>  26
<211>  79
<212>  DNA
<213>  Artificial Sequence

<220>
<223>  synthetic CXCR4 off-target primer

<220>
<221>  misc_feature
<222>  (35)..(39)
<223>  N is A, C, G or T

<400>  26
gtgactggag ttcagacgtg tgctcttccg atctnnnnnc tggctttata tatatacata      60

gatagacgat atagatagc                                                   79

<210>  27
<211>  73
<212>  DNA
<213>  Artificial Sequence

<220>
<223>  synthetic CXCR4 off-target primer

<220>
<221>  misc_feature
<222>  (34)..(38)
<223>  N is A, C, G or T

<400>  27
acactctttc cctacacgac gctcttccga tctnnnnncc tggtcccagc ccagccccag      60

ctattcagca tcc                                                         73

<210>  28
<211>  80
<212>  DNA
<213>  Artificial Sequence

<220>
<223>  synthetic CXCR4 off-target primer

<220>
<221>  misc_feature
<222>  (35)..(39)
<223>  N is A, C, G or T

<400>  28
gtgactggag ttcagacgtg tgctcttccg atctnnnnna ctctgcactg gtatatcaat      60

acacttgttt ttctcatccc                                                  80


<210>  29
<211>  58
<212>  DNA
<213>  Artificial Sequence

<220>
<223>  synthetic adapter primer

<400>  29
aatgatacgg cgaccaccga gatctacact ctttccctac acgacgctct tccgatct        58


<210>  30
<211>  58
<212>  DNA
<213>  Artificial Sequence

<220>
<223>  synthetic barcode primer

<400>  30
caagcagaag acggcatacg agatgtgact ggagttcaga cgtgtgctct tccgatct        58


<210>  31
<211>  23
<212>  DNA
<213>  Artificial Sequence

<220>
<223>  synthetic primer

<400>  31
cctcctcttt gtcatcacgc ttc                                              23


<210>  32
<211>  23
<212>  DNA
<213>  Artificial Sequence

<220>
<223>  synthetic primer

<400>  32
gaagcgtgat gacaaagagg agg                                              23


<210>  33
<211>  78
<212>  DNA
<213>  Artificial Sequence

<220>
<223>  synthetic reference sequence for Cas9 RNP treated CRCX4-hi

<400>  33
gtttgccacg gcatcaactg cccagaaggg aagcgtgatg acaaagagga ggtcggccac      60

tgacaggtgc agcctgta                                                    78


<210>  34
<211>  78
<212>  DNA
<213>  Artificial Sequence

<220>
<223>  synthetic locus sequence for Cas9 RNP treated CRCX4-hi

<400>  34
gtttgccacg gcatcaactg cccagaaggg aagcgtgatg acaaagagga ggtcggccac      60

tgacaggtgc agcctgta                                                    78


<210>  35
<211>  78
<212>  DNA
<213>  Artificial Sequence

<220>
<223>  synthetic locus sequence for Cas9 RNP treated CRCX4-hi

<400>  35
gtttgccacg gcatcaactg cccagaaggg aagcgtgatg acaaagagga ggtcggccac      60

tgacaggtgc agcctgta                                                    78


<210>  36
<211>  78
<212>  DNA
<213>  Artificial Sequence

<220>
<223>  synthetic locus sequence for Cas9 RNP treated CRCX4-hi

<400>  36
gtttgccacg gcatcaactg cccagaaggg aagcgtgatg acaaagagga ggtcggccac      60

tgacaggtgc agcctgta                                                    78


<210>  37
<211>  78
<212>  DNA
<213>  Artificial Sequence

<220>
<223>  synthetic locus sequence for Cas9 RNP treated CRCX4-hi

<400>  37
gtttgccatg gcatcaactg cccagaaggg aagcgtgatg acaaagagga ggtcggccac      60

tgacaggtgc agcctgta                                                    78

<210>  38
<211>  78
<212>  DNA
<213>  Artificial Sequence

<220>
<223>  synthetic locus sequence for Cas9 RNP treated CRCX4-hi

<400>  38
gtttgccacg gcatcaactg cccagaaggg aagcgtgatg acaaagagga ggtcggccac      60

tgacaggtgc agcctgta                                                    78

<210>  39
<211>  72
<212>  DNA
<213>  Artificial Sequence

<220>
<223>  synthetic locus sequence for Cas9 RNP treated CRCX4-hi

<400>  39
gtttgccacg gcatcaactg cccagaaggg aagcgtgatg acaaagtcgg ccactgacag      60

gtgcagcctg ta                                                          72

<210>  40
<211>  78
<212>  DNA
<213>  Artificial Sequence

<220>
<223>  synthetic locus sequence for Cas9 RNP treated CRCX4-hi

<400>  40
gtttgccacg gcatcaactg cccagaaggg aagcgtgatg acaaagagga ggtcggccac      60

tgacaggtgc agcctgta                                                    78

<210>  41
<211>  66
<212>  DNA
<213>  Artificial Sequence

<220>
<223>  synthetic locus sequence for Cas9 RNP treated CRCX4-hi

<400>  41
gtttgccacg gcatcaactg cccagaaggg aagcgggagg tcggccactg acaggtgcag      60

cctgta                                                                 66

<210>  42
<211>  75
<212>  DNA
<213>  Artificial Sequence

<220>
<223>  synthetic locus sequence for Cas9 RNP treated CRCX4-hi

<400>  42
gtttgccacg gcatcaactg cccagaaggg aagcgtgatg acaaggaggt cggccactga      60

caggtgcagc ctgta                                                       75


<210>  43
<211>  78
<212>  DNA
<213>  Artificial Sequence

<220>
<223>  synthetic locus sequence for Cas9 RNP treated CRCX4-hi

<400>  43
gtttgccacg gcatcaactg cccagaaggg aagcgtgatg acaaagagga ggtcggccac      60

tgacaggtgc agcctgta                                                    78


<210>  44
<211>  78
<212>  DNA
<213>  Artificial Sequence

<220>
<223>  synthetic reference sequence for Cas9 RNP treated CRCX4-lo

<400>  44
gtttgccacg gcatcaactg cccagaaggg aagcgtgatg acaaagagga ggtcggccac      60

tgacaggtgc agcctgta                                                    78


<210>  45
<211>  78
<212>  DNA
<213>  Artificial Sequence

<220>
<223>  synthetic locus sequence for Cas9 RNP treated CRCX4-lo

<400>  45
gtttgccacg gcatcaactg cccagaaggg aagcgtgatg acaaagagga ggtcggccac      60

tgacaggtgc agcctgta                                                    78


<210>  46
<211>  78
<212>  DNA
<213>  Artificial Sequence

<220>
<223>  synthetic locus sequence for Cas9 RNP treated CRCX4-lo

<400>  46
gtggcaaact ggtactttgg gaacttccta tgcaaggcag tccatgtcat ctacacagtc      60

aacctctaca gcagtgtc                                                    78


<210>  47
<211>  76
<212>  DNA
<213>  Artificial Sequence

<220>
<223>  synthetic locus sequence for Cas9 RNP treated CRCX4-lo

<400>  47
gtttgccacg gcatcaactg cccagaaggg aagcgtgatg acaaaggagg tcggccactg      60

acaggtgcag cctgta                                                      76


<210>  48
<211>  78
<212>  DNA
<213>  Artificial Sequence

<220>
<223>  synthetic locus sequence for Cas9 RNP treated CRCX4-lo

<400>  48
gtttgccacg gcatcaactg cccagaaggg aagcgtgacg ctgacaggtg ccgcctgtac      60

ttgtccgtca tgcgtctc                                                    78


<210>  49
<211>  78
<212>  DNA
<213>  Artificial Sequence

<220>
<223>  synthetic locus sequence for Cas9 RNP treated CRCX4-lo

<400>  49
gtttgccacg gcatcaactg cccagaagga agtcgtgctc tgacaggagg aggccggcct      60

tggacatgtg gcttctga                                                    78


<210>  50
<211>  33
<212>  DNA
<213>  Artificial Sequence

<220>
<223>  synthetic locus sequence for Cas9 RNP treated CRCX4-lo

<400>  50
gtttgccacg gccactgaca ggtgcagcct gta                                   33


<210>  51
<211>  71
<212>  DNA
<213>  Artificial Sequence

<220>

<223>  synthetic locus sequence for Cas9 RNP treated CRCX4-lo

<400>  51
gtttgccacg gcatcaactg cccagaaggg aagcgtgata ggaggtcggc cactgacagg      60

tgcagcctgt a                                                           71


<210>  52
<211>  78
<212>  DNA
<213>  Artificial Sequence

<220>
<223>  synthetic locus sequence for Cas9 RNP treated CRCX4-lo

<400>  52
gtttgccacg gcatcaactg cccagaaggg aagcgtgatg acaaagagga ggtcggccac      60

tgacaggtgc agcctgta                                                    78


<210>  53
<211>  76
<212>  DNA
<213>  Artificial Sequence

<220>
<223>  synthetic locus sequence for Cas9 RNP treated CRCX4-lo

<400>  53
gtttgccacg gcatcaactg cccagaaggg aagcgtgatg acaaaggacg tccgccgctg      60

agaggtgcag gctgta                                                      76


<210>  54
<211>  77
<212>  DNA
<213>  Artificial Sequence

<220>
<223>  synthetic locus sequence for Cas9 RNP treated CRCX4-lo

<400>  54
gtttgccacg gcatcaactg cccagaaggg aagcgtgatg acaaaaggag gtcggccact      60

gacaggtgca gcctgta                                                     77


<210>  55
<211>  78
<212>  DNA
<213>  Artificial Sequence

<220>
<223>  synthetic reference sequence for CRCX4-lo control

<400>  55
gtttgccacg gcatcaactg cccagaaggg aagcgtgatg acaaagagga ggtcggccac      60

tgacaggtgc agcctgta                                                    78

<210>  56
<211>  78
<212>  DNA
<213>  Artificial Sequence

<220>
<223>  synthetic locus sequence for CRCX4-lo control

<400>  56
gtttgccacg gcatcaactg cccagaaggg aagcgtgatg acaaagagga ggtcggccac      60

tgacaggtgc agcctgta                                                    78


<210>  57
<211>  78
<212>  DNA
<213>  Artificial Sequence

<220>
<223>  synthetic locus sequence for CRCX4-lo control

<400>  57
gtttgccacg gcatcaactg cccagaaggg aagcgtgatg acaaagagga ggtcggccac      60

tgacaggtgc agcctgta                                                    78


<210>  58
<211>  78
<212>  DNA
<213>  Artificial Sequence

<220>
<223>  synthetic locus sequence for CRCX4-lo control

<400>  58
gtttgccacg gcatcaactg cccagaaggg aagcgtgatg acaaagagga ggtcggccac      60

tgacaggtgc agcctgta                                                    78


<210>  59
<211>  78
<212>  DNA
<213>  Artificial Sequence

<220>
<223>  synthetic locus sequence for CRCX4-lo control

<400>  59
gtttgccacg gcatcaactg cccagaaggg aagcgtgatg acaaagagga ggtcggccac      60

tgacaggtgc agcctgta                                                    78


<210>  60
<211>  78
<212>  DNA
<213>  Artificial Sequence

<220>

<223>  synthetic locus sequence for CRCX4-lo control

<400>  60
gtttgccacg gcatcaactg cccagaaggg aagcgtgatg acaaagagga ggtcggccac      60

tgacaggtgc agcctgta                                                    78


<210>  61
<211>  78
<212>  DNA
<213>  Artificial Sequence

<220>
<223>  synthetic locus sequence for CRCX4-lo control

<400>  61
gtttgccacg gcatcaactg cccagaaggg aagcgtgatg acaaagagga ggtcggccac      60

tgacaggtgc agcctgta                                                    78


<210>  62
<211>  78
<212>  DNA
<213>  Artificial Sequence

<220>
<223>  synthetic locus sequence for CRCX4-lo control

<400>  62
gtttgccacg gcatcaactg cccagaaggg aagcgtgatg acaaagagga ggtcggccac      60

tgacaggtgc agcctgta                                                    78


<210>  63
<211>  78
<212>  DNA
<213>  Artificial Sequence

<220>
<223>  synthetic locus sequence for CRCX4-lo control

<400>  63
gtttgccacg gcatcaactg cccagaaggg aagcgtgatg acaaagagga ggtcggccac      60

tgacaggtgc agcctgta                                                    78


<210>  64
<211>  78
<212>  DNA
<213>  Artificial Sequence

<220>
<223>  synthetic locus sequence for CRCX4-lo control

<400>  64
gtttgccacg gcatcaactg cccagaaggg aagcgtgatg acaaagagga ggtcggccac      60

tgacaggtgc agcctgta                                                    78

<210>  65
<211>  78
<212>  DNA
<213>  Artificial Sequence

<220>
<223>  synthetic locus sequence for CRCX4-lo control

<400>  65
gtttgccacg gcatcaactg cccagaaggg aagcgtgatg acaaagagga ggtcggccac      60

tgacaggtgc agcctgta                                                    78


<210>  66
<211>  12
<212>  DNA
<213>  Artificial Sequence

<220>
<223>  synthetic donor sequence

<400>  66
aagcttggat cc                                                          12


<210>  67
<211>  23
<212>  DNA
<213>  Artificial Sequence

<220>
<223>  synthetic FOXP3 Target 2 primer

<400>  67
tcatggctgg gctctccagg ggg                                              23


<210>  68
<211>  23
<212>  DNA
<213>  Artificial Sequence

<220>
<223>  synthetic FOXP3 Target 2 primer

<400>  68
ccccctggag agcccagcca tga                                              23


<210>  69
<211>  23
<212>  DNA
<213>  Artificial Sequence

<220>
<223>  synthetic FOXP3 Target 1 primer

<400>  69
ccctccagct gggcgaggct cct                                              23

<210>  70
<211>  23
<212>  DNA
<213>  Artificial Sequence

<220>
<223>  synthetic FOXP3 Target 1 primer

<400>  70
aggagcctcg cccagctgga ggg                                              23


<210>  71
<211>  10
<212>  DNA
<213>  Artificial Sequence

<220>
<223>  synthetic HDR template sequence

<400>  71
tagtaagctt                                                             10


<210>  72
<211>  53
<212>  DNA
<213>  Artificial Sequence

<220>
<223>  synthetic sgRNA target (3) sequence for PD-1 exon 2

<400>  72
ccacgctcat gtggaagtca cgcccgttgg gcagttgtgt gacacggaag cgg             53


<210>  73
<211>  52
<212>  DNA
<213>  Artificial Sequence

<220>
<223>  synthetic sgRNA target (4) sequence for PD-1 exon 2

<400>  73
cgcttccgtg tcacacaact gcccaacggg cgtgacttcc acatgagcgt gg              52


<210>  74
<211>  32
<212>  DNA
<213>  Artificial Sequence

<220>
<223>  synthetic sgRNA target (2) sequence for PD-1 exon 1

<400>  74
ccgcccagac gactggccag ggcgcctgtg gg                                    32


<210>  75
<211>  32
<212>  DNA

<213>  Artificial Sequence

<220>
<223>  synthetic sgRNA target (1) sequence for PD-1 exon 1

<400>  75
cccacaggcg ccctggccag tcgtctgggc gg                                    32


<210>  76
<211>  23
<212>  DNA
<213>  Artificial Sequence

<220>
<223>  synthetic primer

<400>  76
cctcctcttt gtcatcacgc ttc                                              23


<210>  77
<211>  70
<212>  DNA
<213>  Artificial Sequence

<220>
<223>  synthetic reference sequence for Cas9 RNP treated CRCX4-hi

<400>  77
tgccacggca tcaactgccc agaagggaag cgtgatgaca aagaggaggt cggccactga      60

caggtgcagc                                                             70


<210>  78
<211>  70
<212>  DNA
<213>  Artificial Sequence

<220>
<223>  synthetic locus sequence for Cas9 RNP treated CRCX4-hi

<400>  78
tgccacggca tcaactgccc agaagggaag cgtgatgaca aagaggaggt cggccactga      60

caggtgcagc                                                             70


<210>  79
<211>  70
<212>  DNA
<213>  Artificial Sequence

<220>
<223>  synthetic locus sequence for Cas9 RNP treated CRCX4-hi

<400>  79
tgccacggca tcaactgccc agaagggaag cgtgatgaca aagaggaggt cggccactga      60

caggtgcagc                                                             70

<210>  80
<211>  70
<212>  DNA
<213>  Artificial Sequence

<220>
<223>  synthetic locus sequence for Cas9 RNP treated CRCX4-hi

<400>  80
tgccacggca tcaactgccc agaagggaag cgtgatgaca aagaggaggt cggccactga      60

caggtgcagc                                                              70


<210>  81
<211>  70
<212>  DNA
<213>  Artificial Sequence

<220>
<223>  synthetic locus sequence for Cas9 RNP treated CRCX4-hi

<400>  81
tgccacggca tcaactgccc agaagggaag cgtgatgaca aagaggaggt cggccactga      60

caggtgcagc                                                              70


<210>  82
<211>  70
<212>  DNA
<213>  Artificial Sequence

<220>
<223>  synthetic locus sequence for Cas9 RNP treated CRCX4-hi

<400>  82
tgccatggca tcaactgccc agaagggaag cgtgatgaca aagaggaggt cggccactga      60

caggtgcagc                                                              70


<210>  83
<211>  70
<212>  DNA
<213>  Artificial Sequence

<220>
<223>  synthetic locus sequence for Cas9 RNP treated CRCX4-hi

<400>  83
tgccacggca tcaactgccc agaagggaag cgtgatgaca aagaggaggt cggccactga      60

caggtgcagc                                                              70


<210>  84
<211>  64
<212>  DNA
<213>  Artificial Sequence

<220>
<223>  synthetic locus sequence for Cas9 RNP treated CRCX4-hi

<400>  84
tgccacggca tcaactgccc agaagggaag cgtgatgaca aagtcggcca ctgacaggtg      60

cagc                                                                    64


<210>  85
<211>  70
<212>  DNA
<213>  Artificial Sequence

<220>
<223>  synthetic locus sequence for Cas9 RNP treated CRCX4-hi

<400>  85
tgccacggca tcaactgccc agaagggaag cgtgatgaca aagaggaggt cggccactga      60

caggtgcagc                                                              70


<210>  86
<211>  67
<212>  DNA
<213>  Artificial Sequence

<220>
<223>  synthetic locus sequence for Cas9 RNP treated CRCX4-hi

<400>  86
tgccacggca tcaactgccc agaagggaag cgtgatgaca aggaggtcgg ccactgacag      60

gtgcagc                                                                 67


<210>  87
<211>  58
<212>  DNA
<213>  Artificial Sequence

<220>
<223>  synthetic locus sequence for Cas9 RNP treated CRCX4-hi

<400>  87
tgccacggca tcaactgccc agaagggaag cgggaggtcg gccactgaca ggtgcagc        58


<210>  88
<211>  70
<212>  DNA
<213>  Artificial Sequence

<220>
<223>  synthetic reference sequence for Cas9 RNP treated CRCX4-lo

<400>  88
tgccacggca tcaactgccc agaagggaag cgtgatgaca aagaggaggt cggccactga      60

caggtgcagc                                                              70


<210>  89
<211>  70

<212>  DNA
<213>  Artificial Sequence

<220>
<223>  synthetic locus sequence for Cas9 RNP treated CRCX4-lo

<400>  89
tgccacggca tcaactgccc agaagggaag cgtgatgaca aagaggaggt cggccactga      60

caggtgcagc                                                              70


<210>  90
<211>  69
<212>  DNA
<213>  Artificial Sequence

<220>
<223>  synthetic locus sequence for Cas9 RNP treated CRCX4-lo

<400>  90
tgccacggca tcaactgccc agaagggaag cgtgatgaca aaaggaggtc ggccactgac      60

aggtgcagc                                                               69


<210>  91
<211>  68
<212>  DNA
<213>  Artificial Sequence

<220>
<223>  synthetic locus sequence for Cas9 RNP treated CRCX4-lo

<400>  91
tgccacggca tcaactgccc agaagggaag cgtgatgaca aaggaggtcg gccactgaca      60

ggtgcagc                                                                68


<210>  92
<211>  63
<212>  DNA
<213>  Artificial Sequence

<220>
<223>  synthetic locus sequence for Cas9 RNP treated CRCX4-lo

<400>  92
tgccacggca tcaactgccc agaagggaag cgtgatagga ggtcggccac tgacaggtgc      60

agc                                                                     63


<210>  93
<211>  25
<212>  DNA
<213>  Artificial Sequence

<220>
<223>  synthetic locus sequence for Cas9 RNP treated CRCX4-lo

<400>  93

tgccacggcc actgacaggt gcagc                                              25

<210>  94
<211>  37
<212>  DNA
<213>  Artificial Sequence

<220>
<223>  synthetic locus sequence for Cas9 RNP treated CRCX4-lo

<400>  94
tgccacggca tcaactgccc agaagggaag cgtgatg                                37

<210>  95
<211>  70
<212>  DNA
<213>  Artificial Sequence

<220>
<223>  synthetic reference sequence for CRCX4-lo control

<400>  95
tgccacggca tcaactgccc agaagggaag cgtgatgaca aagaggaggt cggccactga      60

caggtgcagc                                                              70

<210>  96
<211>  70
<212>  DNA
<213>  Artificial Sequence

<220>
<223>  synthetic locus sequence for CRCX4-lo control

<400>  96
tgccacggca tcaactgccc agaagggaag cgtgatgaca aagaggaggt cggccactga      60

caggtgcagc                                                              70

<210>  97
<211>  70
<212>  DNA
<213>  Artificial Sequence

<220>
<223>  synthetic locus sequence for CRCX4-lo control

<400>  97
tgccacggca tcaactgccc agaagggaag cgtgatgaca aagaggaggt cggccactga      60

caggtgcagc                                                              70

<210>  98
<211>  70
<212>  DNA
<213>  Artificial Sequence

<220>

<223>  synthetic locus sequence for CRCX4-lo control

<400>  98
tgccacggca tcaactgccc agaagggaag cgtgatgaca aagaggaggt cggccactga      60

caggtgcagc                                                              70


<210>  99
<211>  70
<212>  DNA
<213>  Artificial Sequence

<220>
<223>  synthetic locus sequence for CRCX4-lo control

<400>  99
tgccacggca tcaactgccc agaagggaag cgtgatgaca aagaggaggt cggccactga      60

caggtgcagc                                                              70


<210>  100
<211>  70
<212>  DNA
<213>  Artificial Sequence

<220>
<223>  synthetic locus sequence for CRCX4-lo control

<400>  100
tgccacggca tcaactgccc agaagggaag cgtgatgaca aagaggaggt cggccactga      60

caggtgcagc                                                              70


<210>  101
<211>  70
<212>  DNA
<213>  Artificial Sequence

<220>
<223>  synthetic locus sequence for CRCX4-lo control

<400>  101
tgccacggca tcaactgccc agaagggaag cgtgatgaca aagaggaggt cggccactga      60

caggtgcagc                                                              70


<210>  102
<211>  70
<212>  DNA
<213>  Artificial Sequence

<220>
<223>  synthetic locus sequence for CRCX4-lo control

<400>  102
tgccacggca tcaactgccc agaagggaag cgtgatgaca aagaggaggt cggccactga      60

caggtgcagc                                                              70

<210>  103
<211>  70
<212>  DNA
<213>  Artificial Sequence

<220>
<223>  synthetic locus sequence for CRCX4-lo control

<400>  103
tgccacggca tcaactgccc agaagggaag cgtgatgaca aagaggaggt cggccactga      60

caggtgcagc                                                              70


<210>  104
<211>  70
<212>  DNA
<213>  Artificial Sequence

<220>
<223>  synthetic locus sequence for CRCX4-lo control

<400>  104
tgccacggca tcaactgccc agaagggaag cgtgatgaca aagaggaggt cggccactga      60

caggtgcagc                                                              70


<210>  105
<211>  102
<212>  DNA
<213>  Artificial Sequence

<220>
<223>  synthetic Cas9 sequence

<400>  105
caaagtacca gtttgccacg gcatcaactg cccagaaggg gaagcgtgat gacaaagagg      60

aggtcggcca ctgacaggtg cagcctgtac ttgtccgtca tg                        102


<210>  106
<211>  101
<212>  DNA
<213>  Artificial Sequence

<220>
<223>  synthetic Cas9 sequence

<400>  106
caaagtacca gtttgccacg gcatcaactg cccagaaggg aagcgtgatg agaaagagga      60

ggtcggccac tgacaggtgc agcctgtact tgtcctgcat g                         101


<210>  107
<211>  39
<212>  DNA
<213>  Artificial Sequence

<220>

<223>  synthetic Cas9 sequence

<400>  107
caaagtacca gtttgccacg gcatcaactg cccagaagg                              39


<210>  108
<211>  23
<212>  DNA
<213>  Artificial Sequence

<220>
<223>  synthetic target sequence

<400>  108
cgactggcca gggcgcctgt ggg                                               23


<210>  109
<211>  23
<212>  DNA
<213>  Artificial Sequence

<220>
<223>  synthetic target sequence

<400>  109
cccacaggcg ccctggccag tcg                                               23


<210>  110
<211>  11
<212>  DNA
<213>  Artificial Sequence

<220>
<223>  synthetic PD-1 HDR template sequence

<400>  110
ttcgaaagta a                                                            11

## Description

### CROSS-REFERENCES TO RELATED APPLICATIONS

This application claims priority to U.S. Provisional Application Nos. 62/110,187, filed January 30, 2015, and 62/209,711, filed August 25, 2015, the contents of each of which are hereby incorporated by reference in the entirety for all purposes.

### BACKGROUND OF THE INVENTION

Methods, compositions, reaction mixtures, kits, and devices, for precise and efficient manipulation primary cells hold great promise for development of cell-based therapeutics, as well as basic research into the function of various cells, tissues, organs, and systems in the body. For example, recent advances in the generation and use of primary antigen-specific T cells holds great promise for immunotherapy against cancer and infectious diseases. As another example, the ability to precisely target regulatory genes in primary cells can be used to study the phenotypic results of such modulation.

### BRIEF SUMMARY OF THE INVENTION

In one aspect, the present invention provides a method of editing the genome of a cell, wherein the cell is a primary hematopoietic cell or a primary hematopoietic stem cell, the method comprising: a) providing a reaction mixture comprising a Cas9 nuclease domain *(e.g.,* a Cas9 apo protein) and the cell; and b) introducing the Cas9 nuclease domain inside the cell, wherein the Cas9 nuclease domain forms a complex with a guide RNA inside the cell. In some embodiments, the guide RNA inside the cell is encoded by a guide RNA gene inside the cell, wherein the guide RNA gene comprises DNA. In some embodiments, the cell does not contain a nucleic acid encoding the Cas9 nuclease domain. In some embodiments, the efficiency of Cas9 delivery is at least about 20% or 30%. In some embodiments, the primary hematopoietic cell or a primary hematopoietic stem cell is modified to express a heterologous protein either before, during, or after the genome of the cell is edited as described above or elsewhere herein. In some embodiments, the heterologous protein is encoded by a viral (e.g., a lentiviral) vector. In some embodiments, the heterologous protein is a chimeric antigen receptor (CAR) protein or a heterologous T-cell Receptor (TCR), including but not limited to a rearranged TCR.

In some embodiments, the present invention provides a method of editing the genome of a cell, wherein the cell is a primary hematopoietic cell or a primary hematopoietic stem cell, the method comprising: a) providing a reaction mixture comprising a Cas9 ribonucleoprotein complex and the cell, wherein the Cas9 ribonucleoprotein complex comprises a Cas9 nuclease domain and a guide RNA, wherein the guide RNA specifically hybridizes to a target region of the genome of the cell; and b) introducing the Cas9 ribonucleoprotein complex inside the cell. In some embodiments the method provides an efficiency of genome editing of at least about 20%. In some embodiments, the cell does not contain a nucleic acid encoding the Cas9 and/or a DNA nucleic acid encoding a guide RNA.

In some embodiments, prior to the providing of a) the cell is not immortalized or transformed. In some cases, after the introducing of b) the cell is not immortalized or transformed. In some embodiments, the cell has not been passaged prior to the providing of a). In some cases, prior to the providing of a), the cell has been directly isolated from a host organism or tissue and cultured. In some cases, prior to the providing of a), the cell has been directly isolated from a host organism or tissue and has not been cultured.

In some embodiments, the introducing comprises electroporation. In some embodiments, the introducing comprises: coating a nanowire or nanotube with the Cas9 ribonucleoprotein complex or Cas9 apo protein; contacting the cell with the nanowire or nanotube coated with the Cas9 ribonucleoprotein complex or Cas9 apo protein; and piercing a cell membrane of the cell with the nanowire or nanotube coated with the Cas9 ribonucleoprotein complex or Cas9 apo protein. In some embodiments, the introducing comprises: forcing the reaction mixture through a cell deforming constriction that is smaller than the diameter of the cell, wherein the forcing introduces transient pores into a cell membrane of the cell; and allowing the Cas9 ribonucleoprotein complex or Cas9 apo protein to enter the cell through the transient pores.

In some embodiments, the Cas9 ribonucleoprotein complex or Cas9 apo protein comprises a ligand for an extracellular receptor on the cell, and the introducing comprises receptor mediated internalization of the Cas9 ribonucleoprotein complex or Cas9 apo protein. In some embodiments, the Cas9 ribonucleoprotein complex or Cas9 apo protein comprises a cell penetrating peptide, and the introducing comprises contacting the cell penetrating peptide to the cell.

In some cases, the electroporation comprises positioning the reaction mixture into a chamber between a cathode and an anode, and applying a voltage potential between the cathode and the anode of from about 20 kV/m to about 100 kV/m. In some cases, the voltage potential is applied as a pulse having a length of from about 5 ms to about 100 ms. In some cases, the method further comprises repeating the application of the voltage potential pulse from 2 to 10 times. In some cases, the chamber is a hollow member having a longitudinal length and a horizontal cross sectional area; the chamber comprises a first and second distal end separated by the longitudinal length; and the chamber has: a first electrode at the first distal end; and a reservoir containing an electrolytic solution in fluid communication with the second distal end of the chamber, said reservoir having a second electrode. In some cases, the chamber has a ratio of longitudinal length to horizontal cross-sectional area in the range of 50 to 10,000.

In some embodiments, the Cas9 ribonucleoprotein complex or the Cas9 apo protein in the reaction mixture is at a concentration of from about 0.25 µM to about 5 µM. In some embodiments, the Cas9 ribonucleoprotein complex or the Cas9 apo protein in the reaction mixture is at a concentration of from about 0.9 µM to about 1.8 µM. In some embodiments, the reaction mixture contains from about 1 x 10⁵ to about 4 x 10⁵ primary hematopoietic cells or primary hematopoietic stem cells or from about 0.9 x 10⁴ to about 3.6 x 10⁴ primary hematopoietic cells or primary hematopoietic stem cells per µL. In some embodiments, the reaction mixture contains from about 2 x 10⁵ to about 2.5 x 10⁵ primary hematopoietic cells or primary hematopoietic stem cells or 1.8 x 10⁴ to about 2.2 x 10⁴ primary hematopoietic cells or primary hematopoietic stem cells per µL. In some embodiments, the cell is a primary hematopoietic cell.

In some cases, the primary hematopoietic cell is an immune cell. In some cases, the immune cell is a T cell. In some cases, the T cell is a regulatory T cell, an effector T cell, or a naive T cell. In some cases, the regulatory T cell, effector T cell, or naïve T cell is a CD4⁺ T cell. In some cases, the T cell is a CD4⁺CD25^{hi}CD127^{lo} regulatory T cell. In some cases, the T cell is a FOXP3⁺ T cell. In some cases, the T cell is a CD4⁺CD25^{lo}CD127^{hi} effector T cell. In some cases, the T cell is a CD4⁺CD25^{lo}CD127^{hi}CD45RA^{hi}CD45RO⁻ naive T cell. In some cases, the T cell is a CD8⁺ T cell. In some cases, the T cell is a CD4⁺CD8⁺ T cell. In some cases, prior to the providing of a), the T cell is pre-activated. In some cases, prior to the providing of a), the T cell is unstimulated. In some cases, the T cell comprises a recombinant antigen receptor.

In some embodiments, the reaction mixture further comprises a double or single-stranded oligonucleotide DNA template, and wherein the method comprises introducing the double or single-stranded oligonucleotide DNA template inside the cell. In some embodiments, the double or single-stranded oligonucleotide DNA template is at a concentration of from about 9 µM to about 180 µM. In some cases, the double or single-stranded oligonucleotide DNA template is at a concentration of about 45 µM. In some cases, the method provides an efficiency of primary hematopoietic cell (e.g., stimulated or unstimulated T cell) or primary hematopoietic stem cell genome editing (e.g., by nick repair, non-homologous end joining repair, or homology directed repair of Cas9 single or double-stranded cleavage sites) of at least about 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, or 80%..

In some cases, the method provides an efficiency of primary hematopoietic cell (e.g., stimulated or unstimulated T cell) or primary hematopoietic stem cell genome editing *(e.g.,* by nick repair, non-homologous end joining repair, or homology directed repair of Cas9 single or double-stranded cleavage sites) of from about 20% to about 80%, from about 25%, to about 70%, from about 30% to about 75%, from about 40% to about 75%, from about 50% to about 70%, from about 20% to about 70%, from about 25% to about 65%, from about 30% to about 60%, or from about 35% to about 55%.

In some cases, the method provides an efficiency of primary hematopoietic cell *(e.g.,* stimulated or unstimulated T cell) or primary hematopoietic stem cell template directed genome editing of at least about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, or 75%.

In some cases, the method provides an efficiency of primary hematopoietic cell *(e.g.,* stimulated or unstimulated T cell) or primary hematopoietic stem cell template directed genome editing of from about 5% to about 30%, from about 7% to about 25%, from about 10% to about 20%, from about 5%, to about 25%, from about 10% to about 25%, from about 5% to about 20%, from about 5% to about 15%, or from about 10% to about 15. In some cases, the single stranded oligonucleotide DNA template encodes a recombinant antigen receptor, a portion thereof, or a component thereof.

In some embodiments, the cell is a T cell, and the method further comprises: c) after the introducing of b), transferring the reaction mixture to a culture medium containing a CD3 agonist and a CD28 agonist and culturing the cells. In some cases, the CD3 agonist or the CD28 agonist are immobilized on a solid surface, or the CD3 agonist and the CD28 agonist are immobilized on a solid surface (e.g., immobilized on a bead or separate beads or on a surface of a culture plate or well). In some cases, the CD3 agonist is an anti-CD3 antibody. In some cases, the CD28 agonist is an anti-CD28 antibody. In some cases, the method further comprises: c) after the culturing of c), transferring the reaction mixture to a culture medium that does not contain a CD3 agonist or a CD28 agonist and culturing the cells.

In some cases, the anti-CD3 antibody (e.g., immobilized or soluble) is at a concentration of about 1, 2, 3, 4, 5, 6, 7, 8, 9, 10, 11, 12, 13, 14, 15, 16, 17, 18, 19, 20, 21, 22, 23, 24, or 25 µg/mL. In some cases, the anti-CD3 antibody (e.g., immobilized or soluble) is at a concentration of from about 0.5 to about 25 µg/mL, from about 1 to about 20 µg/mL, from about 2 to about 15 µg/mL, from about 5 to about 15 µg/mL, or from about 5 to about 10 µg/mL. In some cases, the anti-CD28 antibody (e.g., immobilized or soluble) is at a concentration of about 1, 2, 3, 4, 5, 6, 7, 8, 9, 10, 11, 12, 13, 14, 15, 16, 17, 18, 19, 20, 21, 22, 23, 24, or 25 µg/mL. In some cases, the anti-CD28 antibody (e.g., immobilized or soluble) is at a concentration of from about 0.5 to about 15 µg/mL, from about 1 to about 15 µg/mL, from about 2 to about 10 µg/mL, from about I to about 7.5 µg/mL, or from about 2 to about 5 µg/mL.

In some embodiments, the Cas9 ribonucleoprotein complex or Cas9 apo protein comprises a Cas9 nuclease. In some embodiments, the Cas9 ribonucleoprotein complex or Cas9 apo protein comprises a Cas9 nickase. In some embodiments, the Cas9 ribonucleoprotein complex or Cas9 apo protein comprises a Cas9 nuclease domain fused to a restriction endonuclease or nickase. In some embodiments, the Cas9 ribonucleoprotein complex or Cas9 apo protein comprises a Cas9 nuclease domain fused to a transcriptional modulator or a chromatin modifier.

In some embodiments, the reaction mixture comprises at least two structurally different Cas9 ribonucleoprotein complexes or at least two structurally different Cas9 apo proteins. In some cases, the at least two structurally different Cas9 ribonucleoprotein complexes contain structurally different sgRNAs. In some cases, the at least two structurally different Cas9 ribonucleoprotein complexes or at least two different Cas9 apo proteins contain structurally different Cas9 domains.

In another aspect, the present invention provides a plurality of primary hematopoietic cells or primary hematopoietic stem cells, wherein the plurality of cells do not contain a nucleic acid encoding Cas9 and/or a DNA nucleic acid encoding a guide RNA, and wherein at least 20% of the plurality of cells contains a Cas9 ribonucleoprotein complex. In some embodiments, at least 30% of the plurality of cells contains a Cas9 ribonucleoprotein complex. In some embodiments, at least 20% of the plurality of cells contains a Cas9 ribonucleoprotein complex and a single stranded oligonucleotide DNA template. In some embodiments, the plurality of cells have not enriched for the presence of the Cas9 ribonucleoprotein complex. In some embodiments, at least 20% or 30% of the plurality of cells contains a double stranded break, or an NHEJ or HDR repaired double stranded break at a target genomic region. In some embodiments, the primary hematopoietic cells or a primary hematopoietic stem cells are modified to express a heterologous protein (e.g., a chimeric antigen receptor (CAR)) either before, during, or after the genome of the cell is edited as described above or elsewhere herein. The heterologous protein can be encodes by a viral vector (e.g., a lentiviral vector) introduced into the cells.

### DEFINITIONS

As used in this specification and the appended claims, the singular forms "a," "an," and "the" include plural reference unless the context clearly dictates otherwise.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The term "gene" can refer to the segment of DNA involved in producing or encoding a polypeptide chain. It may include regions preceding and following the coding region (leader and trailer) as well as intervening sequences (introns) between individual coding segments (exons). Alternatively, the term "gene" can refer to the segment of DNA involved in producing or encoding a non-translated RNA, such as an rRNA, tRNA, guide RNA *(e.g.,* a small guide RNA), or micro RNA.

A "promoter" is defined as an array of nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription.

An "expression cassette" is a nucleic acid construct, generated recombinantly or synthetically, with a series of specified nucleic acid elements that permit transcription of a particular polynucleotide sequence in a host cell. An expression cassette may be part of a plasmid, viral genome, or nucleic acid fragment. Typically, an expression cassette includes a polynucleotide to be transcribed, operably linked to a promoter.

A "reporter gene" encodes proteins that are readily detectable due to their biochemical characteristics, such as enzymatic activity or chemifluorescent features. One specific example of such a reporter is green fluorescent protein. Fluorescence generated from this protein can be detected with various commercially-available fluorescent detection systems. Other reporters can be detected by staining. The reporter can also be an enzyme that generates a detectable signal when contacted with an appropriate substrate. The reporter can be an enzyme that catalyzes the formation of a detectable product. Suitable enzymes include, but are not limited to, proteases, nucleases, lipases, phosphatases and hydrolases. The reporter can encode an enzyme whose substrates are substantially impermeable to eukaryotic plasma membranes, thus making it possible to tightly control signal formation. Specific examples of suitable reporter genes that encode enzymes include, but are not limited to, CAT (chloramphenicol acetyl transferase; Alton and Vapnek (1979) Nature 282: 864-869); luciferase (lux); β-galactosidase; LacZ; β.-glucuronidase; and alkaline phosphatase (Toh, et al. (1980) Eur. J. Biochem. 182: 231-238; and Hall et al. (1983) J. Mol. Appl. Gen. 2: 101), each of which are incorporated by reference herein in its entirety. Other suitable reporters include those that encode for a particular epitope that can be detected with a labeled antibody that specifically recognizes the epitope.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, *i.e.,* an α carbon that is bound to a hydrogen, a carboxyl group, an amino group, and an R group, *e.g.,* homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. "Amino acid mimetics" refers to chemical compounds having a structure that is different from the general chemical structure of an amino acid, but that functions in a manner similar to a naturally occurring amino acid.

There are various known methods in the art that permit the incorporation of an unnatural amino acid derivative or analog into a polypeptide chain in a site-specific manner, *see, e.g.,* WO 02/086075.

Amino acids may be referred to herein by either the commonly known three letter symbols or by the one-letter symbols recommended by the IUPAC-IUB Biochemical Nomenclature Commission. Nucleotides, likewise, may be referred to by their commonly accepted single-letter codes.

"Polypeptide," "peptide," and "protein" are used interchangeably herein to refer to a polymer of amino acid residues. All three terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins, wherein the amino acid residues are linked by covalent peptide bonds.

"Conservatively modified variants" applies to both amino acid and nucleic acid sequences. With respect to particular nucleic acid sequences, "conservatively modified variants" refers to those nucleic acids that encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein that encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. In some cases, conservatively modified variants of Cas9 or sgRNA can be utilized as described herein.

The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
(*see, e.g.,* Creighton, Proteins, W. H. Freeman and Co., N. Y. (1984)).

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

A "translocation sequence" or "transduction sequence" refers to a peptide or protein (or active fragment or domain thereof) sequence that directs the movement of a protein from one cellular compartment to another, or from the extracellular space through the cell or plasma membrane into the cell. Translocation sequences that direct the movement of a protein from the extracellular space through the cell or plasma membrane into the cell are "cell penetration peptides." Translocation sequences that localize to the nucleus of a cell are termed "nuclear localization" sequences, signals, domains, peptides, or the like. Examples of translocation sequences include, without limitation, the TAT transduction domain (see, e.g., S. Schwarze et al., Science 285 (Sep. 3, 1999); penetratins or penetratin peptides (D. Derossi et al., Trends in Cell Biol. 8, 84-87); Herpes simplex virus type 1 VP22 (A. Phelan et al., Nature Biotech. 16, 440-443 (1998), and polycationic (e.g., poly-arginine) peptides (Cell Mol. Life Sci. 62 (2005) 1839-1849). Further translocation sequences are known in the art. Translocation peptides can be fused (*e.g.* at the amino or carboxy terminus), conjugated, or coupled to a compound of the present invention, to, among other things, produce a conjugate compound that may easily pass into target cells, or through the blood brain barrier and into target cells.

The "CRISPR/Cas" system refers to a widespread class of bacterial systems for defense against foreign nucleic acid. CRISPR/Cas systems are found in a wide range of eubacterial and archaeal organisms. CRISPR/Cas systems include type I, II, and III subtypes. Wild-type type II CRISPR/Cas systems utilize an RNA-mediated nuclease,Cas9 in complex with guide and activating RNA to recognize and cleave foreign nucleic acid. Guide RNAs having the activity of both a guide RNA and an activating RNA are also known in the art. In some cases, such dual activity guide RNAs are referred to as a small guide RNA (sgRNA).

Cas9 homologs are found in a wide variety of eubacteria, including, but not limited to bacteria of the following taxonomic groups: *Actinobacteria, Aquificae, Bacteroidetes-Chlorobt, Chlamydiae-Verrucomicrobia, Chlroflexi, Cyanobacteria, Firmicutes, Proteobacteria, Spirochaetes,* and *Thermotogae.* An exemplary Cas9 protein is the *Streptococcus pyogenes* Cas9 protein. Additional Cas9 proteins and homologs thereof are described in, *e.g.,* Chylinksi, et al., RNA Biol. 2013 May 1; 10(5): 726-737 ; Nat. Rev. Microbiol. 2011 June; 9(6): 467-477; Hou, et al., Proc Natl Acad Sci U S A. 2013 Sep 24; 110(39): 15644-9; Sampson et al., Nature. 2013 May 9;497(7448):254-7; and Jinek, et al., Science. 2012 Aug 17;337(6096):816-21. The Cas9 nuclease domain can be optimized for efficient activity or enhanced stability in the host cell.

As used herein, the term "Cas9" refers to an RNA-mediated nuclease (e.g., of bacterial or archeal orgin, or derived therefrom). Exemplary RNA-mediated nuclases include the foregoing Cas9 proteins and homologs thereof, and include but are not limited to, CPF1 (*See, e.g.,* Zetsche et al., Cell, Volume 163, Issue 3, p759-771, 22 October 2015). Similarly, as used herein, the term "Cas9 ribonucleoprotein" complex and the like refers to a complex between the Cas9 protein, and a crRNA *(e.g.,* guide RNA or small guide RNA), the Cas9 protein and a trans-activating crRNA (tracrRNA), the Cas9 protein and a small guide RNA, or a combination thereof (*e.g*., a complex containing the Cas9 protein, a tracrRNA, and a crRNA guide RNA).

As used herein, the phrase "editing" in the context of editing of a genome of a cell refers to inducing a structural change in the sequence of the genome at a target genomic region. For example, the editing can take the form of inducing an insertion deletion (indel) mutation into a sequence of the genome at a target genomic region. Such editing can be performed by inducing a double stranded break within a target genomic region, or a pair of single stranded nicks on opposite strands and flanking the target genomic region. Methods for inducing single or double stranded breaks at or within a target genomic region include the use of a Cas9 nuclease domain, or a derivative thereof, and a guide RNA, or pair of guide RNAs, directed to the target genomic region.

As used herein, the phrase "introducing" in the context of introducing a Cas9 ribonucleoprotein complex or introducing a Cas9 nuclease domain refers to the translocation of the Cas9 protein or Cas9 ribonucleoprotein complex from outside a cell to inside the cell. In some cases, introducing refers to translocation of the Cas9 or Cas9 ribonucleoprotein from outside the cell to inside the nucleus of the cell. Various methods of such translocation are contemplated, including but not limited to, electroporation, contact with nanowires or nanotubes, receptor mediated internalization, translocation via cell penetrating peptides, liposome mediated translocation, and the like.

As used herein, the phrase "primary" in the context of a primary cell or primary stem cell refers to a cell that has not been transformed or immortalized. Such primary cells can be cultured, sub-cultured, or passaged a limited number of times (e.g., cultured 0, 1, 2, 3, 4, 5, 6, 7, 8, 9, 10, 11, 12, 13, 14, 15, 16, 17, 18, 19, or 20 times). In some cases, the primary cells are adapted to *in vitro* culture conditions. In some cases, the primary cells are isolated from an organism, system, organ, or tissue, optionally sorted, and utilized directly without culturing or sub-culturing. In some cases, the primary cells are stimulated, activated, or differentiated. For example, primary T cells can be activated by contact with (e.g., culturing in the presence of) CD3, CD28 agonists, IL-2, IFN-γ, or a combination thereof.

As used herein, the phrase "hematopoietic stem cell" refers to a type of stem cell that can give rise to a blood cell. Hematopoietic stem cells can give rise to cells of the myeloid or lymphoid lineages, or a combination thereof. Hematopoietic stem cells are predominantly found in the bone marrow, although they can be isolated from peripheral blood, or a fraction thereof. Various cell surface markers can be used to identify, sort, or purify hematopoietic stem cells. In some cases, hematopoietic stem cells are identified as c-kit⁺ and lin⁻. In some cases, human hematopoietic stem cells are identified as CD34⁺, CD59⁺, Thy1/CD90⁺, CD38^{lo/-}, C-kit/CD117⁺, lin⁻. In some cases, human hematopoietic stem cells are identified as CD34⁻, CD59⁺, Thy1/CD90⁺, CD38^{lo/-}, C-kit/CD117⁺, lin⁻. In some cases, human hematopoietic stem cells are identified as CD133⁺, CD59⁺, Thy1/CD90⁺, CD38^{lo/-}, C-kit/CD117⁺, lin⁻. In some cases, mouse hematopoietic stem cells are identified as CD34^{lo/-}, SCA-1⁺, Thyl^{+/lo}, CD38⁺, C-kit⁺, lin⁻. In some cases, the hematopoietic stem cells are CD150⁺CD48⁻CD244⁻.

As used herein, the phrase "hematopoietic cell" refers to a cell derived from a hematopoietic stem cell. The hematopoietic cell may be obtained or provided by isolation from an organism, system, organ, or tissue (*e.g*., blood, or a fraction thereof). Alternatively, an hematopoietic stem cell can be isolated and the hematopoietic cell obtained or provided by differentiating the stem cell. Hematopoietic cells include cells with limited potential to differentiate into further cell types. Such hematopoietic cells include, but are not limited to, multipotent progenitor cells, lineage-restricted progenitor cells, common myeloid progenitor cells, granulocyte-macrophage progenitor cells, or megakaryocyte-erythroid progenitor cells. Hematopoietic cells include cells of the lymphoid and myeloid lineages, such as lymphocytes, erythrocytes, granulocytes, monocytes, and thrombocytes. In some embodiments, the hematopoietic cell is an immune cell, such as a T cell, B cell, macrophage, or dendritic cell.

As used herein, the phrase "T cell" refers to a lymphoid cell that expresses a T cell receptor molecule. T cells include, but are not limited to, naive T cells, stimulated T cells, primary T cells (*e.g.*, uncultured), cultured T cells, immortalized T cells, helper T cells, cytotoxic T cells, memory T cells, regulatory T cells, natural killer T cells, combinations thereof, or sub-populations thereof. T cells can be CD4⁺, CD8⁺, or CD4⁺ and CD8⁺. T cells can be helper cells, for example helper cells of type Tₕ1, Tₕ2, Tₕ3, Tₕ9, Tₕ17, or T_{FH}. T cells can be cytotoxic T cells. Regulatory T cells can be FOXP3⁺ or FOXP3⁻. T cells can be alpha/Beta T cells or gamma/delta T cells. In some cases, the T cell is a CD4⁺CD25^{hi}CD127^{lo} regulatory T cell. In some cases, the T cell is a regulatory T cell selected from the group consisting of Tr1, Th3, CD8+CD28-, Treg17, and Qa-1 restricted T cells, or a combination or sub-population thereof. In some cases, the T cell is a FOXP3⁺ T cell. In some cases, the T cell is a CD4⁺CD25^{lo}CD127^{hi} effector T cell. In some cases, the T cell is a CD4⁺CD25^{lo}CD127^{hi}CD45RA^{hi}CD45RO⁻ naive T cell.

A T cell can be a recombinant T cell that has been genetically manipulated. In some cases, the recombinant T cell has a recombinant (*e.g*., mutated or heterologous) T cell receptor. For example, the T cell receptor can have one or more mutations in a complementarity determining region of a T cell receptor to alter antigen specificity. As another example, the T cell receptor can be mutated (*e.g.*, in the endodomain) to increase or decrease signaling. As yet another example, the T cell receptor can be replaced with a heterologous T cell receptor. As yet another example, the T cell receptor can be replaced with a polypeptide having a different receptor domain, such as an antibody or antibody fragment. In some cases, the T cell receptor is a chimeric receptor containing a targeting domain (*e.g.*, an antibody fragment), a transmembrane domain, and an intracellular or endodomain domain. The endodomain can contain one or more signaling domains and/or adaptor domains to provide robust T cell activation and anti-antigen activity.

As used herein, the term "non-homologous end joining" or NHEJ refers to a cellular process in which cut or nicked ends of a DNA strand are directly ligated without the need for a homologous template nucleic acid. NHEJ can lead to the addition, the deletion, substitution, or a combination thereof, of one or more nucleotides at the repair site.

As used herein, the term homology directed repair (HDR) refers to a cellular process in which cut or nicked ends of a DNA strand are repaired by polymerization from a homologous template nucleic acid. Thus, the original sequence is replaced with the sequence of the template. The homologous template nucleic acid can be provided by homologous sequences elsewhere in the genome (sister chromatids, homologous chromosomes, or repeated regions on the same or different chromosomes). Alternatively, an exogenous template nucleic acid can be introduced to obtain a specific HDR-induced change of the sequence at the target site. In this way, specific mutations can be introduced at the cut site.

As used herein, the phrase "single-stranded oligonucleotide DNA template" or "ssODT" refers to a DNA oligonucleotide that can be utilized by a cell as a template for HDR. Generally, the ssODT has at least one region of homology to a target site. In some cases, the ssODT has two homologous regions flanking a region that contains a mutation or a heterologous sequence to be inserted at a target cut site.

### BRIEF DESCRIPTION OF THE DRAWINGS

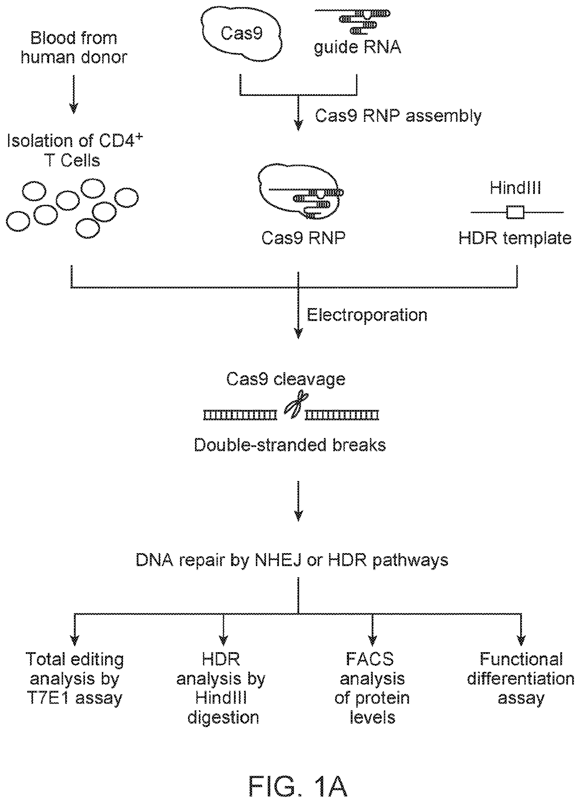

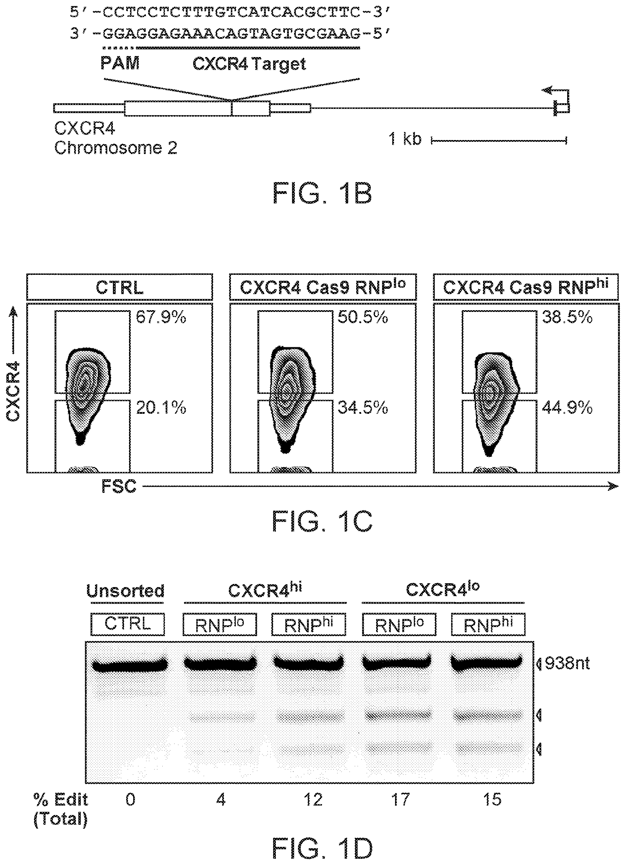

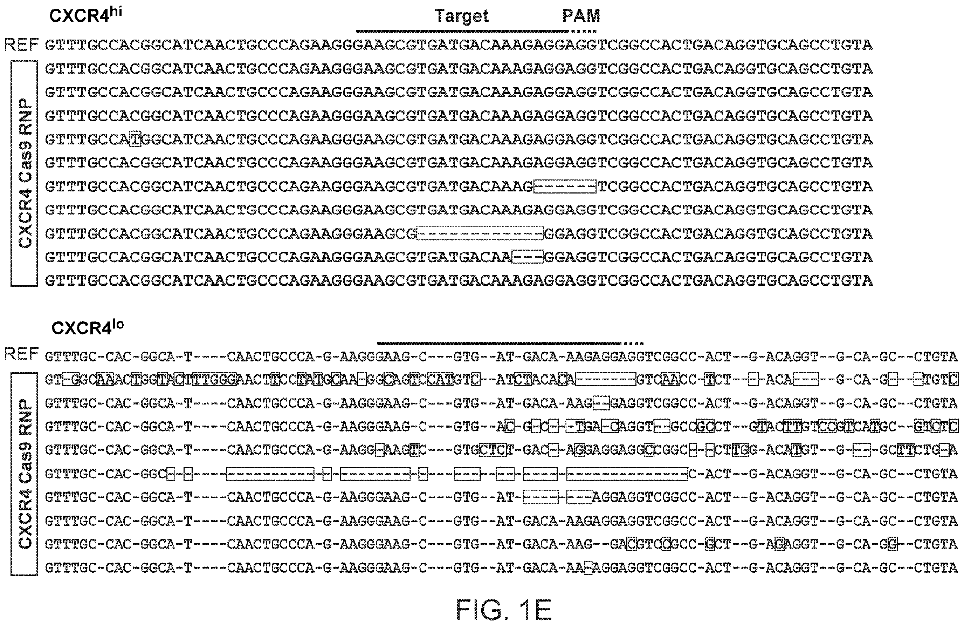

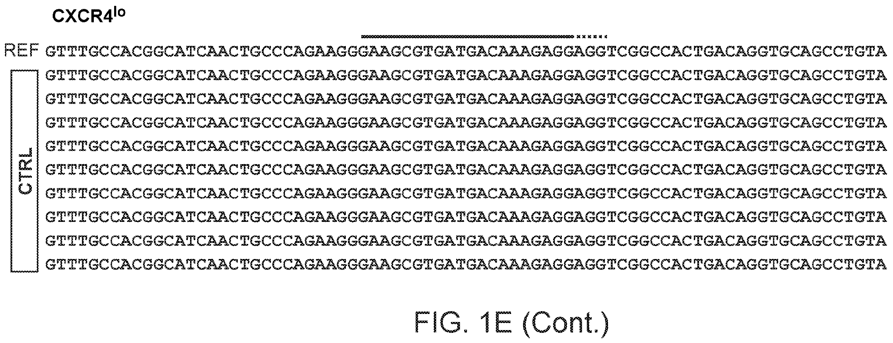

**Figure 1****. Robust editing of human *CXCR4* locus in primary human CD4⁺ T cells. (A)** Experimental scheme of Cas9:single-guide RNA ribonucleoprotein (Cas9 RNP) delivery to primary human CD4⁺ T cells for genome editing, followed by genetic and phenotypic characterization. **(B)** Schematic representation of single-guide RNA (sgRNA) target (blue) and PAM (green) sequence designed to edit coding sequence in the human *CXCR4* locus. (C) FACS plots show increasing percentages of cells with low CXCR4 expression (CXCR4^{lo}) with higher concentrations of CXCR4 Cas9 RNP compared to control treated cells (Cas9 without sgRNA, CTRL). **(D)** T7 endonuclease I (T7E1) assay demonstrates genome editing in the *CXCR4* locus with more editing observed in FACS-sorted CXCR4^{lo} cells than in CXCR4^{hi} cells. Expected PCR product size (938 nucleotides; nt) and approximate expected T7E1 fragment sizes are indicated. The total editing frequency was measured using a T7 endonuclease I assay and analyzed using a formula described in 'Materials and Methods' and numerical results are indicated as % Edit (Total) below the agarose gel image. (E) Mutation patterns detected by sequencing of *CXCR4* locus in sorted Cas9 RNP treated CXCR4^{hi} and CXCR4^{lo} are compared to the sequence from CXCR4^{lo} control treated cells (CTRL). Reference (REF) sequence is shown on top of clonal sequences from each population with sgRNA target (blue) and PAM (green) sequences indicated. Red dashes denote deleted bases and red sequences indicate mutated or inserted nucleotides. Non-mutated sequences from several clones were truncated.

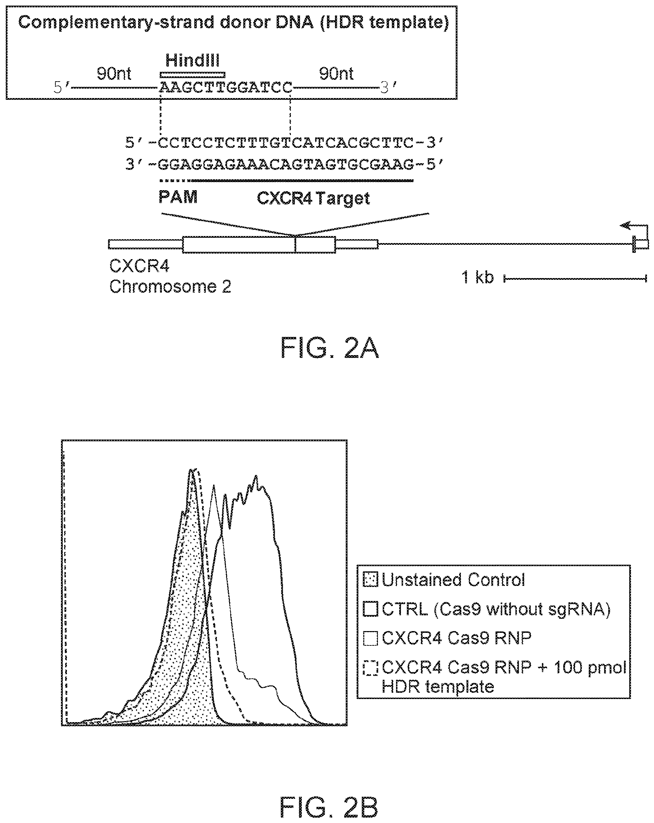

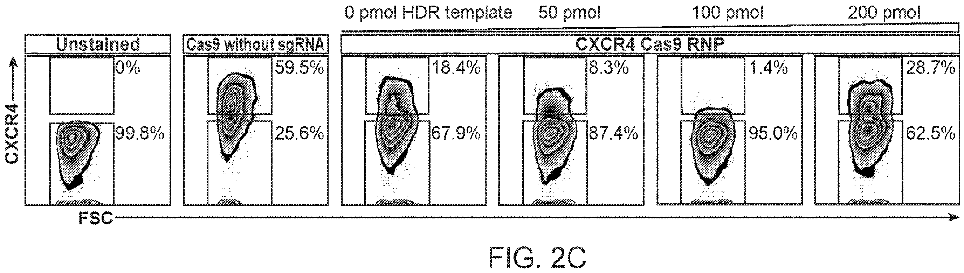

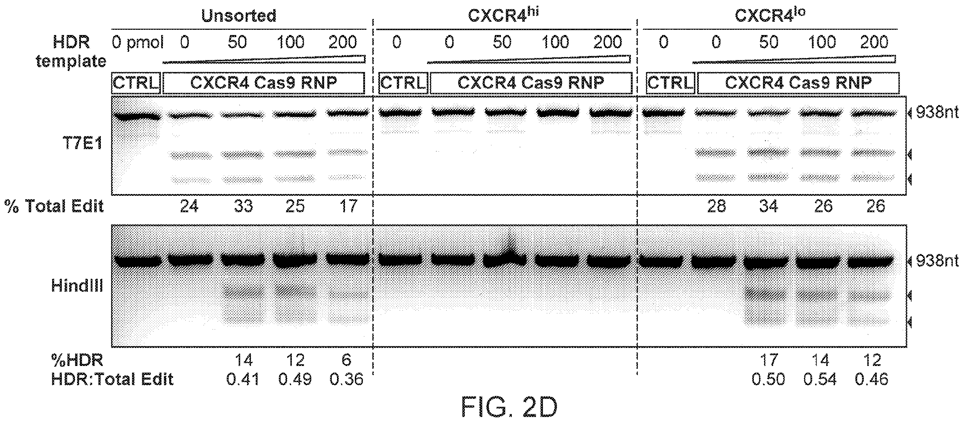

**Figure 2****. Efficient homology-directed repair allows targeted DNA replacement in primary human T cells. (A)** Schematic representation of single-stranded oligonucleotide HDR template with 90 nucleotide (nt) homology arms designed to replace 12 nt and introduce a novel HindIII restriction enzyme cleavage site (orange) at the *CXCR4* locus, where the Cas9 RNP cleaves. sgRNA target (blue) and PAM (green) sequence are indicated. (B) Histogram of CXCR4 cell surface staining assessed by flow cytometry in CXCR4 Cas9 RNP-treated cells in the presence and absence of single-stranded HDR template (compared to control Cas9 protein-treated cells and unstained cells). (C) FACS plots (corresponding to histogram in Panel B) show maximal ablation of CXCR4 with Cas9 RNP treatment and 100 pmol of ssODT. **(D)** T7E1 assay was used to calculate the total editing (defined as the sum of all NHEJ and HDR events that give rise to indels at Cas9 cleavage site) percentage, whereas HDR frequency was determined by HindIII digestion, which specifically cleaved the newly integrated HindIII site, and calculated as the ratio of DNA product to DNA substrate. Expected PCR product size (938 nucleotides; nt) and approximate expected T7E1 and HindIII digestion fragments are indicated. Total editing and HDR frequencies were calculated in control cells and in CXCR4 Cas9 RNP treated in cells with varying concentrations of ssODT (0, 50, 100 and 200 pmol) and numerical results are displayed below agarose gel image.

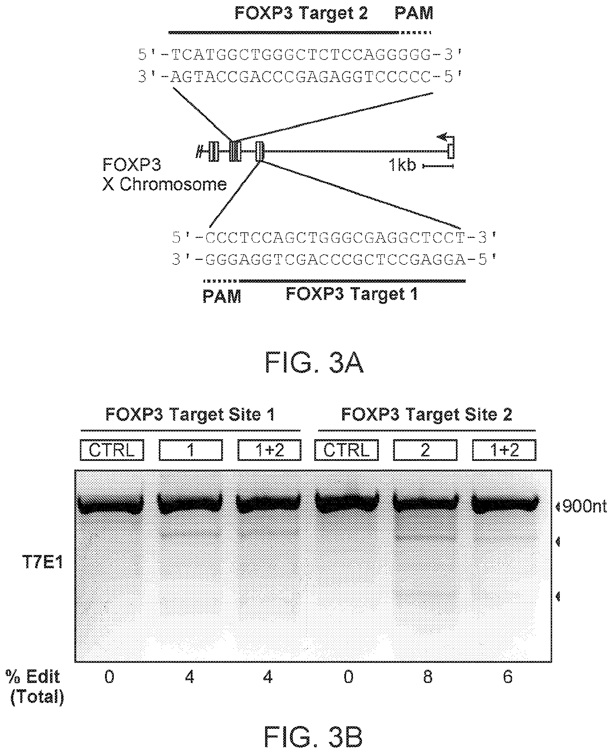

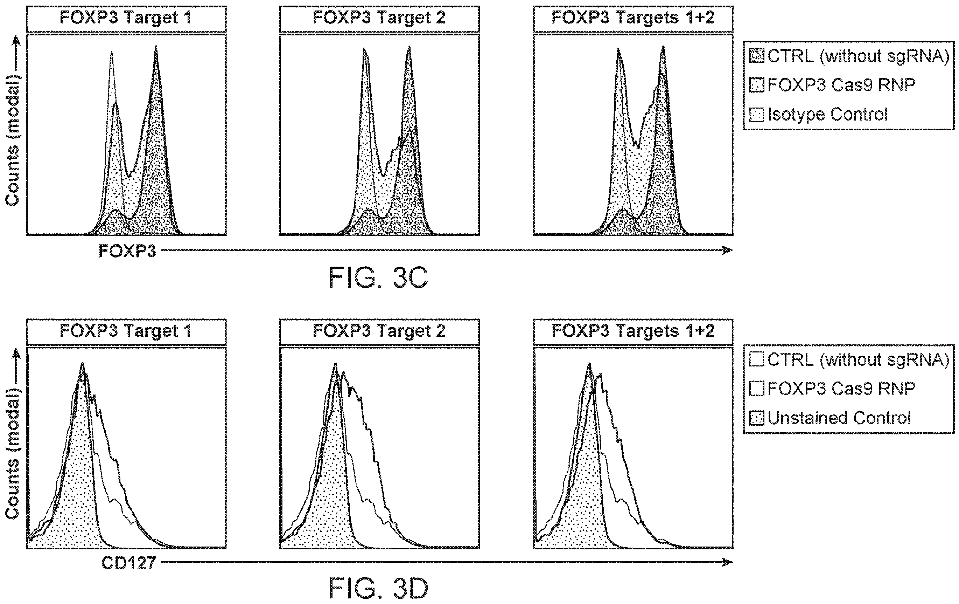

**Figure 3****. Genome editing of *FOXP3* de-stabilizes human Treg cytokine receptor levels. (A)** Schematic representation of two sgRNA targets (blue) and PAM sequences (green) designed to edit coding sequences in the human *FOXP3* locus. (B) T7E1 assay confirms genome editing at two targets in the *FOXP3* locus with expected PCR product size (900 nucleotides; nt) and approximate expected T7E1 fragment sizes indicated. (C) Histogram of intracellular FOXP3 levels assessed by flow cytometry in FOXP3 Cas9 RNP treated cells compared to controls (Cas9 protein without sgRNA and isotype staining control). (D) Histogram of CD127 (IL7Ra) cell surface staining assessed by flow cytometry in FOXP3 Cas9 RNP treated cells compared to controls (Cas9 protein without sgRNA and unstained control).

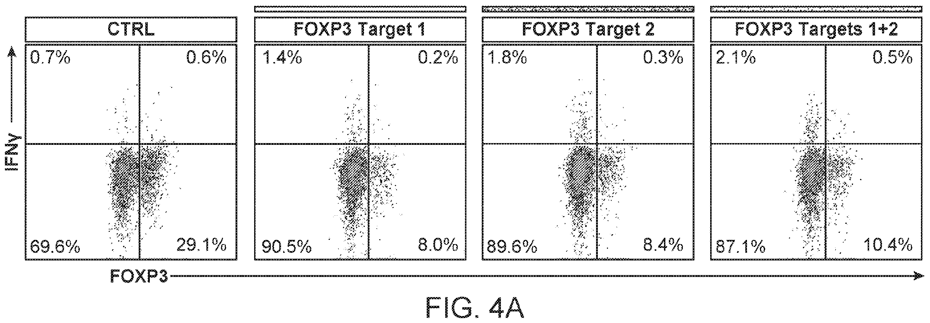

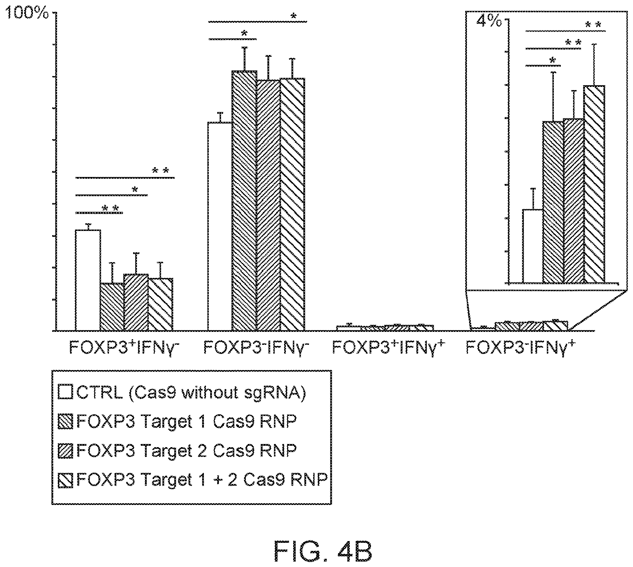

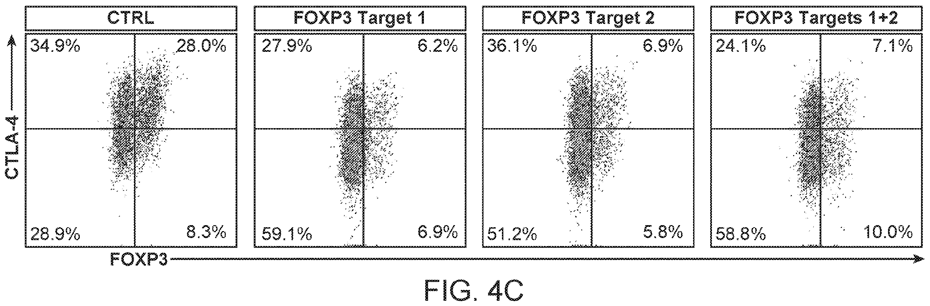

**Figure 4****. Cas9 RNPs targeting *FOXP3* impair human induced Treg differentiation.** (A) Naive CD4⁺ T cells were electroporated with Cas9 RNPs following two days of *ex vivo* stimulation. Following Cas9 RNP treatment, cells were cultured in iTreg generating conditions with IL-2 and TGF-β. FOXP3 Cas9 RNPs reduced FOXP3⁺ iTreg generation and led to an increased percentage of cells secreting IFNγ, a pro-inflammatory cytokine (assessed by flow cytometry). **(B)** The quantities of FOXP3⁺ and IFNγ secreting cells with FOXP3 Cas9 RNPs or control RNP were calculated from three experiments (error bars show standard deviation; significant differences relative to control cells are indicated: * p<0.05, ** p<0.01). Insert shows percentages of FOXP3⁺IFNγ⁺ on a magnified scale. (C) FOXP3 Cas9 RNPs reduced FOXP3⁺CTLA-4⁺ iTreg generation (assessed by FACS). CTLA-4⁺ expression in the FOXP3⁻ population was less affected, consistent with FOXP3-dependent and FOXP3-independent mechanisms both contributing to CTLA-4 expression.

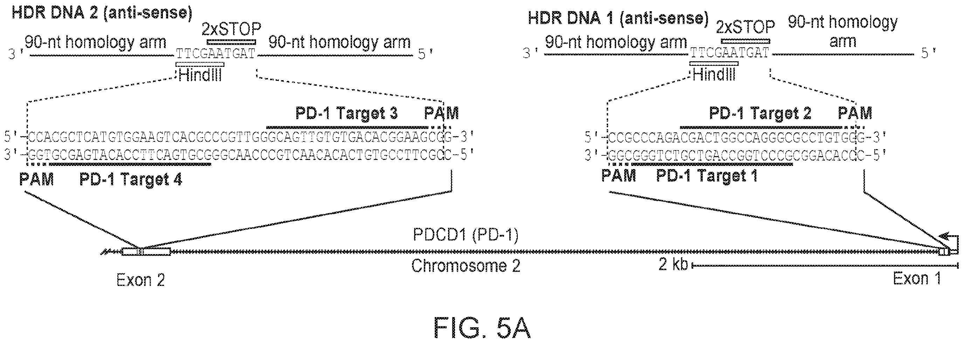

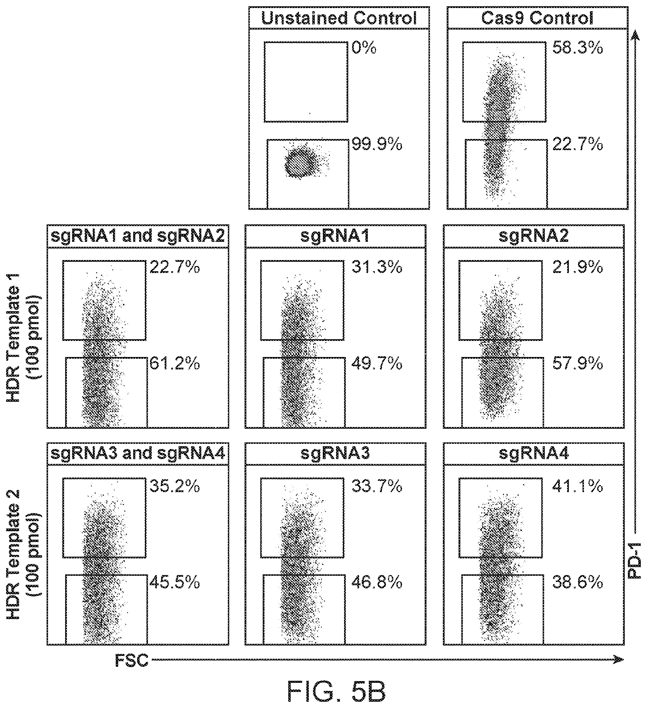

**Figure 5****:** Illustrates successful editing of the PD-1 encoding genomic region in primary human effector T cells (CD4⁺CD25^{lo}CD127^{hi}).

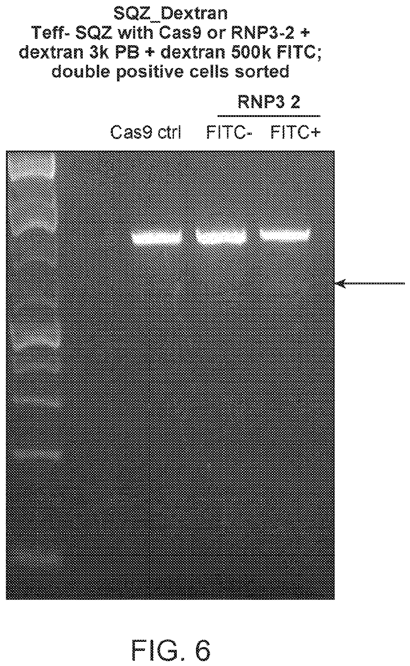

**Figure 6****:** Illustrates the results of Cas9 RNP delivery to unstimulated effector CD4⁺ T cells using a cell squeezing apparatus in which a reaction mixture containing the cells and the Cas9 RNP is forced through a cell deforming constriction that is smaller than the diameter of the cell. The forcing introduces transient pores into a cell membrane of the cell, which allows the Cas9 RNP to enter the cell through the transient pores. Cells were sorted based on uptake of a Pacific Blue (PB)-labeled Dextran (3 kD) FITC-labeled Dextran (500 kD). A T7 endonuclease 1 assay confirmed enrichment of editing in cells that had taken up both Dextrans.

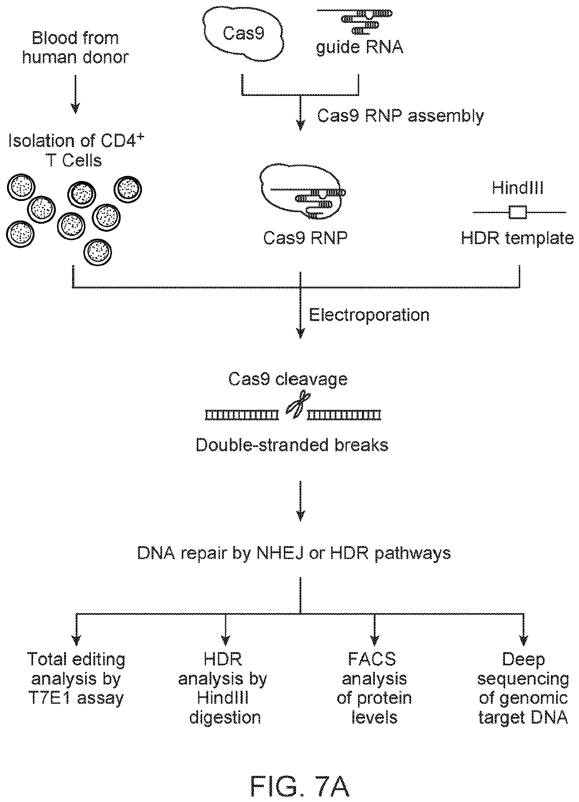

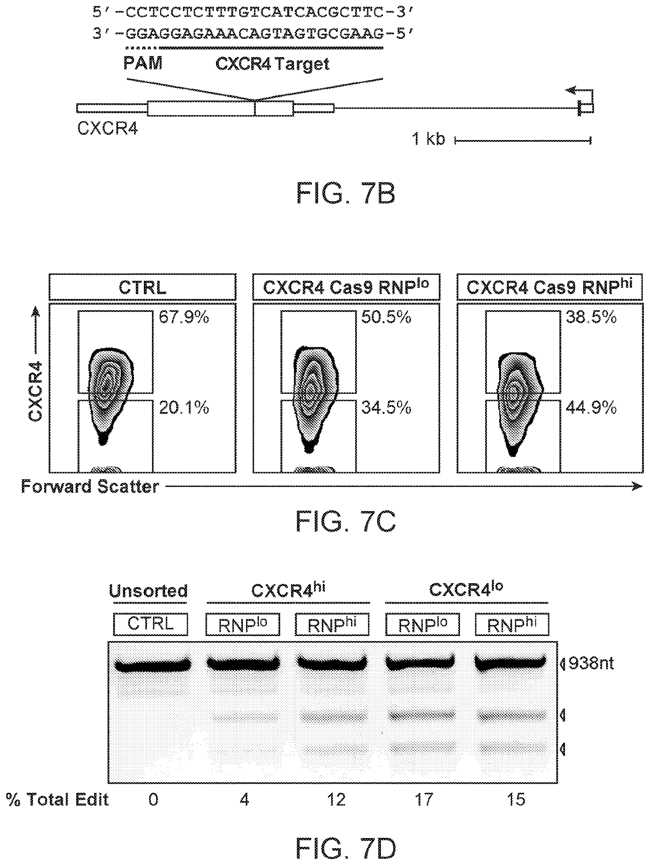

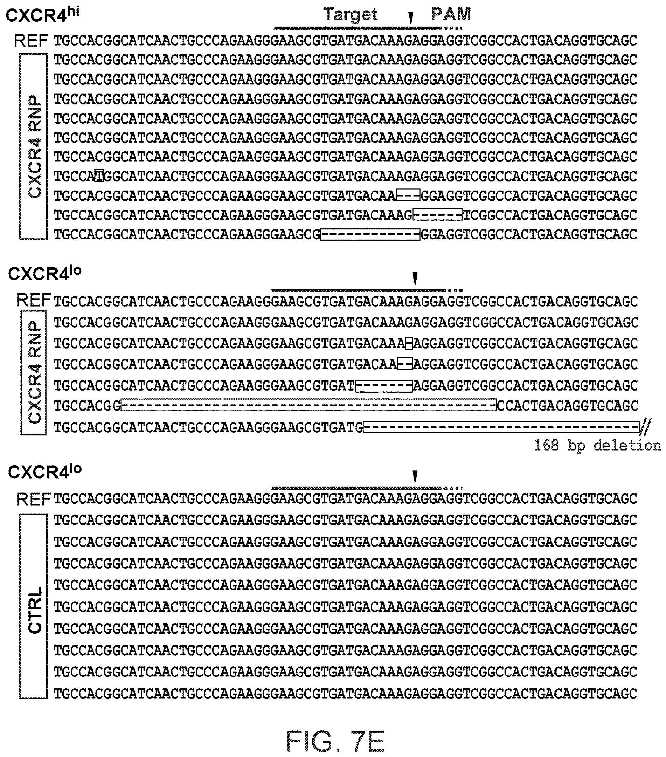

**Figure 7****:** Illustrates Efficient editing of ***CXCR4*** in primary human CD4⁺ T cells. (A) Experimental scheme of Cas9:single-guide RNA ribonucleoprotein (Cas9 RNP) delivery to primary human CD4⁺ T cells for genome editing, followed by genetic and phenotypic characterizations. (B) Schematic representation of single-guide RNA (sgRNA) target and PAM sequence designed to edit coding sequence in the human *CXCR4* locus. (C) FACS plots show increasing percentages of cells with low CXCR4 expression (CXCR4^{lo}) with higher concentrations of CXCR4 Cas9 RNP (Cas9 RNP^{lo}: 0.9 µM; Cas9 RNP^{hi}: 1.8 µM) compared to control treated cells (Cas9 without sgRNA, CTRL; final concentration: 1.8 µM). (D) T7 endonuclease I (T7E1) assay demonstrates genome editing in the *CXCR4* locus with more editing observed in FACS-sorted CXCR4^{lo} cells than in CXCR4^{hi} cells. Expected PCR product size (938 nucleotides; nt) and approximate expected sizes of T7E1 digested fragments are indicated. The total editing frequencies are indicated as % Total Edit below the agarose gel image. (E) Mutation patterns detected by cloning and Sanger sequencing of *CXCR4* locus in sorted Cas9 RNP (1.8 µM) treated CXCR4^{hi} and CXCR4^{lo} cells are compared to the sequence from CXCR4^{lo} control treated cells (CTRL). Reference (REF) sequence is shown on top of clonal sequences from each population with sgRNA target (blue) and PAM (green) sequences indicated. Red dashes denote deleted bases and red sequences indicate mutated nucleotides. Arrowhead indicates the predicted Cas9 cut site. Poor quality sequences obtained from three additional CXCR4^{lo} clones were removed from the sequence alignment.

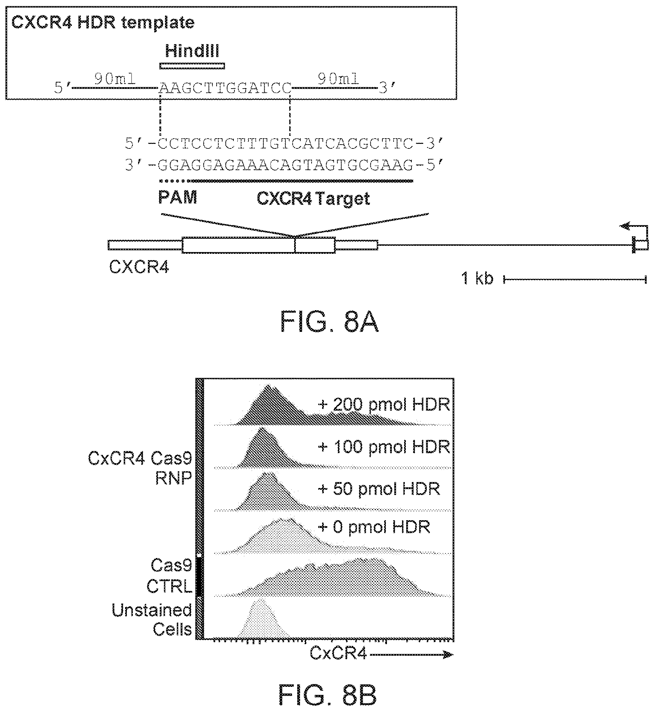

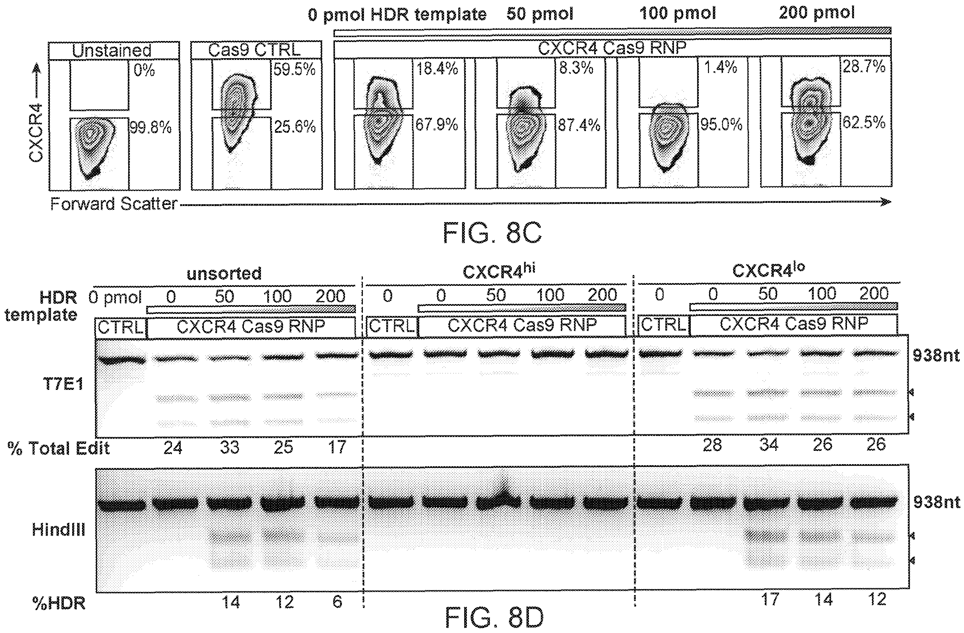

**Figure 8****:** Efficient homology-directed repair allows targeted DNA replacement in primary human T cells. (A) Schematic representation of single-stranded oligonucleotide HDR template with 90 nt homology arms designed to replace 12 nt including the PAM sequence and introduce a novel HindIII restriction enzyme cleavage site at the *CXCR4* locus, where the Cas9 RNP cleaves. sgRNA target and PAM sequence are indicated. (B) Histograms of CXCR4 cell surface staining assessed by flow cytometry in CXCR4 Cas9 RNP-treated cells in the presence of varying concentrations of single-stranded HDR template (compared to control Cas9 protein-treated cells and unstained cells). (C) FACS plots (corresponding to histograms in Panel B) show maximal ablation of CXCR4 with Cas9 RNP treatment and 100 pmol of HDR template. (D) T7E1 assay was used to estimate the % Total Edit (defined as the sum of all NHEJ and HDR events that give rise to indels at Cas9 cleavage site) percentage, whereas HDR frequency was determined by HindIII digestion, which specifically cleaved the newly integrated HindIII site, and calculated as the ratio of DNA product to DNA substrate. Expected PCR product size (938 nt) and approximate expected T7E1 and HindIII digestion fragments are indicated.

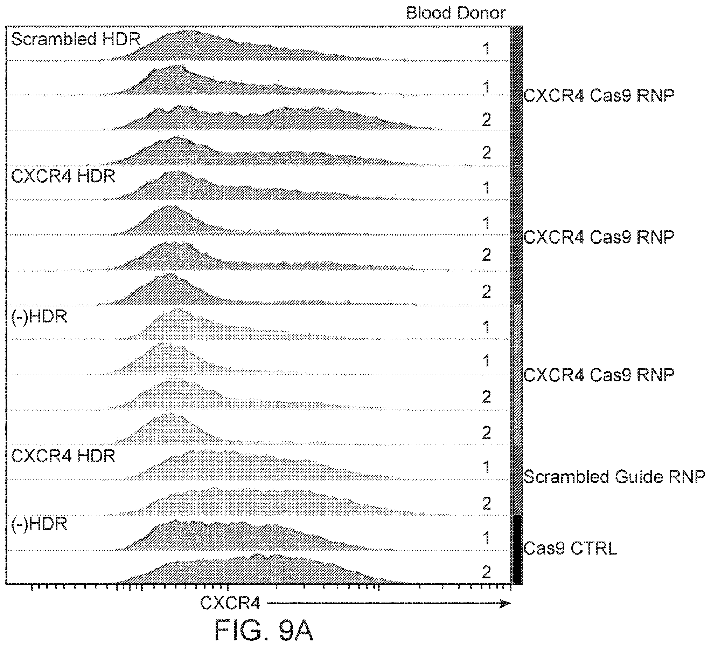

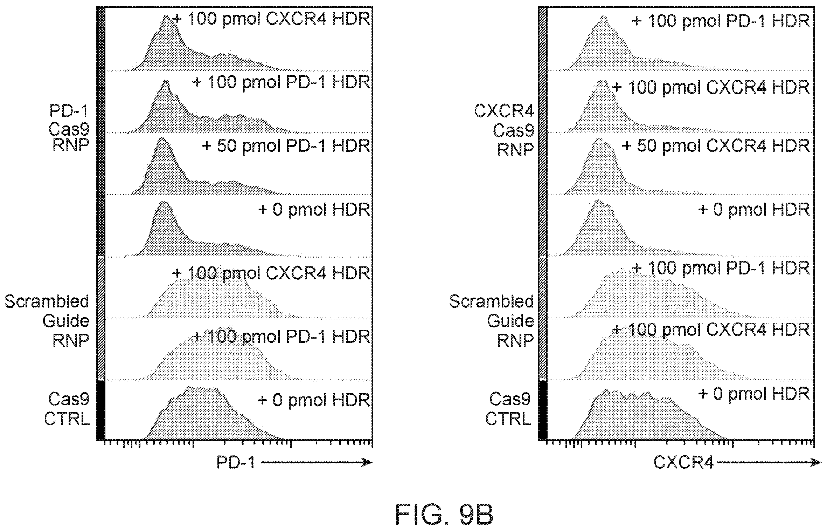

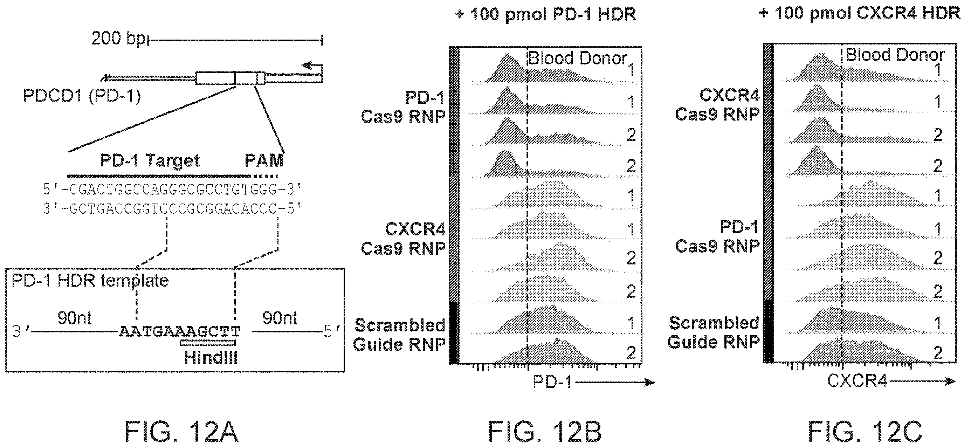

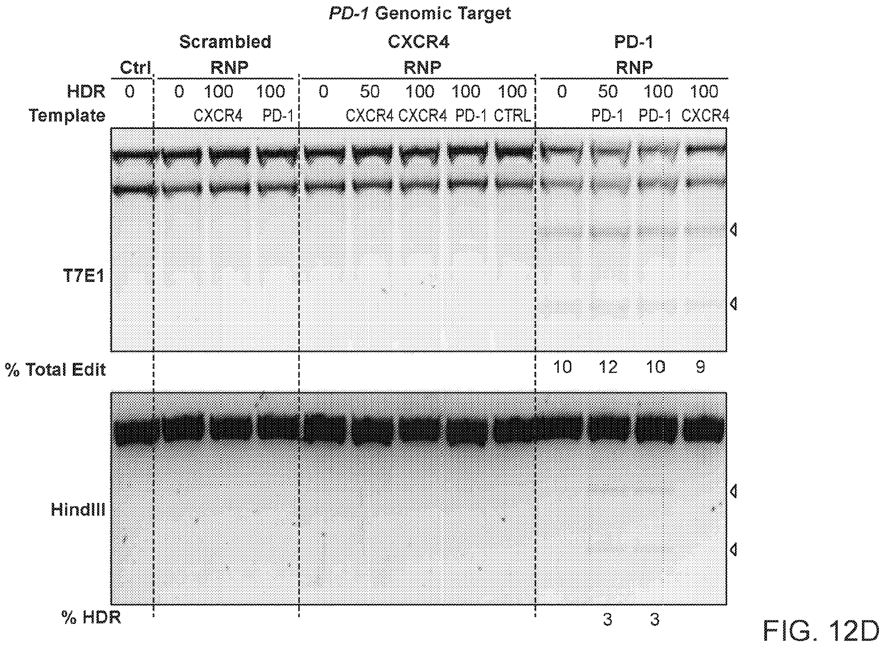

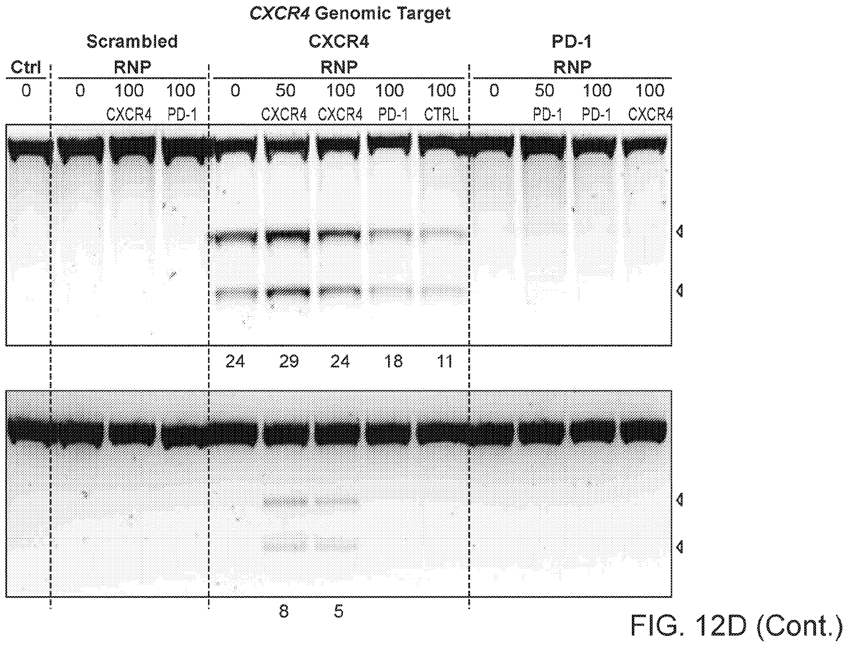

**Figure 9****:** Effects of 'on-target' and control HDR templates on PD-1 and CXCR4 surface expression levels. (A) The effects on CXCR4 expression were tested for two different HDR templates with the same nucleotide composition. In cells that were all treated with CXCR4 Cas9 RNP, CXCR4 HDR template (rows 5-8) was compared with a control HDR template consisting of the same nucleotides as the original CXCR4 HDR in randomized order including a HindIII restriction site (rows 1-4) and with no HDR template treatment (rows 9-12). Further controls are Cas9 CTRL (Cas9 without HDR template; final two rows) and scrambled guide Cas9 RNP (no predicted cut within the human genome) with 100 pmol CXCR4 HDR template (rows 13 and 14). The histograms show the results of 4 experiments with 2 differently *in vitro* transcribed CXCR4 sgRNAs (two different purification strategies, see **Materials and Methods** section of Example 4) tested in 2 different blood donors. As in Figure 12, for each blood donor, experiments done with phenol/chloroform extracted sgRNAs are shown on top and experiments with PAGE purified sgRNAs are shown below; scrambled guides were prepared for both experiments with phenol/chloroform extraction. (B) PD-1 (left panel) and CXCR4 (right panel) surface expression levels after editing with the respective Cas9 RNPs and on- or off-target HDR templates. Targeted cells were compared to cells treated with Cas9 CTRL (dark grey) or scrambled guide Cas9 RNP as indicated.

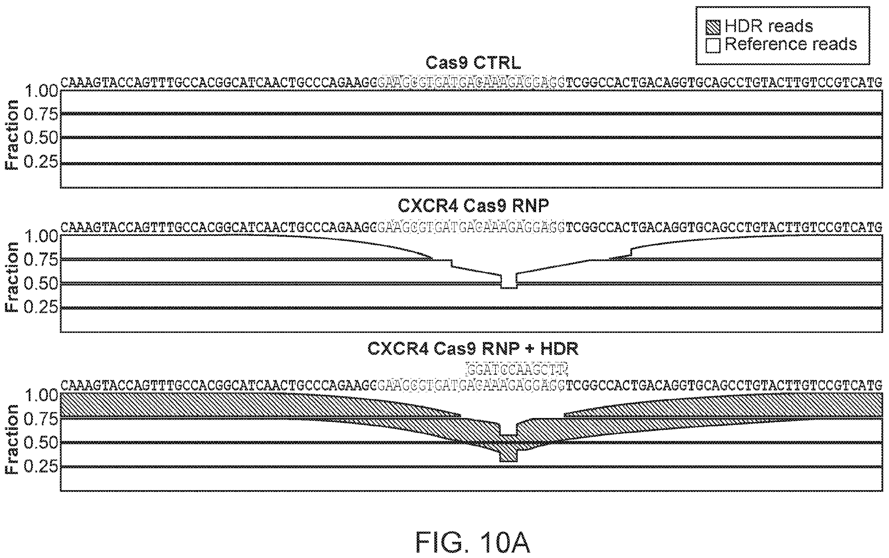

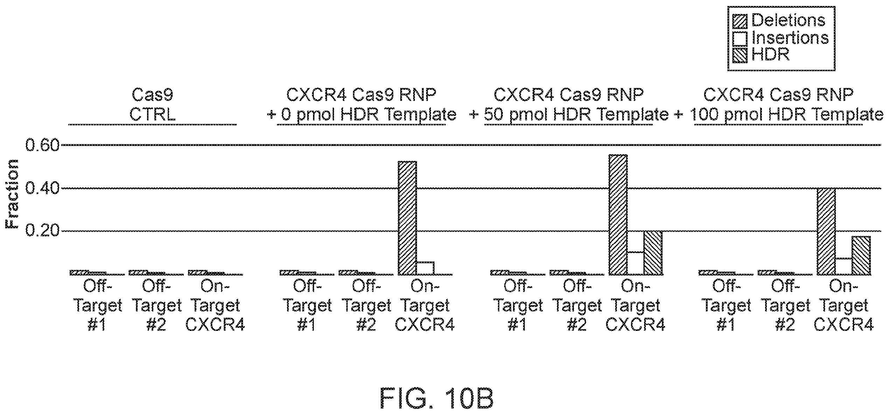

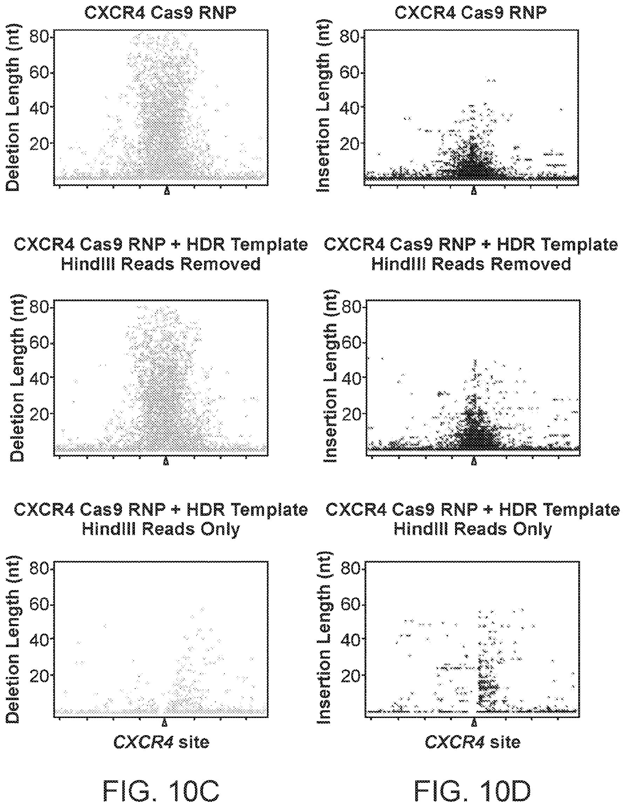

**Figure 10****:** Quantitative analysis of Cas9 RNP-mediated editing and HDR by deep-sequencing. (A) CXCR4 Cas9 RNP-mediated indels and HDR from experiments in Figure 8 were analyzed by targeted deep sequencing of the *CXCR4* locus. A total of 100 nt centered on the predicted cut site are shown with sgRNA target, PAM, and predicted sequence after HDR genome targeting. At each position, the fraction of reads that correctly aligned to the reference genome or HDR template-derived sequence are shown. Although rare (∼1-2%), edits were detected with Cas9 only control treatment, including at the predicted *CXCR4* cut site, potentially indicating trace amounts of experimental contamination of the Cas9 RNPs. (B) Bar graph summarizes the fractions of reads edited with deletions, insertions, or successful HDR targeting in Cas9 CTRL, CXCR4 Cas9 RNP and CXCR4 Cas9 RNP cells with 50 pmol or 100 pmol CXCR4 HDR template at the *CXCR4* site and two predicted off-target sites. Reads with HDR template-derived sequence incorporated were removed to calculate fractions with deletions and insertions. Scatter plots show the genomic localization (+/- 100 nt around the expected Cas9 cut side; chromosome2:136873140-136873340) and the length of (C) deletions and (D) insertions. Top panel shows deletions/insertions for CXCR4 RNP treated cells; middle shows deletions/insertions in reads without HDR template sequence incorporated in cells treated with CXCR4 RNP and CXCR4 HDR template; bottom shows deletions/insertions in reads with HDR template-derived sequence incorporated. Arrowheads indicate approximate location of expected Cas9 cut site.

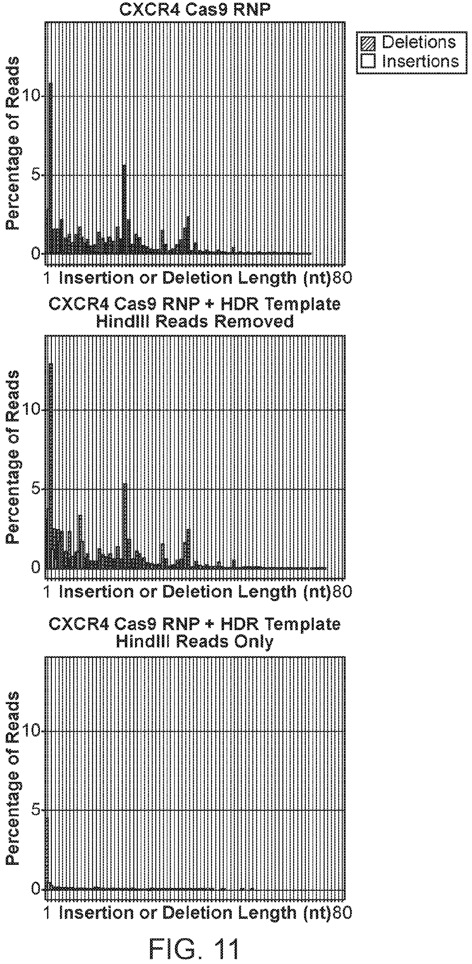

**Figure 11****:** Distribution of insertion and deletion lengths near expected *CXCR4* cut site. Histograms show the percent of reads that contain varying sizes of deletions (grey bars) and insertions (black bars) within +/- 20 nt of the predicted cut site. Top shows insertions and deletions for CXCR4 RNP treated cells. Middle shows insertions and deletions in reads without HDR template-derived sequence incorporated in the cells treated with CXCR4 RNP and CXCR4 HDR template (bottom). Insertions and deletions in reads that did incorporate the HDR template-derived sequence in the cells treated with CXCR4 RNP and CXCR4 HDR template.
**Figure 12****:** Figure 4. Cas9 RNPs can be programmed for knock-in editing of *PD-1* or *CXCR4.* (A) Schematic representation of the single-stranded PD-1 HDR template with 90 nt homology arms designed to replace 12 nt with 11 nt introducing a novel HindIII restriction enzyme cleavage site to replace the PAM sequence. sgRNA target and PAM sequence are indicated. (B) Histograms of PD-1 cell surface expression levels assessed by flow cytometry. All cells were treated with 100 pmol of PD-1 HDR template. PD-1 Cas9 RNP-treated cells are shown in blue, CXCR4 Cas9 RNP-treated cells in light grey and scrambled guide (no predicted cut within the human genome) Cas9 RNP-treated cells in dark grey. (C) Histograms of CXCR4 cell surface expression levels assessed by flow cytometry. All cells were treated with 100 pmol of CXCR4 HDR template. CXCR4 Cas9 RNP-treated cells are shown in first four rows, PD-1 Cas9 RNP-treated cells in the next four rows and scrambled guide Cas9 RNP-treated in the final two rows. Panels B and C show the results of 4 experiments with 2 differently *in vitro* transcribed and purified CXCR4 and PD-1 sgRNAs (see Supplementary Information Materials and Methods section of Example 4) tested in 2 different blood donors. For each blood donor, experiments done with phenol/chloroform extracted sgRNAs are shown on top and experiments with PAGE purified sgRNAs are shown below; scrambled guides were prepared for both experiments with phenol/chloroform extraction. Dotted line indicates gating on PD-1 high expressing or CXCR4 high expressing cells, respectively. The percentage of PD-1 high expressing cells was significantly lower with PD-1 Cas9 RNP treatment compared either CXCR4 Cas9 RNP treatment (p < 0.001) or scrambled guide Cas9 RNP treatment (p < 0.001). The percentage of CXCR4 high expressing cells was significantly lower with CXCR4 Cas9 RNP treatment compared to either PD-1 Cas9 RNP treatment (p < 0.001) or scrambled guide Cas9 RNP treatment (p < 0.001) (Pearson's chi-squared). (D) Genome editing was analyzed by T7E1 assay, whereas HDR was detected by HindIII digestion, which specifically cleaved the newly integrated HindIII site; cleavage products for both assays are indicated with arrowheads. Concentrations of various HDR templates are indicated above the agarose gels. CTRL HDR template refers to a scrambled version of the original CXCR4 HDR template including a HindIII restriction site. A non-specific second gel band of unclear significance was noted in the T7E1 of the *PD-1* amplicon under all conditions. Total editing and HDR frequencies were calculated and are displayed below agarose gel images.

### DETAILED DESCRIPTION

### I. Introduction

Delivery of nucleic acids, proteins, and complexes of proteins and nucleic acids to primary cells, such as primary hematopoietic cells or primary hematopoietic stem cells, can be limited by low efficiency. Described herein are methods and compositions for achieving surprisingly high efficiency delivery of Cas9 protein or Cas9 ribonucleoprotein complex to a primary cell or primary stem cell. Such high efficiency delivery of Cas9 or a ribonucleoprotein complex thereof can enable improved methods of genome editing, chromatin modification, gene regulation, cell differentiation, and control of cellular activity. In some embodiments, the high efficiency delivery of the Cas9 or a ribonucleoprotein complex thereof is performed in a primary hematopoietic cell or primary hematopoietic stem cell.

High efficiency delivery of Cas9 or Cas9 ribonucleoproteins to primary hematopoietic cells can be used, for instance, for genome editing, chromatin modification, gene regulation, cell differentiation, and control of the activity of immune cells, such as T cells. For example genome editing reagents, chromatin modifying reagents, or agents for modulating the expression of one or more genes can be delivered into a T cell. As another example, reagents that control T cell activity, differentiation, or dedifferentiation, can be delivered into a T cell. Such methods can be used to treat or prevent cancer, infectious diseases, or autoimmune diseases.

In some cases, the methods and compositions described herein can be used for generation, modification, use, or control of recombinant T cells, such as chimeric antigen receptor T cells (CAR T cells). Such CAR T cells can be used to treat or prevent cancer, infectious diseases, or autoimmune diseases. For example, in some embodiments, one or more gene products are knocked-in or knocked out in a cell modified to express a heterologous protein (e.g., a chimeric antigen receptor (CAR)). Exemplary gene products to knock out can include, e.g., PD-1. The CAR can be introduced by any method available, e.g., by viral (e.g., lentiviral) expression. The CAR vector can be introduced into the cell before, during, or after the genome of the cell is edited to knock in or knock out the gene product.

### I. Methods

Methods for delivery of Cas9 protein to primary cells can include providing a reaction mixture comprising a Cas9 nuclease domain and introducing the Cas9 nuclease domain inside the cell. In some cases, the method includes providing a reaction mixture comprising a Cas9 ribonucleoprotein complex and the cell and b) introducing the Cas9 ribonucleoprotein complex inside the cell. In some cases, the Cas9 ribonucleoprotein complex comprises a Cas9 nuclease domain and a guide RNA (e.g., small guide RNA). The guide RNA can be configured to specifically hybridize to a target region of the genome of the cell.

In some cases, a plurality of structurally different ribonucleoprotein complexes is introduced into the cell. For example a Cas9 protein can be complexed with a plurality (e.g., 2, 3, 4, 5, or more, e.g., 2-10, 5-100, 20-100) of structurally different guide RNAs to target a plurality of structurally different target genomic regions. As another example, a plurality of structurally different Cas9 proteins (*e.g*., 2, 3, 4, 5, or more) can be complexed with a guide RNA, or a plurality of structurally different guide RNAs to introduce a plurality of different effector functions into the cell. In some cases, the Cas9 ribonucleoprotein complexes are formed separately, such that a selected Cas9 effector function (*e.g*., genome editing, transcription modulation, etc.) can be coupled with a selected guide RNA and thus targeted to a selected target genomic region. Once formed, the plurality of structurally different Cas9 ribonucleoproteins can be provided in a reaction mixture containing a cell and introduced into the cell as described herein.

In some embodiments, the methods described herein provide an efficiency of delivery of the Cas9 or Cas9 ribonucleoprotein complex of at least about 20%, 25%, 30%, 35%, 40%, 45%, 50%, 55%, 60%, 65%, 70%, 75%, 80%, 85%, 90%, 95%, 97.5%, 99%, 99.5%, 99%, or higher. In some embodiments, the methods described herein provide an efficiency of delivery of the Cas9 or Cas9 ribonucleoprotein complex of from about 20% to about 99%, from about 30% to about 90%, from about 35% to about 85% or 90% or higher, from about 40% to about 85% or 90% or higher, from about 50% to about 85% or 90% or higher, from about 50% to about 85% or 90% or higher, from about 60% to about 85% or 90% or higher, or from about 70% to about 85% or 90% or higher. In some cases, the efficiency is determined with respect to cells that are viable after the introducing of the Cas9 or Cas9 ribonucleoprotein into the cell. In some cases, the efficiency is determined with respect to the total number of cells (viable or non-viable) to which the introducing of the Cas9 or Cas9 ribonucleoprotein into the cell.

Methods for determining efficiency of delivery include, but are not limited to one or more of the following: detection of a detectable label fused, or otherwise attached, to Cas9, a guide RNA, or a Cas9 ribonucleoprotein complex. For example, the Cas9 or guide RNA can be fused to a fluorescent label, the internalization of which into a cell can be detected by means known in the art. As another example, guide RNA can be detected by lysing the cell, amplifying the guide RNA, and detecting the amplified guide RNA. In some cases, the amplification includes a reverse transcription step to produce guide cDNA, and the guide cDNA is amplified and detected.

As another example, the efficiency of delivery can be determined by detecting a downstream effect of the Cas9 or Cas9 ribonucleoprotein complex. For example, delivery can be estimated by quantifying the number of genome edited cells or genome edited alleles in a population of cells (as compared to total cells/alleles or total viable cells obtained after the introducing step). Various methods for quantifying genome editing can be utilized. These methods include, but are not limited to, the use of a mismatch-specific nuclease, such as T7 endonuclease I; sequencing of one or more target loci (*e.g*., by sanger sequencing of cloned target locus amplification fragments); tracking of indels by decomposition (TIDE); and high-throughput deep sequencing.

In the T7 enndonuclease I assay, a plurality of cells that contain a fraction of edited cells is harvested, the genomic DNA is extracted, the target genomic region amplified, and the amplicons are hybridized. The edited genomic DNA amplicons will form mismatched hybrid structures with wild-type DNA amplicons. The DNA is digested with a mismatch specific nuclease that cleaves double stranded DNA containing one or more mismatched base pairs. The extent of cleavage can be assayed to determine editing efficiency. Alternative approaches for quantification of editing efficiency can include quantitative PCR or digital PCR. In some cases, the number of edited cells can be lower than the number of cells to which delivery has been achieved due to downstream inefficiencies in binding to, or cleavage of target genomic regions, or inefficiencies in the detection of editing events. Similarly, the number of cells exhibiting transcriptional modulation or chromatin modification when the delivered Cas9 protein is a fusion with an effector domain providing such activity can be lower than the number of cells to which the delivery has been achieved. As such, the efficiency of a detected downstream effect can be considered as a lower limit of delivery efficiency.

In some cases, the methods described herein provide for high cell viability of cells to which the Cas9 or Cas9 ribonucleoprotein has been introduced into the cell. In some cases, the high viability is achieved by the formation in the extracellular membrane of a limited number of pores having a short lifetime. In some cases, the viability of the cells to which the Cas9 or Cas9 ribonucleoprotein has been introduced into the cell is at least about 20%, 25%, 30%, 35%, 40%, 45%, 50%, 55%, 60%, 65%, 70%, 75%, 80%, 85%, 90%, 95%, 97.5%, 99%, 99.5%, 99%, or higher. In some cases, the viability of the cells to which the Cas9 or Cas9 ribonucleoprotein has been introduced into the cell is from about 20% to about 99%, from about 30% to about 90%, from about 35% to about 85% or 90% or higher, from about 40% to about 85% or 90% or higher, from about 50% to about 85% or 90% or higher, from about 50% to about 85% or 90% or higher, from about 60% to about 85% or 90% or higher, or from about 70% to about 85% or 90% or higher.

In some cases, the cell to which the Cas9 protein is delivered does not otherwise contain nucleic acid encoding Cas9. In some cases, the cell to which the Cas9 protein is delivered does not contain nucleic acid encoding a Cas9 protein that is structurally identical to the delivered Cas9 protein. In such cases, determination of delivery efficiency can be with respect to the number of cells in which the structurally distinct delivered Cas9 protein has been introduced, not the number of cells that have any Cas9 protein. In some cases, the cell to which the Cas9 is delivered does not contain DNA encoding a guide RNA. For example, the Cas9, in the form of a Cas9 ribonucleoprotein complex can be introduced into a cell that does not contain DNA encoding a guide RNA, does not contain DNA encoding a Cas9 protein, and/or does not contain DNA encoding a Cas9 protein structurally identical to the delivered Cas9 protein in the ribonucleoprotein complex.

### A. Introducing Cas9 or Cas9 ribonucleoprotein into a cell

Methods for introducing Cas9 or Cas9 ribonucleoprotein complex into a cell *(e.g.,* a hematopoietic cell or hematopoietic stem cell, including, e.g., such cells from humans) include forming a reaction mixture containing the Cas9 or Cas9 ribonucleoprotein complex and introducing transient holes in the extracellular membrane of the cell. Such transient holes can be introduced by a variety of methods, including, but not limited to, electroporation, cell squeezing, or contacting with nanowires or nanotubes. Generally, the transient holes are introduced in the presence of the Cas9 or Cas9 ribonucleoprotein complex and the Cas9 or Cas9 ribonucleoprotein complex allowed to diffuse into the cell.

Methods, compositions, and devices for electroporating cells to introduce a Cas9 or Cas9 ribonucleoprotein complex can include those described in the examples herein. Additional or alternative methods, compositions, and devices for electroporating cells to introduce Cas9 or Cas9 ribonucleoprotein complex can include those described in WO/2006/001614 or Kim, J.A. et al. Biosens. Bioelectron. 23, 1353-1360 (2008). Additional or alternative methods, compositions, and devices for electroporating cells to introduce Cas9 or Cas9 ribonucleoprotein complex can include those described in U.S. Patent Appl. Pub. Nos. 2006/0094095; 2005/0064596; or 2006/0087522. Additional or alternative methods, compositions, and devices for electroporating cells to introduce Cas9 or Cas9 ribonucleoprotein complex can include those described in Li, L.H. et al. Cancer Res. Treat. 1, 341-350 (2002); U.S. Patent Nos.: 6,773,669; 7,186,559; 7,771,984; 7,991,559; 6485961; 7029916; and U.S. Patent Appl. Pub. Nos: 2014/0017213; and 2012/0088842. Additional or alternative methods, compositions, and devices for electroporating cells to introduce Cas9 or Cas9 ribonucleoprotein complex can include those described in Geng, T. et al.. J. Control Release 144, 91-100 (2010); and Wang, J., et al. Lab. Chip 10, 2057-2061 (2010).

In some cases, the methods or compositions described in the patents or publications cited herein are modified for Cas9 or Cas9 ribonucleoprotein delivery. Such modification can include increasing or decreasing voltage, pulse length, or the number of pulses. Such modification can further include modification of buffers, media, electrolytic solutions, or components thereof. Electroporation can be performed using devices known in the art, such as a Bio-Rad Gene Pulser Electroporation device, an Invitrogen Neon transfection system, a MaxCyte transfection system, a Lonza Nucleofection device, a NEPA Gene NEPA21 transfection device, a flow though electroporation system containing a pump and a constant voltage supply, or other electroporation devices or systems known in the art.

In an exemplary embodiment, the electroporation is performed with a device having a long distance between the cathode and anode. In some cases, the distance between the cathode and anode is 3, 4, 5, 6, 7, 8, 9, 10, 11, 12, 13, 14, 15,16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 mm. In some cases, the device is configured with an electrode having a relatively small surface area in contact with the reaction mixture containing the cell. In some cases, the surface area of at least one of the electrodes, or the surface area of at least one of the electrodes that is in contact with the reaction mixture is, or is about, 0.1 mm², 0.2 mm², 0.3 mm², 0.33 mm², 0.4 mm², 0.5 mm², 0.6 mm², 0.7 mm², 0.8 mm², 0.9 mm², or 1 mm². In some cases, the ratio of the distance between the cathode and anode and the electrode surface area is from 1/50 to 1/1000. In some cases, the ratio of the length of the long axis of the electroporation chamber to the cross sectional area of the electroporation chamber is from 50 to 10,000. In some cases, the electroporation device has an electroporation chamber with a first and second distal end separated by the longitudinal length, where the first electrode is at the first distal end and a reservoir containing the second electrode is in fluid communication with the second distal end.

In another exemplary embodiment, the electroporation is performed with a Lonza 4D Nucleofector^{™} device. For example, electroporation can be performed with the Amaxa P3 primary cell 96-well Nucleofector^{™} kit or P3 primary cell 4D-Nucleofector X kit S. In some cases, the electroporation is performed by resuspending cells in a suitable electroporation buffer (*e.g*., Amaxa buffer P3 with buffer supplement), placing the cells in an electroporation chamber, and electroporating the cells. In some cases, activated T cells can be electroporated with a Nucleofector^{™} device using any one of the following programs: EH-115, CA-137, DS-150, CM-138, DS-120, CM-137, EH-100, CM-150, EO-100, DN-100, EN-138, DS-138, EN-150, DS-137, EW-113, or DS-130. In some cases, activated T cells can be electroporated with a Nucleofector^{™} device using the EH-115 program. In some cases, naive T cells can be electroporated with a Nucleofector^{™} device using any one of the following programs: EH-100, DN-100, EO-100 EN-138, EW-113, or EN-150. In some cases, naive T cells can be electroporated with a Nucleofector^{™} device using the EH-100 or DN-100 program.

The electroporation can be performed by positioning a reaction mixture containing Cas9 or a Cas9 ribonucleoprotein and a cell into a chamber between a cathode and an anode and applying a voltage potential between the cathode and the anode. The voltage potential can be from about 20 kV/m to about 100 kV/m. In some cases, the voltage potential is from about 30 kV/m to about 90 kV/m, from about 30 kV/m to about 80 kV/m, from about 30 kV/m to about 70 kV/m, from about 30 kV/m to about 60 kV/m, from about 40 kV/m to about 60 kV/m, from about 45 to about 55 or 60 kV/m, or from about 50 to about 55 kV/m. In some cases, the voltage potential is at least about 20 kV/m, 30 kV/m, 40 kV/m, 50 kV/m, 53 kV/m, 60 kV/m, 70 kV/m, 80 kV/m, 90 kV/m, or 100 kV/m. In some cases, the voltage potential is, or is about, 0.5, 0.75, 1, 1.2, 1.3, 1.4, 1.5, 1.6, 1.7, 1.8, 1.9, 2, 2.1, 2.2, 2.3, 2.4, or 2.5 kV. In some cases, the voltage potential is from about 0.5 to about 2 kV, from about 0.75 to about 2 kV, from about 1 to about 2 kV, from about 1.1 to about 1.9 kV, form about 1.2 to about 1.8 kV, from about 1.3 to about 1.7 kV, from about 1.4 to about 1.7 kV, or from about 1.5 to about 1.7 kV.

The voltage potential can be applied as a pulse or continuously. For continuous voltage application, the reaction mixture can be flowed through an electrode chamber using a pump or other liquid handling apparatus. In some cases, the reaction mixture is flowed through the electrode chamber once. Alternatively, the reaction mixture can be recirculated through the electrode chamber. For pulse voltage application, the pulse length, number of pulses, and duration between pulses can be optimized to achieve high efficiency delivery of Cas9 or Cas9 ribonucleoprotein complex.

The voltage potential can be applied as a pulse once, or multiple times. In some cases, the voltage potential is pulsed from 1 to 10 times, from 1 to 9 times, from 1 to 8 times, from 1 to 7 times, from 1 to 6 times, from 1 to 5 times, or from 1 to 4 times. In some cases, the voltage potential is pulsed from 2 to 9 times, from 2 to 8 times, from 2 to 7 times, from 2 to 6 times, from 2 to 5 times, or from 2 to 4 times. In some cases, the voltage potential is pulsed 1, 2, 3, 4, 5, 6, 7, 8, 9, or 10 times.

The voltage potential pulse length can be from 1 to 100 ms, from 2 to 90 ms, from 3 to 80 ms, from 4 to 70 ms, from 5 to 60 ms, from 5 to 50 ms, from 5 to 40 ms, from 6 to 30 ms, from 7 to 20 ms, or from 8 to 15 ms. In some cases, the pulse length is, or is about, 1, 2, 3, 4, 5, 6, 7, 8, 9, 10, 11, 12, 13, 14, 15, 20, 25, 30, 35, 40, 45, 50, 55, 60, 70, 75, 80, 85, 90, 95, or 100 ms.

In some cases, the voltage pulses are interspersed with rest periods of a defined duration. In some cases the rest period is of a length identical to any of the foregoing pulse lengths described herein. In some cases, the rest period is significantly longer than the pulse length. For example, a reaction mixture can be subject to a voltage pulse, recovered for 1, 2, 5, 10, 15, 20, or 30 minutes, or longer, and a voltage potential reapplied. In some cases, the magnitude, duration, or rest period for the multiple voltage pulses is variable. For example, the first pulse can be of higher voltage potential, or longer duration, than a second pulse, or vice versa.

Methods, compositions, and devices for the use of nanowires or nanotubes to introduce a Cas9 or Cas9 ribonucleoprotein complex can include those described in Proc Natl Acad Sci U S A. Feb 2, 2010; 107(5): 1870-1875; U.S. Patent Appl. Publ. Nos. 2012/0094382; and 2013/0260467; and WO/2014/031173. Generally, the Cas9 protein or Cas9 ribonucleoprotein complex is coated onto one or more nanowires or nanotubes and brought into contact with a cell in a reaction mixture. The nanowires or nanotubes can pierce the cellular membrane and thereby deliver the Cas9 protein or Cas9 ribonucleoprotein complex.

Methods, compositions, and devices for squeezing or deforming a cell to introduce a Cas9 or Cas9 ribonucleoprotein complex can include those described herein. Additional or alternative methods, compositions, and devices can include those described in Nano Lett. 2012 Dec 12;12(12):6322-7; Proc Natl Acad Sci U S A. 2013 Feb 5;110(6):2082-7; J Vis Exp. 2013 Nov 7;(81):e50980; and Integr Biol (Camb). 2014 Apr;6(4):470-5. Additional or alternative methods, compositions, and devices can include those described in U.S. Patent Appl. Publ. No. 2014/0287509. Generally, the Cas9 protein or Cas9 ribonucleoprotein complex is provided in a reaction mixture containing the cell and the reaction mixture is forced through a cell deforming orifice or constriction. In some cases, the constriction is smaller than the diameter of the cell. In some cases, the constriction contains cell-deforming components such as regions of strong electrostatic charge, regions of hydrophobicity, or regions containing nanowires or nanotubes. The forcing can introduce transient pores into a cell membrane of the cell allowing the Cas9 or Cas9 ribonucleoprotein complex to enter the cell through the transient pores. In some cases, squeezing or deforming a cell to introduce Cas9 or a Cas9 ribonucleoprotein can be effective even when the cell is in a non-dividing state.

Methods for introducing Cas9 or Cas9 ribonucleoprotein complex into a cell include forming a reaction mixture containing the Cas9 or Cas9 ribonucleoprotein complex and contacting the cell with the Cas9 or Cas9 ribonucleoprotein complex to induce receptor-mediated internalization. Compositions and methods for receptor mediated internalization are described, *e.g.,* in Wu et al., J. Biol. Chem. 262, 4429-4432 (1987); and Wagner et al., Proc. Natl. Acad. Sci. USA 87, 3410-3414 (1990). Generally, the receptor-mediated internalization is mediated by interaction between a cell surface receptor and a ligand fused to the Cas9 or fused to the Cas9 ribonucleoprotein complex (*e.g*., covalently attached or fused to a guide RNA in the Cas9 ribonucleoprotein complex). The ligand can be any protein, small molecule, polymer, or fragment thereof that binds to, or is recognized by, a receptor on the surface of the cell. An exemplary ligand is an antibody or an antibody fragment (*e.g.*, scFv).

For example, the Cas9 protein can be a fusion between a Cas9 nuclease domain and an anti-CD3 scFv. The scFv can bind to the T cell co-receptor CD3, which is expressed on T cells, and induce receptor-mediated internalization of a Cas9 or Cas9 ribonucleoprotein complex. Other suitable receptor targets include, but are not limited to any cell surface protein on the target cell. In some cases, the suitable receptor target is a heterologous receptor expressed on the surface of the target cell *(e.g.,* a receptor generated by introduction of a recombinant nucleic acid into the target cell). In the case of delivery to T cells, the receptor can be any cell surface protein on the surface of the T cell such as CD28, CTLA-4, PD-1, an integrin, a lectin receptor, a cytokine receptor, or a chemokine receptor. In the case of delivery to other immune cells, such as macrophages, dendritic cells, monocytes, *etc.,* the receptor can be a cell surface protein on the surface of the target macrophage, dendritic cell, monocyte, *etc.*

In some cases, the fusion is a cleavable fusion. In some cases, the fusion is a cleavable fusion that is cleaved at the cell surface or upon receptor mediated internalization. For example, the fusion can contain a linker between the ligand and a Cas9 protein, which linker is a peptide containing one or more cleavage sites for intracellular or membrane bound proteases. As another example, the fusion can contain an ester linkage, which linkage is labile in the presence of one or more membrane-bound or intracellular esterases.

In some cases, the Cas9 or Cas9 ribonucleoprotein complex is conjugated to a ligand for receptor-mediated internalization *in vitro.* Various in vitro methods for conjugating small molecule, peptide, and polymer ligands to proteins or nucleic acids are known in the art. For example, the Cas9 nuclease can be fused to a sortase recognition site (*e.g*., LPXTG) at the C-terminus, and the ligand can contain an oligo-glycine motif with a free N-terminus. Upon addition of sortase to the Cas9-ligand mixture or Cas9 ribonucleoprotein and ligand mixture, the Cas9 protein and ligand are covalently linked through a native peptide bond.

In some embodiments, the Cas9 or Cas9 ribonucleoprotein complex is introduced into the cell with a cell penetrating peptide. In some cases, the cell penetrating peptide induces receptor-mediated internalization. In other cases, the cell penetrating peptide penetrates the cellular membrane. In some cases, the cell penetrating peptide forms a transitory inverted micelle structure for entering the cell. One or more cell penetrating peptides, or one or more copies of a cell penetrating peptide, or a combination thereof, can be fused to Cas9, a Cas9 ribonucleoprotein complex, or to a guide RNA.

Exemplary cell penetrating peptides can include HIV TAT, TAT2-M1, MPG, PEP-1, penetratin, transportan, poly-arginine, CADY, or derivatives, analogues, and mutants thereof. Exemplary cell penetrating peptides can further include those described in U.S. Patent Nos. 8575305; 8772449; 8389481; 8691528; 8372951; or 8614194. Small molecule mimics of cell penetrating peptides can also be used in the methods described herein to deliver Cas9 or Cas9 ribonucleoprotein, such as those described in Nature Methods, 4 (2). pp. 153-159 (2007).

The cells (*e.g.,* T cells) can be stimulated (*e.g*., by contact with soluble or solid surface immobilized anti-CD3 antibodies, anti-CD28 antibodies, or a combination thereof) or unstimulated prior to one of the Cas9 or Cas9 RNP introduction methods described herein (*e.g.,* electroporation). In some cases, the cells (*e.g.,* T cells) can be stimulated (*e.g.,* by contact with soluble or solid surface immobilized anti-CD3 antibodies, anti-CD28 antibodies, or a combination thereof) or incubated without stimulation after one of the Cas9 or Cas9 RNP introduction methods described herein (*e.g.*, electroporation). In some cases, an appropriate cytokine (*e.g*., IL-2) can be contacted with the cells prior to mixing with Cas9 or Cas9 RNP introduction reagents (*e.g*., electroporation buffer), or contacted with the cells after Cas9 or Cas9 RNP introduction, or a combination thereof.

### B. Cas9

The delivered Cas9 protein, whether as an apo protein or in complex with RNA, can be in an active endonuclease form, such that when bound to target nucleic acid as part of a complex with a guide RNA, a double strand break is introduced into the target nucleic acid. The double strand break can be repaired by NHEJ to introduce random mutations, or HDR to introduce specific mutations. Various Cas9 nucleases can be utilized in the methods described herein. For example, a Cas9 nuclease that requires an NGG protospacer adjacent motif (PAM) immediately 3' of the region targeted by the guide RNA can be utilized. Such Cas9 nucleases can be targeted to any region of a genome that contains an NGG sequence. As another example, Cas9 proteins with orthogonal PAM motif requirements can be utilized to target sequences that do not have an adjacent NGG PAM sequence. Exemplary Cas9 proteins with orthogonal PAM sequence specificities include, but are not limited to, CFP1, those described in Nature Methods 10, 1116-1121 (2013), and those described in Zetsche et al., Cell, Volume 163, Issue 3, p759-771, 22 October 2015.

In some cases, the Cas9 protein is a nickase, such that when bound to target nucleic acid as part of a complex with a guide RNA, a single strand break or nick is introduced into the target nucleic acid. A pair of Cas9 nickases, each bound to a structurally different guide RNA, can be targeted to two proximal sites of a target genomic region and thus introduce a pair of proximal single stranded breaks into the target genomic region. Nickase pairs can provide enhanced specificity because off-target effects are likely to result in single nicks, which are generally repaired without lesion by base-excision repair mechanisms. Exemplary Cas9 nickases include Cas9 nucleases having a D10A or H840A mutation.

In some cases, the Cas9 protein is in a nuclease inactive form. For example, the Cas9 protein can be in a nuclease inactive form that is fused to another accessory protein or effector domain. Thus, the Cas9 nuclease, in complex with a guide RNA, can function to target the accessory protein, effector domain, or the activity thereof, to the target genomic region. In some cases, the nuclease inactive Cas9 protein is fused to an endonuclease or nickase. For example, the nuclease inactive Cas9 can be fused to an obligate heterodimer endonuclease or nickase (*e.g.,* an obligate heterodimer of Fok I endonuclease). A pair of such nuclease inactive endonucleases fused to corresponding members of an obligate heterodimer nuclease can be used to localize endonuclease activity at a target genomic region with enhanced specificity. Exemplary Cas9 heterodimer endonuclease fusions include those described in Nat Biotechnol. Jun 2014; 32(6): 577-582.

In some cases, a Cas9 protein, such as a nuclease inactive Cas9 protein can be used to modulate gene expression or modify chromatin structure. In some cases, a nuclease inactive Cas9 protein can form a complex with a guide RNA targeted to a gene or the promoter of a gene. The nuclease inactive Cas9 protein can thereby interfere with binding of transcription factors or other transcription machinery and thus down-regulate transcription of the target gene. The use of multiple structurally different guide RNAs targeting the same gene or promoter region, or a combination thereof, can be used to further decrease transcription of the target gene.

As another example, a Cas9 protein, such as a nuclease inactive Cas9 protein can be fused to a transcription activator or repressor to modulate transcription of a target gene. Exemplary activators include, but are not limited to, one or more copies of a VP8, VP16, VP64, or a p65 activation domain (p65AD). Exemplary repressors include, but are not limited to, a KRAB domain, a chromoshadow domain, a SID domain, or an EAR-repression domain (SRDX). The transcriptional activator or repressor can be optimized for efficient activity or enhanced stability in the host cell.

In some cases, the Cas9 nuclease, such as a nuclease inactive Cas9 nuclease can be fused to one or more effector domains that regulate DNA methylation, histone methylation or demethylation, histone deacetylation, RNA polII phosphorylation, or promote an increase in nucleosome compaction as measured by reduced DNAse I hypersensitivity or decreased micrococcal nuclease accessibility. A combination of activation effector domains or enzymes which could promote transcription could include DNA demethylases, histone demethylases or methylases, histone acetylases, RNA polII phosphorylases, or enzymes or effector domains that reduce nucleosome compaction as measured by increased DNAse I hypersensitivity or increase micrococcal nuclease accessibility, or promote natural or un-natural chromosomal looping between distal enhancer elements and proximal promoter elements. A combination of repressor effector domains or enzymes which could repress transcription could include DNA methylases, histone demethylases or methylases, histone de-acetylases, RNA polII de-phosphorylases, or enzymes or effector domains that increase nucleosome compaction as measured by decreased DNAse I hypersensitivity or decreased micrococcal nuclease accessibility, or inhibit chromosomal looping between distal enhancer elements and proximal promoter elements.

Cas9 nuclease can be fused to one or more nuclear translocation sequences. The use N-terminal, C-terminal, or internal nuclear translocation sequences, or one or more nuclear translocation sequences fused to a domain or accessory protein that is fused to a Cas9 nuclease can enhance delivery of the Cas9 or Cas9 ribonucleoprotein complex to the nucleus of the cell. Directing the delivery of the Cas9 or Cas9 ribonucleoprotein complex to the nucleus of the cell can increase the level of genome editing or transcriptional control provided by introducing the Cas9 or Cas9 ribonucleoprotein into the cell.

The reaction mixture for introducing the Cas9 or Cas9 ribonucleoprotein complex into the cell can have a concentration of Cas9 or Cas9 ribonucleoprotein of from about 0.25 µM to about 5 µM, from about 0.5 µM to about 2.5 µM, or from about 0.9 µM to about 1.8 µM. The concentration of the Cas9 or Cas9 ribonucleoprotein complex can be at a concentration of, or be at a concentration of about, 0.25 µM, 0.3 µM, 0.4 µM, 0.5 µM, 0.6 µM, 0.7 µM, 0.8 µM, 0.9 µM, 1 µM, 1.1 µM, 1.2 µM, 1.3 µM, 1.4 µM, 1.5 µM, 1.6 µM, 1.7 µM, 1.8 µM, 1.9 µM, 2 µM, 2.1 µM, 2.2 µM, 2.3 µM, 2.4 µM, 2.5 µM, or higher. In some cases, the concentration of the Cas9 or Cas9 ribonucleoprotein complex is less than, or less than about, 5 µM, 4 µM, or 3 µM.

The reaction mixture for introducing the Cas9 or Cas9 ribonucleoprotein complex into the cell can contain from about 1 x 10⁵ to about 4 x 10⁵ target cells, from about 1.5 x 10⁵ to about 3.5 x 10⁵ target cells, from about 1.75 x 10⁵ to about 3 x 10⁵ target cells, or from about 2 x 10⁵ to about 2.5 x 10⁵ target cells. In some cases, the concentration of the cells in the reaction mixture is from about 0.5 x 10⁴ to about 5 x 10⁴ target cells per µL, from about 0.75 x 10⁴ to about 4 x 10⁴ target cells per µL, from about 1 x 10⁴ to about 3 x 10⁴ target cells per µL, from about 1.5 x 10⁴ to about 2.5 x 10⁴ or 3 x 10⁴ target cells per µL, or from about 1.8 x 10⁴ to about 2.3 x 10⁴ target cells per µL.

### C. Template nucleic acids

In some embodiments, the reaction mixture for introducing the Cas9 or Cas9 ribonucleoprotein complex into the cell can contain a nucleic acid for directing homology directed repair (HDR) of Cas9 mediated, or Cas9 fusion mediated, cleavage or nicking at the target genomic region. The template nucleic acid is generally a double or single-stranded DNA oligonucleotide. In some cases, the template nucleic acid is a single stranded oligonucleotide DNA template (ssODT).

The template nucleic acid can contain from 15 bases (b) or base pairs (bp) to about 5 kilobases (kb) or kilobase pairs (kbp) in length (e.g., from about 50, 75, or 100 b or bp to about 110, 120, 125, 150, 200, 225, or 250 b or bp in length). Generally longer template nucleic acids are provided in the form of a circular or linearized plasmid or as a component of a vector (e.g., as a component of a viral vector), or an amplification or polymerization product thereof. Shorter template nucleic acids can be provided as single or double stranded oligonucleotides. Exemplary single or double-stranded template oligonucleotides are, or are least about 15, 16, 17, 18, 19, 20, 21, 22, 23, 24, 25, 26, 27, 28, 29, 30, 31, 32, 33, 34, 35, 36, 37, 38,39, 40, 41, 42, 43, 44, 45,46, 47, 48,49, 50, 51, 52, 53, 54,55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 115, 120, 125, 150, 175, 200, 225, or 250 b or bp in length. Such template oligonucleotides can contain one or two (*e.g.,* flanking homology arms) homology arms that are identical or substantially identical to a region adjacent to or flanking the target cut site. In some cases, the homology arm(s) are from 25 to about 90 nucleotides in length. For example, the homology arm(s) can be about 25, 30, 35, 40, 45, 50, 55, 60, 65, 70, 75, 80, 85, 90, 95, 100, 110, 115, or 120 nucleotides in length.

The template nucleic acid can be provided in the reaction mixture for introduction into the cell at a concentration of from about 1 µM to about 200 µM, from about 2 µM to about 190 µM, from about 2 µM to about 180 µM, from about 5 µM to about 180 µM, from about 9 µM to about 180 µM, from about 10 µM to about 150 µM, from about 20 µM to about 140 µM, from about 30 µM to about 130 µM, from about 40 µM to about 120 µM, or from about 45 or 50 µM to about 90 or 100 µM. In some cases, the template nucleic acid can be provided in the reaction mixture for introduction into the cell at a concentration of, or of about, 1 µM, 2 µM, 3 µM, 4 µM, 5 µM, 6 µM, 7 µM, 8 µM, 9 µM, 10 µM, 11 µM, 12 µM, 13 µM, 14 µM, 15 µM, 16 µM, 17 µM, 18 µM, 19 µM, 20 µM, 25 µM, 30 µM, 35 µM, 40 µM, 45 µM, 50 µM, 55 µM, 60 µM, 70 µM, 80 µM, 90 µM, 100 µM, 110 µM, 115 µM, 120 µM, 130 µM, 140 µM, 150 µM, 160 µM, 170 µM, 180 µM, 190 µM, 200 µM, or more.

In some cases, the efficiency of template directed and NHEJ genome editing in the presence of a template nucleic acid (*e.g*., ssODT) can be at least, or at least about, 20%, 25%, 30%, 35%, 40%, 45%, 50%, 55%, 60%, 65% 70%, 75%, 80%, 85%, 90%, 95%, 99%, or higher. In some cases, the efficiency of incorporation of the sequence of the template nucleic acid (*e.g*., ssODT) by HDR can be at least, or at least about, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65% 70%, 75%, 80%, 85%, 90%, 95%, 99%, or higher.

The template nucleic acid can contain a wide variety of different sequences. In some cases, the template nucleic acid encodes a stop codon, or frame shift, as compared to the target genomic region prior to cleavage and HDR. Such a template nucleic acid can be useful for knocking out or inactivating a gene or portion thereof. In some cases, the template nucleic acid encodes one or more missense mutations or in-frame insertions or deletions as compared to the target genomic region. Such a template nucleic acid can be useful for altering the expression level or activity (*e.g*., ligand specificity) of a target gene or portion thereof.

For example, the template nucleic acid can be used to replace one or more complementary determining regions, or portions thereof, of a T cell receptor chain or antibody gene. Such a template nucleic acid can thus alter the antigen specificity of a target cell. For instance, the target cell can be altered to recognize, and thereby elicit an immune response against, a tumor antigen or an infectious disease antigen.

As another example, the template nucleic acid can encode a wild-type sequence for rescuing the expression level or activity of a target endogenous gene or protein. For instance, T cells containing a mutation in the FoxP3 gene, or a promoter region thereof, can be rescued to treat X-linked IPEX or systemic lupus erythematous. Alternatively, the template nucleic acid can encode a sequence that results in lower expression or activity of a target gene. For example, an increased immunotherapeutic response can be achieved by deleting or reducing the expression or activity of FoxP3 in T cells prepared for immunotherapy against a cancer or infectious disease target.

As another example, the template nucleic acid can encode a mutation that alters the function of a target gene. For instance the template nucleic acid can encode a mutation of a cell surface protein necessary for viral recognition or entry. The mutation can reduce the ability of the virus to recognize or infect the target cell. For example, mutations of CCR5 or CXCR4 can confer increased resistance to HIV infection in CD4⁺ T cells.

In some cases, the template nucleic acid encodes a sequence that, although adjacent to or flanked by a sufficient region of homology, is entirely orthogonal to the endogenous sequence. For example, the template nucleic acid can encode an inducible promoter or repressor element unrelated to the endogenous promoter of a target gene. The inducible promoter or repressor element can be inserted into the promoter region of a target gene to provide temporal and/or spatial control of the target gene expression or activity. As another example, the template nucleic acid can encode a suicide gene, a reporter gene, or a rheostat gene, or a portion thereof. A suicide gene can be used to remove antigen specific immunotherapy cells from a host after successful treatment. A rheostat gene can be used to modulate the activity of an immune response during immunotherapy. A reporter gene can be used to monitor the number, location, and activity of cells *in vitro* or *in vivo* after introduction into a host.

Exemplary rheostat genes are immune checkpoint genes. An increase or decrease in expression or activity of one or more immune checkpoint genes can be used to modulate the activity of an immune response during immunotherapy. For example, an immune checkpoint gene can be increased in expression resulting in a decreased immune response. Alternatively, the immune checkpoint gene can be inactivated, resulting in an increased immune response. Exemplary immune checkpoint genes include, but are not limited to, CTLA-4, and PD-1. Additional rheostat genes can include any gene that modulates proliferation or effector function of the target cell. Such rheostate genes include transcription factors, chemokine receptors, cytokine receptors, or genes involved in co-inhibitory pathways such as TIGIT or TIMs. In some cases the rheostat gene is a synthetic or recombinant rheostat gene that interacts with the cell signaling machinery. For example, the synthetic rheostat gene can be a drug-dependent or light-dependent molecule that inhibits or activates cell signaling. Such synthetic genes are described in, *e.g.,* Cell 155(6):1422-34 (2013); and Proc Natl Acad Sci U S A. 2014 Apr 22;111(16):5896-901.

Exemplary suicide genes include, but are not limited to, thymidine kinase, herpes simplex virus type 1 thymidine kinase (HSV-tk), cytochrome P450 isoenzyme 4B1 (cyp4B1), cytosine deaminase, human folylpolyglutamate synthase (fpgs), or inducible casp9. In some embodiments, the suicide gene is chosen from the group consisting of the gene encoding the HSV-1 thymidine kinase (abbreviated to HSV-tk), the splice- corrected HSV-tk (abbreviated to cHSV-tk, see Fehse B et al., Gene Ther (2002) 9(23): 1633- 1638), the genes coding for the highly Gancyclovir-sensitive HSV-tk mutants (mutants wherein the residue at position 75 and/or the residue at position 39 are mutated (see Black ME et al. Cancer Res (2001) 61(7):3022-3026; and Qasim W et al., Gene Ther (2002) 9(12) :824-827). Suicide genes other than thymidine kinase based gene can be used instead. For instance, genes coding for human CD20 (the target of clinical-grade monoclonal antibodies such as Rituximab^{®}; see Serafini M et al., Hum Gene Ther. 2004;15:63-76.), inducible caspases (as an example: modified human caspase 9 fused to a human FK506 binding protein (FKBP) to allow conditional dimerization using a small molecule pharmaceutical; see Di Stasi A et al., N Engl J Med. 2011 Nov 3; 365 (18): 1673-83; Tey SK et al., Biol Blood Marrow Transplant. 2007 Aug;) '3(8):9) '3-24. Epub 2007 May 29) and FCU1 (that transforms a non-toxic prodrug 5-fluorocytosine or 5-FC to its highly cytotoxic derivatives 5-fluorouracil or 5-FU and 5'-fluorouridine-5'monophosphate or 5'-FUMP; Breton E et al., C R Biol. 2010 Mar;333(3):220-5. Epub 2010 Jan 25.) can be used as suicide gene.

In some embodiments, the template nucleic acid encodes a recombinant antigen receptor, a portion thereof, or a component thereof. Recombinant antigen receptors, portions, and components thereof include those described in U.S. Patent Appl. Publ. Nos. 2003/0215427; 2004/0043401; 2007/0166327; 2012/0148552 ;2014/0242701; 2014/0274909; 20140314795; 2015/0031624; and International Appl. Publ. Nos.: WO/2000/023573; and WO/2014/134165. Such recombinant antigen receptors can be used for immunotherapy targeting a specific tumor associated or infectious disease associated antigen. In some cases, the methods described herein can be used to knockout an endogenous antigen receptor, such as a T cell receptor, B cell receptor, or a portion, or component thereof. The methods described herein can also be used to knockin a recombinant antigen receptor, a portion thereof, or a component thereof. In some embodiments, the endogenous receptor is knocked out and replaced with the recombinant receptor (*e.g*., a recombinant T cell Receptor or a recombinant chimeric antigen receptor). In some cases, the recombinant receptor is inserted into the genomic location of the endogenous receptor. In some cases, the recombinant receptor is inserted into a different genomic location as compared to the endogenous receptor.

### D. Target genomic regions

The methods and compositions described herein can be utilized to target essentially any genomic sequence of a host cell. The targeting can result in mutation or replacement of at least a portion of the target genomic sequence. Alternatively, the targeting can result in modification of the chromatin within and/or near the target genomic region, *e.g*., by recruiting a chromatin modifying effector protein. Such chromatin modification can be used to increase or decrease transcription of genes at or near the target genomic region. As yet another alternative, the targeting can repress or activate a gene at or near the target genomic region by recruiting a repressor (*e.g.,* KRAB) or activator (*e.g*., VP64) domain to the target genomic region.

Exemplary target genomic regions include regions within or near the PD-1 gene or the CTLA-4 gene. PD-1 and CTLA-4 are immune checkpoint genes and modulation or ablation of one or more of the genes can be used to control the immunogenic activity of the target cell. Exemplary target genomic regions include regions within or near genes encoding for a receptor used for viral recognition or entry. For example, the CCR5 or CXCR4 gene can be targeted to mutate or downregulate these receptors and thus confer resistance to HTV infection in the targeted cell.

Exemplary target genomic regions include regions within or near genes that encode proteins involved in cell trafficking and target homing or target recognition. Such genes include, but are not limited to, T cell and B cell receptors, T cell chemokine receptors such as CXCR4, CCR9, CCR7, pattern recognition receptors, cutaneous lymphocyte antigen, CD34, L-selectin, CD28, and GLYCAM-1.

Exemplary target genomic regions include genes containing mutations that are implicated in, associated with, or cause disease. For example, a target genomic region at or near the gene encoding FOXP3 can be targeted to increase or rescue FOXP3 function and thereby treat patients suffering from an autoimmune disease such as IPEX. As another example, a target genomic region at or near the gene encoding IL2RA can be targeted to increase or rescue IL2RA function and thereby treat patients suffering from an autoimmune disease. As yet another example, a target genomic region at or near the gene encoding IL2RG can be targeted to increase or rescue IL2RG function and thereby treat patients suffering from an immunodeficiency, such as severe combined immunodeficiency. As yet another example, a target genomic region at or near the gene encoding GATA2 can be targeted to increase or rescue GATA2 function and thereby treat patients suffering from MonoMAC.

### E. Guide RNAs

Described herein are guide RNAs and libraries of guide RNAs. The guide RNAs can contain from 5' to 3': a binding region, a 5' hairpin region, a 3' hairpin region, and a transcription termination sequence. The guide RNA can be configured to form a stable and active complex with a Cas9 protein. In some cases, the guide RNA is optimized to enhance expression of a polynucleotide encoding the guide RNA in a host cell.

The 5' hairpin region can be between about 15 and about 50 nucleotides in length (*e.g.,* about 15, 16, 17, 18, 19, 20, 21, 22, 23, 24, 25, 26, 27, 28, 29, 30, 31, 32, 33, 34, 35, 36, 37, 38, 39,40,41,42,43,44,45,46,47,48,49, or about 50 nucleotides in length). In some cases, the 5' hairpin region is between about 30-45 nucleotides in length (*e.g.,* about 31, 32, 33, 34, 35, 36, 37, 38, 39, 40, 41, 42, 43, 44, or 45 nucleotides in length). In some cases, the 5' hairpin region is, or is at least about, 31 nucleotides in length (*e.g*., is at least about 31, 32, 33, 34, 35, 36, 37, 38, 39, 40, 41, 42, 43, 44, or 45 nucleotides in length). In some cases, the 5' hairpin region contains one or more loops or bulges, each loop or bulge of about 1, 2, 3, 4, 5, 6, 7, 8, 9, or 10 nucleotides. In some cases, the 5' hairpin region contains a stem of between about 10 and 30 complementary base pairs (*e.g*., 11, 12, 13, 14, 15, 16, 17, 18, 19, 20, 21, 22, 23, 24, 25, 26, 27, 28, 29, or 30 complementary base pairs).

In some embodiments, the 5' hairpin region can contain protein-binding, or small molecule-binding structures. In some cases, the 5' hairpin function (*e.g*., interacting or assembling with a Cas9 protein) can be conditionally activated by drugs, growth factors, small molecule ligands, or a protein that binds to the protein-binding structure of the 5' stem-loop. In some embodiments, the 5' hairpin region can contain non-natural nucleotides. For example, non-natural nucleotides can be incorporated to enhance protein-RNA interaction, or to increase the thermal stability or resistance to degradation of the guide RNA.

The guide RNA can contain an intervening sequence between the 5' and 3' hairpin regions. The intervening sequence between the 5' and 3' hairpin regions can be between about 0 to about 50 nucleotides in length, preferably between about 10 and about 50 nucleotides in length (*e.g.,* at a length of, or about a length of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides). In some cases, the intervening sequence is designed to be linear, unstructured, substantially linear, or substantially unstructured. In some embodiments, the intervening sequence can contain non-natural nucleotides. For example, non-natural nucleotides can be incorporated to enhance protein-RNA interaction or to increase the activity of the guide RNA:Cas9 ribonucleoprotein complex. As another example, natural nucleotides can be incorporated to enhance the thermal stability or resistance to degradation of the guide RNA.

The 3' hairpin region can contain an about 3, 4, 5, 6, 7, or 8 nucleotide loop and an about 4, 5, 6, 7, 8, 9, 10, 11, 12, 13, 14, 15, 16, 17, 18, 19, 20,21, 22, 23, 24, or 25 nucleotide or longer stem. In some cases, the 3' hairpin region can contain a protein-binding, small molecule-binding, hormone-binding, or metabolite-binding structure that can conditionally stabilize the secondary and/or tertiary structure of the guide RNA. In some embodiments, the 3' hairpin region can contain non-natural nucleotides. For example, non-natural nucleotides can be incorporated to enhance protein-RNA interaction or to increase the activity of the guide RNA:Cas9 ribonucleoprotein complex. As another example, natural nucleotides can be incorporated to enhance the thermal stability or resistance to degradation of the guide RNA.

In some embodiments, the guide RNA includes a termination structure at its 3' end. In some cases, the guide RNA includes an additional 3' hairpin region, *e.g*., before the termination and after a first 3' hairpin region, that can interact with proteins, small-molecules, hormones, *etc.,* for stabilization or additional functionality, such as conditional stabilization or conditional regulation of guide RNA: Cas9 ribonucleoprotein assembly or activity.

Generally, the binding region is designed to complement or substantially complement, and thus bind or hybridize to, a target genomic region or a set of target genomic regions. In some cases, the binding region can incorporate wobble or degenerate bases to bind multiple target genomic regions. In some cases, the binding region can complement a sequence that is conserved amongst a set of target genomic regions to bind multiple target genomic regions. In some cases, the binding region can be altered to increase stability. For example, non-natural nucleotides, can be incorporated to increase RNA resistance to degradation. In some cases, the binding region can be altered or designed to avoid or reduce secondary structure formation in the binding region. In some cases, the binding region can be designed to optimize G-C content. In some cases, G-C content is preferably between about 40% and about 60% (*e.g.,* 40%, 45%, 50%, 55%, 60%). In some cases, the binding region, can be selected to begin with a sequence that facilitates efficient transcription of the guide RNA. For example, the binding region can begin at the 5' end with a G nucleotide. In some cases, the binding region can contain modified nucleotides such as, without limitation, methylated or phosphorylated nucleotides.

Guide RNAs can be modified by methods known in the art. In some cases, the modifications can include, but are not limited to, the addition of one or more of the following sequence elements: a 5' cap (*e.g*., a 7-methylguanylate cap); a 3' polyadenylated tail; a riboswitch sequence; a stability control sequence; a hairpin; a subcellular localization sequence; a detection sequence or label; or a binding site for one or more proteins. Modifications can also include the introduction of non-natural nucleotides including, but not limited to, one or more of the following: fluorescent nucleotides and methylated nucleotides.

In some embodiments, the guide RNAs are selected so as not to have significant off-target effects. In some cases, the similarity of a guide RNA binding region for off-target genetic element sequences can be determined. Guide RNAs directed to target genomic regions having a high similarity to one or more off-target genomic regions exceeding a predesignated threshold can be filtered out. In some cases, candidate binding regions, including the protospacer adjacent motif (PAM) sequences can be scored using a scoring metric in a manual or automated fashion. Guide RNA binding regions having an acceptable number of off-target mismatches can then be selected.

In some embodiments, the sgRNAs are targeted to specific regions at or near a gene. For example, an sgRNA can be targeted to a region at or near the 0-750 bp region 5' (upstream) of the transcription start site of a gene. In some cases, the 0-750 bp targeting of the region can provide, or provide increased, transcriptional activation by a guide RNA:Cas9 ribonucleoprotein complex. For instance, a cell can be contacted with a Cas9 domain fused to a transcriptional activator or epitope fusion domain and an guide RNA, or library of guide RNAs, targeted to the 0-750 bp region 5' of the transcription start site of one or more genes.

As another example, a guide RNA can be targeted to a region at or near the 0-1000 bp region 3' (downstream) of the transcription start site of a gene. In some cases, the 0-1000 bp targeting of the region can provide, or provide increased, transcriptional repression by an guide RNA:Cas9 ribonucleoprotein complex. For instance, a cell can be contacted with a dCas9 fused to a transcriptional repressor or epitope fusion domain and a guide RNA, or library of guide RNAs, targeted to the 0-1000 bp region 3' of the transcription start site of one or more genes.

In some embodiments, the guide RNAs are targeted to a region at or near the transcription start site (TSS) based on an automated or manually annotated database. For example, transcripts annotated by Ensembl/GENCODE or the APPRIS pipeline (Rodriguez et al., Nucleic Acids Res. 2013 Jan;41(Database issue):D110-7 can be used to identify the TSS and target genetic elements 0-750 bp upstream (*e.g*., for targeting one or more transcriptional activator domains) or 0-1000 bp downstream (*e.g*., for targeting one or more transcriptional repressor domains) of the TSS.

In some embodiments, the sgRNAs are targeted to a genomic region that is predicted to be relatively free of nucleosomes. The locations and occupancies of nucleosomes can be assayed through use of enzymatic digestion with micrococcal nuclease (MNase). MNase is an endo-exo nuclease that preferentially digests naked DNA and the DNA in linkers between nucleosomes, thus enriching for nucleosome-associated DNA. To determine nucleosome organization genome-wide, DNA remaining from MNase digestion is sequenced using high-throughput sequencing technologies (MNase-seq). Thus, regions having a high MNase-seq signal are predicted to be relatively occupied by nucleosomes and regions having a low MNase-seq signal are predicted to be relatively unoccupied by nucleosomes. Thus, in some embodiments, the sgRNAs are targeted to a genomic region that has a low MNase-Seq signal.

In some cases, the guide RNAs are targeted to a region predicted to be highly transcriptionally active. For example, the guide RNAs can be targeted to a region predicted to have a relatively high occupancy for RNA polymerase II (PolII). Such regions can be identified by PolII chromatin immunoprecipitation sequencing (ChIP-seq), which includes affinity purifying regions of DNA bound to PolII using an anti-PolII antibody and identifying the purified regions by sequencing. Therefore, regions having a high PolII Chip-seq signal are predicted to be highly transcriptionally active. Thus, in some cases, guide RNAs are targeted to regions having a high PolII ChIP-seq signal as disclosed in the ENCODE-published PolII ChIP-seq database (Landt, et al., Genome Research, 2012 Sep;22(9):1813-31).

As another example, the sgRNAs can be targeted to a region predicted to be highly transcriptionally active as identified by run-on sequencing or global run-on sequencing (GRO-seq). GRO-seq involves incubating cells or nuclei with a labeled nucleotide and an agent that inhibits binding of new RNA polymerase to transcription start sites (*e.g*., sarkosyl). Thus, only genes with an engaged RNA polymerase produce labeled transcripts. After a sufficient period of time to allow global transcription to proceed, labeled RNA is extracted and corresponding transcribed genes are identified by sequencing. Therefore, regions having a high GRO-seq signal are predicted to be highly transcriptionally active. Thus, in some cases, guide RNAs are targeted to regions having a high GRO-seq signal as disclosed in a published GRO-seq data (*e.g.,* Core et al., Science. 2008 Dec 19;322(5909): 1845-8; and Hah et al., Genome Res. 2013 Aug;23(8):1210-23).

In some embodiments, guide RNAs can be targeted to putative regulatory sequences (*e.g*., putative mammalian or human regulatory sequences), such as promoters, enhancers, insulators, silencers, splice regulators, and the like, based on DNA sequence motifs, ChIP-seq, ATAC-seq, and/or RNA-seq data.

Also described herein are expression cassettes and vectors for producing guide RNAs in a host cell. The expression cassettes can contain a promoter (*e.g.,* a heterologous promoter) operably linked to a polynucleotide encoding a guide RNA. The promoter can be inducible or constitutive. The promoter can be tissue specific. In some cases, the promoter is a U6, HI, or spleen focus-forming virus (SFFV) long terminal repeat promoter. In some cases, the promoter is a weak mammalian promoter as compared to the human elongation factor 1 promoter (EF1A). In some cases, the weak mammalian promoter is a ubiquitin C promoter or a phosphoglycerate kinase 1 promoter (PGK). In some cases, the weak mammalian promoter is a TetOn promoter in the absence of an inducer. In some cases, when a TetOn promoter is utilized, the host cell is also contacted with a tetracycline transactivator. In some embodiments, the strength of the selected guide RNA promoter is selected to express an amount of guide RNA that is proportional to (*e.g.,* within about 0.5-fold, 1-fold, 2-fold, 5-fold, 7.5-fold, or 10-fold of) the amount of Cas9 that is delivered. The expression cassette can be in a vector, such as a plasmid, a viral vector, a lentiviral vector, *etc.* In some cases, the expression cassette is in a host cell. The guide RNA expression cassette can be episomal or integrated in the host cell.

Also described herein are expression cassettes and vectors for producing guide RNAs by *in vitro* transcription.

### EXAMPLES

The following examples are offered to illustrate, but not to limit the claimed invention.

### Example 1:

T cell genome engineering holds great promise for cancer immunotherapies and cell-based therapies for HIV and autoimmune diseases, but genetic manipulation of primary human T cells has been inefficient. The present inventors have developed a way to achieve high efficiency delivery of Cas9. This high efficiency delivery of Cas9 can be used for high efficiency genome editing, gene silencing, and chromatin or chromosome modification. The delivered Cas9 can be delivered as a pre-assembled complex with guide RNAs. These active Cas9 ribonucleoproteins (RNPs) enabled the first successful Cas9-mediated homology directed repair (HDR) in primary human T cells. Thus specific nucleotide sequences in mature immune cells can be replaced with high efficiency- a longstanding goal in the field that enables diverse research and therapeutic applications. These studies establish Cas9 (*e.g*., Cas9 RNP) technology for diverse experimental and therapeutic genome engineering applications, including efficient DNA sequence replacement with HDR, in primary human T cells.

### Introduction

The CRISPR/Cas9 system has been used increasingly to edit mammalian germ-line sequence and cell-lines (1, 2). Considerable efforts are underway to employ this powerful system directly in primary human tissues, but efficiency has been limited, especially in primary hematopoietic cells, such as human CD4⁺ T cells. Plasmid delivery of *cas9* and small guide RNAs (sgRNAs) was efficient in other cell types, but only ablated 1-5% percent of target protein expression in CD4⁺ T cells (3). Improved ability to ablate key targets and correct pathogenic genome sequences in human T cells has therapeutic applications, *e.g*., allowing T cells to be edited *ex vivo* and then reintroduced into patients.

Multiple scientific and clinical trials are underway to manipulate T cell genomes with available technologies, including gene deletions with Transcription Activator-like Effector Nucleases (TALENs) and Zinc Finger Nucleases (ZFNs), and exogenous gene introduction by viral transduction (4). Genetic manipulations have been attempted to knockout HIV co-receptors CXCR4 and CCR5 in T cells to gain resistance to HIV infection (5-7). There also has been marked success in engineering T cells to recognize and kill hematological malignancies, but additional genetic modifications appear necessary for solid organ tumor immunotherapy (8-10). Further therapeutic opportunities would be possible if targeted T cell genomic loci could be corrected with specific replacement sequences, rather than deleted (11). Robust technology to promote homologous recombination in T cells can allow therapeutic correction of mutations that affect specialized T cell functions, including mutations that disrupt regulatory T cell (Treg) development and cause severe, multi-organ autoimmune disease in patients with Immunodysregulation Polyendocrinopathy Enteropathy X-linked Syndrome (IPEX) (12, 13).

Recent reports in mammalian cell lines demonstrate that Cas9 ribonucleoproteins (RNPs; recombinant Cas9 protein complexed with an *in vitro* transcribed single-guide RNA) can accomplish efficient and specific genome editing (14-16). Here the inventors show that delivery of Cas9 (*e.g*., in the form of Cas9 RNPs) to primary hematopoietic cells or primary hematopoietic stem cells can be performed with high efficiency. High efficiency delivery of Cas9 in the form of a Cas9 ribonucleoprotein complex with sgRNA leads to highly efficient genome editing of CD4⁺ T cells. Not only were the inventors able to ablate CXCR4 expression with random insertion and deletion mutations (reducing by up to 70% the number of cells with high cell surface expression of CXCR4; 18% vs. 60% in control treated cells), but the inventors were also able to introduce a precisely targeted genome sequence in primary T cells by homology-directed repair (HDR) using an exogenous single-stranded DNA template (reducing by up to 98% the number of cells with high cell surface expression; 1% vs. 60% in control treated cells). This genetic 'knock-in' technology, not previously reported with Cas9-mediated editing in primary T cells, had ∼15% efficiency and accounted for roughly half of the observed genomic edits, demonstrating that it can be useful for therapeutic replacement of disease associated mutations. Further, the inventors demonstrate the functional consequences of gene manipulation using Cas9 RNP technology to mutate *FOXP3,* which encodes the master transcription factor of Tregs. Cas9 RNPs enabled a human *in vitro* model of the multi-organ autoimmune disease IPEX, where *FOXP 3* mutations impair regulatory T cell differentiation. These studies establish Cas9 RNP technology for experimental and therapeutic editing of the genome in primary human T cells.

### Results

We aimed to overcome long-standing challenges in genetic manipulation of primary T cells, and establish a robust genome engineering toolkit. Recent reports in mammalian cell lines suggest Cas9 RNPs can accomplish efficient and specific genome editing (14-17). Given the significant challenges of efficient genome editing of T cells with DNA delivery of Cas9, we tested the efficacy of RNP delivery for targeted genome editing in primary human T cells **(****Figure 1A****).**

### Ablation of HIV co-receptor CXCR4 with Cas9 RNPs

A major goal in T cell engineering is targeted ablation of specific cell surface receptors, including co-receptors for HIV infection and co-inhibitory immune checkpoints that impair tumor immune response. Here, we programmed the Cas9 RNPs to target coding sequence of *CXCR4,* which encodes a chemokine receptor expressed on CD4⁺ T cells that serves as a co-receptor for HIV entry (18, 19). We purified recombinant *Streptococcus pyogenes* Cas9 carrying two nuclear localization signal sequences (NLS) fused at the C terminus. This Cas9 protein was incubated with *in vitro* transcribed single-guide RNA (sgRNA) designed to uniquely recognize the human *CXCR4* genomic sequence (Figure 1**B****)**. These pre-assembled RNP complexes were electroporated into human CD4⁺ T cells isolated from healthy donors **(Methods).**

Electroporation of *CXCR4* Cas9 RNPs caused efficient, site-specific editing of genomic DNA. The Cas9 RNP-induced double-stranded breaks in the *CXCR4* gene were likely repaired by non-homologous end joining (NHEJ), a predominant DNA repair pathway in cells that gives rise to variable insertions and deletions (indels) and often results in frameshift mutations (20). Flow cytometry revealed an RNP dose-dependent increase in the percentage of T cells expressing low levels of CXCR4, consistent with mutation of the *CXCR4* gene (**Figure 1C****).** The T7 endonuclease 1 (T7E1) assay is a convenient method to assess genome editing. Here, T7E1 confirmed genomic DNA editing in cells treated with *CXCR4* RNPs, but not in control cells treated with spCas9 protein not complexed with a sgRNA (CTRL) **(****Figure 1D****).** Cas9 RNP-treated cells were separated based on CXCR4 expression with fluorescence activated cell sorting (FACS) and we found an enrichment of editing in the CXCR4^{lo} cells (15-17%) compared to CXCR4^{hi} cells (4-12%). Sanger sequencing of the target *CXCR4* genomic locus, performed to directly identify editing events, suggested that the T7E1 assay underestimated editing efficiency. Sequencing of the *CXCR4* gene in CXCR4^{lo} cells showed that 8/9 clones had mutations/deletions whereas such mutations/deletions were observed in only 4/10 clones and 0/9 clones in CXCR4^{hi} and CTRL treated CXCR4^{lo} cells, respectively. None of the observed edits in the CXCR4^{hi} population terminated the coding sequence (one missense mutation and three in-frame deletions), consistent with the maintenance of protein expression. By contrast, the CXCR4^{lo} population was enriched for cells with a more extensive mutation burden in the locus **(****Figure 1E****).** These findings demonstrated successful genomic targeting with Cas9 RNPs and a functional effect on protein expression in human CD4⁺ T cells. FACS was able to purify edited cells, providing an additional useful tool for Cas9 RNP applications in primary T cells.

### Efficient genetic 'knock-in' with homology-directed repair (HDR)

Exogenous template-mediated HDR is a powerful technique for precise gene modifications that enables experimental and therapeutic editing of specific variant sequences. Given the high editing efficiency of Cas9 RNPs, we next tested whether we could achieve exogenous template-mediated HDR in primary T cells. We used a single-stranded oligonucleotide DNA template (ssODT) with 90 nucleotide (nt) homology arms to recombine with the *CXCR4* locus at the Cas9 RNP cleavage site (15). The ssODT was designed to replace 12 nt from the human reference genome and introduce a novel HindIII restriction enzyme cleavage site **(****Figure 2A****).** Cas9 RNPs were electroporated into primary CD4⁺ T cells in the presence of four different concentrations of ssODT (0, 50, 100 and 200 pmol). Cas9 RNP without ssODT again reduced the percentage of CXCR4^{Hi} cells. Notably, addition of ssODT significantly improved the efficacy of CXCR4 ablation. In the experiment shown here, we were able to achieve up to 98% reduction in the number of cells with high cell surface CXCR4 expression with 100 pmol ssODT and Cas9 RNP (1% vs. 60% in control treated cells) **(****Figure 2B** and **C****).**

Remarkably efficient HDR was observed in cells treated with Cas9 RNP and the ssODT **(****Figure 2D****).** We observed 24% total editing (defined as the sum of all NHEJ and HDR events that give rise to indels at Cas9 cleavage site) without ssODT, as measured by T7E1 assays. Up to 33% total editing was observed in the presence of 50 pmol ssODT. At this concentration, 14% HDR was observed by HindIII digest of the target locus, indicating that >40% of editing resulted from HDR (the remaining ∼60% of observed editing likely resulted from NHEJ). Although the percentage of HDR was slightly lower with 100 pmol ssODT (12%), a higher ratio of HDR to total editing was calculated (0.48 with 100 pm vs. 0.42 with 50 pmol). The near complete loss of CXCR4 staining in this condition demonstrates that the mutation introduced by HDR (84DLLFV88→ 84ESLDP88) strongly affected the cell surface expression of CXCR4 or its recognition by the antibody **(****Figure 2B** and **C****).** In this experiment, the editing efficiency was reduced with 200 pmol ssODT.

Both total editing and HDR could be enriched by sorting the CXCR4^{lo} population, although the effect was less pronounced than in **Figure 1****,** consistent with the larger fraction of CXCR4^{lo} cells in the unsorted population. Note that in these experiments a more stringent gate was applied to separate the cells with the highest expression of CXCR4, and in this CXCR4^{hi} population no editing was observed. These studies collectively demonstrated the power of Cas9 RNPs coupled with ssODT to precisely replace targeted DNA sequences in primary human T cells.

### Functional effects of FOXP3 mutation during Treg differentiation

We next tested whether Cas9 RNP-mediated genome editing could alter the balance between pro-inflammatory effector T cell subsets, which are associated with protection against pathogens and malignancy, and suppressive FOXP3⁺ Tregs, which are essential to prevent the development of autoimmunity. FOXP3 is essential for functional Tregs in mice (21-24). Mutations in the *FOXP 3* gene in humans lead to impaired Treg development and function causing IPEX, a multi-organ autoimmunity syndrome (12, 13). Cas9 RNP-mediated genome editing provides a unique opportunity to experimentally introduce mutations into the human *FOXP 3* gene and test their effects on the development of Tregs.

To test the functional consequences of *FOXP 3* mutations, we targeted two exonic sites with Cas9 RNPs **(****Figure 3A****).** To aid in the interpretation of editing in the *FOXP3* locus on the X chromosome, these experiments were conducted with cells from male donors. We tested the efficacy of Cas9 RNPs in primary CD4⁺CD25⁺CD127^{lo} Tregs that were isolated from human male donors as previously described (25). Successful genome editing was detected by the T7E1 assay in Tregs treated with FOXP3 Cas9 RNPs, but not in the control cells transfected with Cas9 protein only **(****Figure 3B****).** *FOXP 3* Cas9 RNPs caused an increased percentage of FOXP3 negative cells assessed by intracellular staining **(****Figure 3C****).** Flow cytometry results showed that up to 40% of cells lost FOXP3 expression as a result of Cas9 RNP treatment (85% FOXP3⁺ in control treated cells vs. 63% with FOXP3 Cas9 RNP1, 46% with FOXP3 Cas9 RNP2 and 54% with FOXP3 Cas9 RNP 1 and 2 combined). The fraction of FOXP3 ablated cells is likely higher initially as FOXP3 Cas9 RNP treatment appeared to cause a proliferation defect in Tregs (data not shown).

Cas9 RNP editing revealed the phenotypic consequences of *FOXP 3* ablation in primary human Tregs. Flow cytometry confirmed altered cytokine receptor expression in the FOXP3 Cas9 RNP treated cells with increased levels of CD127 (IL7Rα) **(****Figure 3D****).** *CD127* is transcriptionally repressed directly by FOXP3 (25), suggesting that Cas9 RNP treatment causes expected dysregulation from loss of the master regulator of Tregs. The findings were consistent with de-stabilization of the gene expression program required for Treg function as a result of Cas9 RNP-mediated *FOXP3* ablation.

We next attempted to recapitulated *in vitro* the defective Treg differentiation associated with *FOXP 3* mutations in IPEX patients. Cas9 RNPs were delivered to *ex vivo* stimulated naive T cells, which were subsequently cultured in IL-2 and TGF-β to promote the generation of iTregs (26-28). In control cells treated with Cas9 protein alone, 30% FOXP3⁺ iTregs developed. FOXP3 Cas9 RNP 1, FOXP3 Cas9 RNP 2, and treatment with both FOXP3 Cas9 RNP 1 and 2 all resulted in reduced percentages of FOXP3⁺ iTregs (8%, 9% and 11% respectively) **(****Figure 4A****).** Reduced percentages of FOXP3⁺ iTregs, and small but reproducible increases in the fraction of cells producing the pro-inflammatory cytokine interferon-γ (IFNγ) were observed across three independent experiments **(****Figure 4B****).**

To further examine the functional effects of *FOXP 3* mutations during iTreg differentiation, we subjected the Cas9 RNP-treated cells to FACS analysis of CTLA-4, a key cell surface receptor involved in Treg suppression (29). Treatment with FOXP3 Cas9 RNPs reduced the percentage of cells that express CTLA-4 **(****Figure 4C****).** In control cells, CTLA-4 was induced in iTregs as well as in stimulated FOXP3⁻ effector T cells. We found that *FOXP3* targeting strongly diminished the percentage of CTLA-4⁺FOXP3⁺ iTregs, but had modest effects on CTLA-4 expression in FOXP3⁻ cells, consistent with FOXP3-dependent and FOXP3-independent mechanisms both contributing to CTLA-4 expression (23, 30). Electroporation with the short-lived Cas9 RNPs altered the developmental potential of the FOXP3 ablated T cells. This technology can be used to screen for additional genes or regulatory elements required for human Treg differentiation. Importantly, the highly efficient genome editing in T cells by the Cas9 RNP approach enabled a human *in vitro* disease model of IPEX confirming that *FOXP 3* mutations impair iTreg differentiation.

### Discussion

Efficient delivery of Cas9 to primary hematopoietic cells and/or primary hematopoietic stem cells provides a powerful platform for basic research of cell, tissue, and system function, as well as development and use of cell-based therapeutics. For example, Cas9-mediated genome engineering can be used to experimentally and therapeutically target DNA elements crucial to inflammatory and suppressive human T cell subsets. We report here successful genome engineering in human conventional and regulatory CD4⁺ T cells by delivery of *in vitro* assembled and functional Cas9 RNPs. Electroporation of Cas9 RNPs allowed targeted 'knock-out' of the CXCR4 cell surface receptor. RNPs also promoted the first successful Cas9-mediated genetic 'knock-in' primary human T cells. The highly efficient targeted DNA replacement in mature immune cells achieves a longstanding goal in the field that enables diverse research and therapeutic applications. Finally, we also employed Cas9 RNPs to target *FOXP 3,* a master transcriptional regulator, in stimulated human naive T cells and Tregs to model the functional impairment of Treg differentiation in patients with IPEX. The studies collectively establish a broadly applicable toolkit for genetic manipulation of human primary T cells.

There are notable advantages to genome engineering with transient RNP delivery compared to other CRISPR/Cas9 delivery methods. Recent work reported ablation of cell surface markers in bulk human CD4⁺ T cells by transfection of plasmid carrying the *cas9* gene and guide RNA coding sequence (3). Although successful, efficiency was notably low in CD4⁺ T cells compared to other cell types, possibly due to suboptimal levels of Cas9 or sgRNA, suboptimal nuclear translocation or suboptimal intracellular RNP complex formation (or some combination of these factors). RNP-based delivery circumvents these challenges. Delivery of Cas9 RNPs offers fast editing action and rapid protein turnover in the cells as they are reportedly degraded within 24 hours of delivery (14). This limited temporal window of Cas9 editing can make Cas9 RNPs safer for therapeutic applications than other delivery modes where cells are exposed to Cas9 for a longer time frame. Our findings now show that Cas9 RNPs are able to rapidly and efficiently edit human T cells.

We were able to achieve remarkably efficient HDR here, with 98% reduction in CXCR4^{hi} cells with Cas9 RNPs and an HDR template targeting CXCR4 in one experiment. Remaining variables that affect editing and HDR efficiency in primary T cells can be optimized to achieve even higher genome editing efficiency. For example, variation in cell type and cell cycle dynamics can significantly alter Cas9 RNP efficiency (15). In primary human T cells, editing efficiency can also be affected by T cell donor specific factors (e.g. genetics, recent infection), *in vitro* T cell activation status, and characteristics of the targeted genomic locus (e.g. DNA sequence, chromatin state).

The ability to edit specific DNA sequences in human T cell subsets enables experimental investigation of transcription factors, *cis*-regulatory elements, and target genes implicated in T cell inflammatory and suppressive functions. Here we demonstrate, as a proof-of-principle, the ability to knock-out *FOXP 3,* a key transcriptional regulator, to assess the functional effects on down-stream expression programs and cellular differentiation. These experiments model *in vitro* the Treg differentiation associated with the mendelian multi-organ autoimmune syndrome, IPEX. Extensive efforts have mapped key gene regulatory circuitry controlling the development and function of diverse and specialized T cell subsets (31). We recently reported that most causal genetic variants contributing to risk of human autoimmune diseases map to key regulatory elements in T cells (32). Genome editing of primary T cells provides a powerful perturbation test to assess the function of regulatory elements and characterize the effects of disease-associated coding and non-coding variation.

Therapeutic editing requires improved techniques to identify successfully edited cells in a population. Selection of edited cells is notably challenging in primary cells that cannot be maintained indefinitely in culture, unlike transformed cell lines. Here we demonstrate FACS enrichment of edited cells, based on expected phenotypic changes in cell surface receptor expression. The success of Cas9 RNP-mediated HDR also allows introduction of genetic markers to purify homogenously edited cells for certain applications.

Therapeutic T cell engineering requires highly efficient and precisely targeted genome editing in primary cells. The highly efficient Cas9 delivery technology reported here can provide, e.g., highly efficient and precisely targeted genome editing in primary cells. Such highly efficient delivery can be used to correct genetic variants and engineer human T cell function for the treatment of infection, autoimmunity and cancer.

### Materials and Methods

### Human T cell isolation and culture

In accordance with protocols approved by the UCSF Committee on Human Research (CHR), whole blood was collected from human donors into sodium heparinized vacutainer tubes (Becton Dickinson) and processed within 12 hrs. Peripheral blood mononuclear cells (PBMCs) were isolated by Ficoll gradient centrifugation. The blood was mixed in a 1:1 ratio with Ca²⁺ and Mg²⁺ free Hank's balanced salt solution (HBSS), transferred to 50 ml Falcon tubes (30 ml of blood HBSS mixture/tube) and underlayed with 12 ml Ficoll-Paque PLUS (Amersham/GE healthcare). After density gradient centrifugation (1000g, 20 min, no brakes) the PBMC layer was carefully removed and the cells washed twice with Ca²⁺ and Mg²⁺ free HBSS. CD4⁺ T cells were pre-enriched with Easysep Human CD4⁺ T cell enrichment kit (Stemcell technologies) according to the manufacturer's protocol. Pre-enriched CD4⁺ T cells were stained with following antibodies: αCD4-PerCp (SK3; Becton Dickinson), aCD25-APC (BC96; TONBO Biosciences), aCD127-PE (R34-34; TONBO Biosciences), αCD45RA-violetFluor450 (HI100; TONBO Biosciences) and αCD45RO-FITC (UCHL1; TONBO Biosciences). CD4⁺CD25^{hi}CD127^{lo} Tregs, CD4⁺CD25^{lo}CD127^{hi} T effectors (Teffs), and CD4⁺CD25^{lo}CD127^{hi}CD45RA^{hi}CD45RO⁻naïve T cells (Tnaives) were isolated using a FACS Aria Illu (Becton Dickinson). Treg, Teff and Tnaive purity was > 97%.

For Cas9 RNP transfection, Tregs, Teffs, or Tnaives were pre-activated on αCD3 (UCHT1; BD Pharmingen) and aCD28 (CD28.2; BD Pharmingen) coated plates for 48 hrs. Plates were coated with 10 µg/ml αCD3 and αCD28 in PBS for at least 2 hrs at 37°C. For iTreg differentiation, FACS-sorted Tnaives were activated with plate-coated αCD3 and aCD28 in the presence of 100 IU/ml IL-2 (Aldesleukin, UCSF Pharmacy) and 10 ng/ml TGF-β1 (Tonbo Biosciences). One iTreg differentiation experiment conducted in the presence of anti-IFNy and anti-IL-4 blocking antibodies was excluded from the analysis in Figure 4.

Teffs were activated in RPMI complete (RPMI-1640 (UCSF CCF) supplemented with 5 mmol/l 4-(2-hydroxyethyl)-1-piperazineethanesulfonic acid (HEPES) (UCSF CCF), 2 mmol/l Glutamax (Gibco), 50 µg/ml penicillin/streptomycin (Coming), 50 µmol/l 2-mercaptoethanol (Sigma-Aldrich), 5 mmol/l nonessential amino acids (Corning), 5 mmol/l sodium pyruvate (UCSF CCF), and 10% fetal bovine serum (Atlanta Biologicals) in a cell density of 5x10⁵ cells/ml). After electroporation the medium was supplemented with 40 IU/ml IL-2.

Tregs were activated in RPMI complete. Post-electroporation, 300 IU/ml IL-2 was added to the medium to further expand the cells. Tregs, Teffs or Tnaives were further supplemented with their respective medium at day 1, day 3 and day 5 after electroporation. Teffs and Teffs were kept at a cell density of 5x10⁵/ml. Tregs were cultured at a cell density of 2.5x10⁵ cells/ml.

### Expression and Purification of Cas9

The recombinant *S. pyogenes* Cas9 used in this study carries at the C terminus an HA tag and two nuclear localization signal peptides which facilitates transport across nuclear membrane. The protein was expressed with a N-terminal hexahistidine tag and maltose binding protein in *E. coli* Rosetta 2 cells (EMD Millipore) from plasmid pMJ915. The His tag and maltose binding protein were cleaved by TEV protease, and Cas9 was purified by the protocols described in Jinek et al., 2012. Cas9 was stored in 20 mM HEPES at pH 7.5, 150 mM KCl, 10% glycerol, 1 mM tris(2-chloroethyl) phosphate (TCEP) at -80°C.

### In vitro T7 transcription of sgRNA

The DNA template encoding for a T7 promoter, a 20 nt target sequence and the chimeric sgRNA scaffold was assembled from synthetic oligonucleotides by overlapping PCR. Briefly, for the CXCR4 sgRNA template, the PCR reaction contains 20 nM premix of SLKS3 (5'- TAA TAC GAC TCA CTA TAG GAA GCG TGA TGA CAA AGA GGG TIT TAG AGC TAT GCT GGA AAC AGC ATA GCA AGT TAA AAT AAG G -3') and SLKS1 (5'- GCA CCG ACT CGG TGC CAC TTT TTC AAG TTG ATA ACG GAC TAG CCT TAT TTT AAC TTG CTA TGC TGT TTC CAG C -3'), 1 µM premix of T25 (5'-TAA TAC GAC TCA CTA TAG-3') and SLKS1 (5'- GCA CCG ACT CGG TGC CAC TTT TTC AAG -3'), 200 µM dNTP and Phusion Polymerase (NEB) according to manufacturer's protocol. The thermocycler setting consisted of 30 cycles of 95°C for 10 sec, 57°C for 10 sec and 72°C for 10 sec. The PCR product was extracted once with phenol:chloroform:isoamylalcohol and then once with chloroform, before isopropanol precipitation overnight at -20°C. The DNA pellet was washed three times with 70% ethanol, dried by vacuum and dissolved in DEPC-treated water. The FOXP3 sgRNA template was assembled from T25, SLKS1, SLKS2 and SLKS4 (5'- TAA TAC GAC TCA CTA TAG AGG AGC CTC GCC CAG CTG GAG TIT TAG AGC TAT GCT GGA AAC AGC ATA GCA AGT TAA AAT AAG G -3') by the same procedure.

A 100 µl T7 *in vitro* transcription reaction consisted of 30 mM Tris-HCl (pH 8), 20 mM MgCl₂, 0.01% Triton X-100, 2 mM spermidine, 10 mM fresh dithiothreitol, 5 mM of each ribonucleotide triphosphate, 100 µg/ml T7 Pol and 0.1 µM DNA template. The reaction was incubated at 37°C for 4 h, and 5 units of RNase-free DNaseI (Promega) was added to digest the DNA template 37°C for 1 h. The reaction was quenched with 2xSTOP solution (95% deionized formamide, 0.05% bromophenol blue and 20 mM EDTA) at 60°C for 5 min. The RNA was purified by electrophoresis in 10% polyacrylamide gel containing 6 M urea. The RNA band was excised from the gel, grinded up in a 15 ml tube, and eluted with 5 volumes of 300 mM sodium acetate (pH 5) overnight at 4°C. One equivalent of isopropanol was added to precipitate the RNA at -20°C. The RNA pellet was collected by centrifugation, washed three times with 70% ethanol, and dried by vacuum. To refold the sgRNA, the RNA pellet was first dissolved in 20 mM HEPES (pH 7.5), 150 mM KCl, 10% glycerol and 1 mM TCEP. The sgRNA was heated to 70°C for 5 min and cooled to room temperature. MgCl₂ was added to a final concentration of 1 mM. The sgRNA was again heated to 50°C for 5 min, cooled to room temperature and kept on ice. The sgRNA concentration was determined by OD₂₆₀ₙₘ using Nanodrop and adjusted to 100 µM using 20 mM HEPES (pH 7.5), 150 mM KCl, 10% glycerol, 1 mM TCEP and 1 mM MgCl₂. The sgRNA was store at -80°C.

### Cas9 RNP assembly and electroporation

Cas9 RNP was prepared immediately before experiments by incubating 20 µM Cas9 with 20 µM sgRNA at 1:1 ratio in 20 µM HEPES (pH 7.5), 150 mM KCl, 1 mM MgCl₂, 10% glycerol and 1 mM TCEP at 37°C for 10 min to a final concentration of 10 µM.

T cells were electroporated with a Neon transfection kit and device (Invitrogen). 2 - 2.5 x10⁵ T cells were washed three times with PBS before resuspension in 9 µl of buffer T (Neon kit, Invitrogen). Cas9 RNP (1 - 2 µl of 10 µM Cas9 only (CTRL) or Cas9:sgRNA RNP; final concentration 0.9 - 1.8 µM) as well as HDR template (0 - 200 pmol) were added to the cell suspension, mixed and transfected into the cells with a Neon electroporation device (Invitrogen; 1600V, 10 msec, 3 pulses). The HDR template is a single-stranded oligonucleotide complementary (- strand) to the target sequence, and contains a HindIII restriction sequence flanked by 90-nt homology arms (sequence: 5'-GGG CAA TGG ATT GGT CAT CCT GGT CAT GGG TTA CCA GAA GAA ACT GAG AAG CAT GAC GGA CAA GTA CAG GCT GCA CCT GTC AGT GGC CGA AAG CTT GGA TCC CAT CAC GCT TCC CTT CTG GGC AGT TGA TGC CGT GGC AAA CTG GTA CTT TGG GAA CTT CCT ATG CAA GGC AGT CCA TGT CAT CTA CAC AGT-3').

Electroporated Tregs, Teffs or Tnaives were transferred to 500 µl of their respective culture medium in a αCD3/CD28 coated 48-well plate. 24 hrs after electroporation cells were resuspended and transferred to a non-coated well plate. 4 - 6 days after electroporation, T cells were analyzed by FACS and T7 endonuclease I assay.

### PCR amplification of target region

5x10⁴ - 2x10⁵ cells were resuspended in 100 µl of Quick Extraction solution (Epicenter) were added to lyse the cells and extract the genomic DNA. The cell lysate was incubated at 65°C for 20 min and then 95°C for 20 min, and stored at -20°C. The concentration of genomic DNA was determined by NanoDrop (Thermo Scientific).

Genomic regions, containing the CXCR4 Target, FOXP3 Target 1 or FOXP3 Target 2 target sites, were PCR amplified using the following primer sets. For CXCR4: forward 5'-AGA GGA GTT AGC CAA GAT GTG ACT TTG AAA CC -3' and reverse 5'- GGA CAG GAT GAC AAT ACC AGG CAG GAT AAG GCC -3' (938 bp). For FOXP3 Target 1: forward 5'-TTC AAA TAC TCT GCA CTG CAA GCC C-3' and reverse 5'- CAT GTA CCT GTG TTC TTG GTG TGT GT-3' (900 bp) For FOXP3 Target 2: forward 5'- GCT GAC ATT TTG ACT AGC TTT GTA AAG CTC TGT GG-3' and reverse 5'- TCT CCC CGA CCT CCC AAT CCC-3' (900 bp). The CXCR4 primers were designed to avoid amplifying the HDR templates by annealing outside of the homology arms. The PCR reaction contained 200 ng of genomic DNA and Kapa Hot start high-fidelity polymerase (Kapa Biosystems) in high GC buffer according to the manufacturer's protocol. The thermocycler setting consisted of one cycle of 95°C for 5 min, 35 cycles of 98°C for 20 sec, 62°C (CXCR4 and FOXP3 Target 2) or 60°C (FOXP3 Target 1) for 15 sec and 72°C for 1 min, and one cycle of 72°C for 1 min. The PCR products were purified on 2% agarose gel containing SYBR Safe (Life Technologies). The PCR products were eluted from the agarose gel using QIAquick gel extraction kit (Qiagen). The concentration of PCR DNA was quantitated with a NanoDrop device (Thermo scientific). 200 ng of PCR DNA was used for T7 endonuclease I and HindIII analyses.

### Analysis of Editing Efficiency by T7 endonuclease I assay

Editing efficiency was determined by T7 endonuclease I assay. T7 endonuclease I recognizes and cleaves mismatched heteroduplex DNA that arises from hybridization of wild-type and mutant DNA strands. The hybridization reaction contained 200 ng of PCR DNA in KAPA high GC buffer and 50 mM KCl, and was performed on a thermocycler with the following setting: 95°C, 10 min, 95-85°C at -2°C/sec, 85°C for 1 min, 85-75°C at - 2°C/sec, 75°C for 1 min, 75-65°C at -2°C/sec, 65°C for 1 min, 65-55°C at -2°C/sec, 55°C for 1 min, 55-45°C at -2°C/sec, 45°C for 1 min, 45-35°C at -2°C/sec, 35°C for 1 min, 35-25°C at -2°C/sec, 25°C for 1 min, and hold at 4°C. Buffer 2 and 5 units of T7 endonuclease I (NEB) were added to digest the re-annealed DNA. After 1 hr of incubation at 37°C, the reaction was quenched with 6x blue gel loading dye (Thermo Scientific) at 70°C for 10 min. The product was resolved on 2% agarose gel containing SYBR gold (Life technologies). The DNA band intensity was quantitated using Image Lab. The percentage of editing was calculated using the following equation (1 - (1 - (b + c / a + b +c))^{1/2}) x 100, where "a" is the band intensity of DNA substrate and "b" and "c" are the cleavage products.

### Analysis of HDR by HindID restriction digestion

The CXCR4 HDR template introduces a HindIII restriction site into the gene locus. A 938 bp region as PCR amplified using the primers 5'- AGA GGA GTT AGC CAA GAT GTG ACT TTG AAA CC -3' and 5'- GGA CAG GAT GAC AAT ACC AGG CAG GAT AAG GCC -3'. The reaction consisted of 200 ng of PCR DNA and 10 units of HindIII High Fidelity in CutSmart Buffer (NEB). After 2 hr of incubation at 37°C, the reaction was quenched with one volume of gel loading dye at 70°C for 10 min. The product was resolved on 2% agarose gel containing SYBR gold (Life technologies). The band intensity was quantitated using Image Lab. The percentage of HDR was calculated using the following equation (b + c / a + b +c) x 100, where "a" is the band intensity of DNA substrate and "b" and "c" are the cleavage products.

### FACS analysis of edited T cells

CXCR4 cell surface staining was performed with aCXCR4-APC (12G5; BD Pharmingen) for 15 min on ice. Cells were kept at 4°C throughout the staining procedure until cell sorting to avoid antibody-mediated internalization and degradation of the antibody. Cells were sorted using a FACS Aria Illu (Becton Dickinson).

For analysis of Cas9 RNP-edited Tregs and iTregs following antibodies were used: aCD-PacificBlue (RPA-T4; BD Pharmingen), αFOXP3-AlexaFluor488 (206D; Biolegend), aCD25-APC (BC96; TONBO Biosciences), aCD127-PECy7 (HIL-7R-M21; BD Pharmingen), αIL-17a-PerCp-Cy5.5 (N49-653; BD Pharmingen), αIL-10-PE (JES3-9D7; BD Pharmingen), αIFNγ-AlexaFluor700 (B27; Biolegend), aCTLA-4-PE (L3D10; Biolegend).

Cells were stimulated for 2 hrs with 100 ng/ml PMA (Sigma-Aldrich) and 1 µg/ml Iononmycin (Sigma-Aldrich). 1 µM Monensin (Biolegend) was added for 3 hrs of additional cell stimulation. Cells were stained for surface markers for 20 min at RT followed by 30 min incubation with FOXP3/Transcription Factor Fix/Perm (TONBO Biosciences). To increase FOXP3 signal, Tregs were incubated with 100 U/ml DNAseI (Sigma-Aldrich) in Flow Cytometry Perm buffer (TONBO Biosciences). iTregs were not treated with DNaseI because of subsequent cell sorting and T7EI analysis. Intracellular cytokine and transcription factor staining was carried out for 30 min at RT. Tregs were acquired with an LSRFortessaDual (Becton Dickinson), iTregs were acquired and sorted using a FACS Aria Illu (Becton Dickinson).

### Statistics

The quantities of FOXP3⁺ and IFNγ secreting cells following FOXP3 Cas9 RNP treatment in three iTreg differentiation experiments were compared to the quantities following control treatment using a t-test. Standard deviations were calculated and shown as error bars. Results of analysis are shown in **Figure 4B****.**

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

### References

1. Doudna JA & Charpentier E (2014) Genome editing. The new frontier of genome engineering with CRISPR-Cas9. Science 346(6213): 1258096.
2. Hsu PD, Lander ES, & Zhang F (2014) Development and applications of CRISPR-Cas9 for genome engineering. Cell 157(6):1262-1278.
3. Mandal PK, et al. (2014) Efficient Ablation of Genes in Human Hematopoietic Stem and Effector Cells using CRISPR/Cas9. Cell Stem Cell 15(5):643-652.
4. Maus MV, et al. (2014) Adoptive immunotherapy for cancer or viruses. Annual Review of Immunology 32:189-225.
5. Tebas P, et al. (2014) Gene editing of CCR5 in autologous CD4 T cells of persons infected with HIV. The New England Journal of Medicine 370(10):901-910.
6. Didigu CA, et al. (2014) Simultaneous zinc-finger nuclease editing of the HIV coreceptors ccr5 and cxcr4 protects CD4+ T cells from HIV-1 infection. Blood 123(1):61-69.
7. Hutter G, et al. (2009) Long-term control of HIV by CCR5 Delta32/Delta32 stemcell transplantation. The New England Journal of Medicine 360(7):692-698.
8. Restifo NP, Dudley ME, & Rosenberg SA (2012) Adoptive immunotherapy for cancer: harnessing the T cell response. Nature Reviews. Immunology 1.2(4):269-281.
9. Porter DL, Levine BL, Kalos M, Bagg A, & June CH (2011) Chimeric antigen receptor-modified T cells in chronic lymphoid leukemia. The New England Journal of Medicine 365(8):725-733.
10. Moon EK, et al. (2014) Multifactorial T-cell hypofunction that is reversible can limit the efficacy of chimeric antigen receptor-transduced human T cells in solid tumors. Clinical Cancer Research : An Official Journal of the American Association for Cancer Research 20(16):4262-4273.
11. Genovese P, et al. (2014) Targeted genome editing in human repopulating haematopoietic stem cells. Nature 510(7504):235-240.
12. Bennett CL, et al. (2001) The immune dysregulation, polyendocrinopathy, enteropathy, X-linked syndrome (IPEX) is caused by mutations of FOXP3. Nature Genetics 27(1):20-21.
13. Wildin RS, et al. (2001) X-linked neonatal diabetes mellitus, enteropathy and endocrinopathy syndrome is the human equivalent of mouse scurfy. Nature Genetics 27(1):18-20.
14. Kim S, Kim D, Cho SW, Kim J, & Kim JS (2014) Highly efficient RNA-guided genome editing in human cells via delivery of purified Cas9 ribonucleoproteins. Genome Research 24(6): 1012-1019.
15. Lin S, Staahl B, Alla RK, & Doudna JA (2014) Enhanced homology-directed human genome engineering by controlled timing of CRISPR/Cas9 delivery. eLife 3.
16. Zuris JA, etal. (2014) Cationic lipid-mediated delivery of proteins enables efficient protein-based genome editing in vitro and in vivo. Nature Biotechnology*.*
17. Sung YH, et al. (2014) Highly efficient gene knockout in mice and zebrafish with RNA-guided endonucleases. Genome Research 24(1):125-131.
18. Berson JF, et al. (1996) A seven-transmembrane domain receptor involved in fusion and entry of T-cell-tropic human immunodeficiency virus type 1 strains. Journal of Virology 70(9):6288-6295.
19. Feng Y, Broder CC, Kennedy PE, & Berger EA (1996) HIV-1 entry cofactor: functional cDNA cloning of a seven-transmembrane, G protein-coupled receptor. Science 272(5263):872-877.
20. Symington LS & Gautier J (2011) Double-strand break end resection and repair pathway choice. Annual Review of Genetics 45:247-271.
21. Khattri R, Cox T, Yasayko SA, & Ramsdell F (2003) An essential role for Scurfin in CD4+CD25+ T regulatory cells. Nature Immunology 4(4):337-342.
22. Fontenot JD, Gavin MA, & Rudensky AY (2003) Foxp3 programs the development and function of CD4+CD25+ regulatory T cells. Nature Immunology 4(4):330-336.
23. Hori S, Nomura T, & Sakaguchi S (2003) Control of regulatory T cell development by the transcription factor Foxp3. Science 299(5609):1057-1061.
24. Brunkow ME, et al. (2001) Disruption of a new forkhead/winged-helix protein, scurfin, results in the fatal lymphoproliferative disorder of the scurfy mouse. Nature Genetics 27(1):68-73.
25. Liu W, et al (2006) CD127 expression inversely correlates with FoxP3 and suppressive function of human CD4+ T reg cells. The Journal of Experimental Medicine 203(7):1701-1711.
26. Chen W, et al. (2003) Conversion of peripheral CD4+CD25- naive T cells to CD4+CD25+ regulatory T cells by TGF-beta induction of transcription factor Foxp3. The Journal of Experimental Medicine 198(12):1875-1886.
27. Fantini MC, et al. (2004) Cutting edge: TGF-beta induces a regulatory phenotype in CD4+CD25- T cells through Foxp3 induction and down-regulation of Smad7. Journal of Immunology 172(9):5149-5153.
28. Zheng SG, Gray JD, Ohtsuka K, Yamagiwa S, & Horwitz DA (2002) Generation ex vivo of TGF-beta-producing regulatory T cells from CD4+CD25- precursors. Journal of Immunology 169(8):4183-4189.
29. Wing K, et al. (2008) CTLA-4 control over Foxp3+ regulatory T cell function. Science 322(5899):271-275.
30. Wu Y, et al. (2006) FOXP3 controls regulatory T cell function through cooperation with NFAT. Cell 126(2):375-387.
31. Vahedi G, et al. (2013) Helper T-cell identity and evolution of differential transcriptomes and epigenomes. Immunological Reviews 252(1):24-40.
32. Farh KK. et al. (2014) Genetic and epigenetic fine mapping of causal autoimmune disease variants. Nature*.*

### Example 2:

sgRNAs directed to PD-1 exon 1 (PD-1 target 1, targeted by sgRNA1 and PD-1 target 2, targeted by sgRNA2) and exon 2 (PD-1 target 3, targeted by sgRNA3 and PD-1 target 3, targeted by sgRNA4) were designed **(****Figure 5A****).** HDR oligonucleotides to provide template directed repair of double strand breaks induced at the sgRNA target sites were also generated **(****Figure 5A****).** Cas9 RNPs containing sgRNA1-4 were generated and delivered to primary human effector T cells (CD4+CD25^{lo}CD127^{hi}), and the cells were recovered. Analysis of the cells after recovery by FACS reveals high efficiency ablation of PD-1 using multiple Cas9 RNPs and combinations thereof. The functional effects of various combinations of Cas9 RNPs targeting PD-1 coding sequence with two different HDR templates were assessed by FACS analysis of PD-1 cell surface expression. Ablation was observed with multiple combinations of Cas9 RNPs with each of the two HDR templates (designed to delete a portion of the coding sequence and introduce premature stop codons and a new HindIII restriction enzyme digestion site).

We also edited chimeric antigen receptor expressing (CAR) CD4+ and CD8+ T cells. T cells were edited with PD-1 Cas9 RNPs (PD-1 sgRNA 2) as described before. Nucleofection with PD-1 Cas9 RNPs was followed by transduction with CAR-GFP lentivirus. CAR-GFP expression and PD-1 surface expression levels were assessed by FACS. We were able to generate PD-1-/low CAR+ T cells.

### Example 3:

A reaction mixture containing Cas9 RNP, FITC labeled dextran, Pacific Blue (PB) labeled dextran, and unstimulated CD4⁺ T cells was provided and squeezed through an SQZ cell squeezing device (SQZ Biotech). Cells were sorted by FACS into a population of double-labeled (FITC and PB) and unlabeled cells. The two populations of cells were assayed for Cas9-mediated genome editing using a T7 endonuclease 1 (T7E1) assay. Cells were sorted based on uptake of a Pacific Blue (PB)-labeled Dextran (3000 MW) FTTC-labeled Dextran (500,000 MW) and T7 endonuclease 1 assay confirmed enrichment of editing in cells that had taken up both Dextrans. **(****Figure 6****).**

### Example 4:

### Introduction

This example provides additional details of the experiments performed in Example 1 as well as additional related experimental methods and results. This Examiner demonstrates the ability of the methods and compositions described herein to ablate a target gene with the random insertion and deletion mutations that likely result from non-homologous end joining (NHEJ) repair of a Cas9-induced double-stranded DNA break (DSB). Cells with genomic edits in CXCR4 could be enriched by sorting based on low CXCR4 expression. This Example further demonstrates the ability to use the methods and compositions described herein to introduce precisely targeted nucleotide replacements in primary T cells at CXCR4 and PD-1 by homology-directed repair (HDR) using Cas9 RNPs and exogenous single-stranded DNA templates. This technology enabled Cas9-mediated generation of 'knock-in' primary human T cells. Deep sequencing of a target site confirmed that Cas9 RNPs promoted 'knock-in' genome modifications with up to ∼20% efficiency (∼22% was achieved with 50 pmol and ∼18% with 100 pmol of HDR template), which accounted for up to approximately one third of the total editing events. These findings show that Cas9 RNP-mediated nucleotide replacement can prove useful for therapeutic correction of disease-associated mutations. This establishes the utility of Cas9 RNP technology for experimental and therapeutic knock-out and knock-in editing of the genome in primary human T cells.

### Results

The methods and compositions described herein overcome long-standing challenges in genetic manipulation of primary T cells, and establish an efficient genome engineering toolkit. Recent reports in mammalian cell lines suggest Cas9 RNPs can accomplish efficient and specific genome editing (15-18). The experiments described herein demonstrate the efficacy of Cas9 RNP delivery for targeted genome editing in primary human T cells (Figure 7A).

**Ablation of HIV co-receptor CXCR4 with Cas9 RNPs.** A major goal in T cell engineering is targeted ablation of specific cell surface receptors, including co-receptors for HIV infection and co-inhibitory immune checkpoints that impair tumor immune response. This Example demonstrates the use of programmed the Cas9 RNPs to target the exonic sequence of *CXCR4,* which encodes a chemokine receptor with multiple roles in hematopoiesis and cell homing that is expressed on CD4⁺ T cells and serves as a co-receptor for HIV entry (19-21). Purified recombinant *Streptococcus pyogenes* Cas9 carrying two nuclear localization signal sequences (NLS) fused at the C terminus was utilized. This Cas9 protein was incubated with *in vitro* transcribed single-guide RNA (sgRNA) designed to uniquely recognize the human *CXCR4* genomic sequence (Figure 7B). These pre-assembled Cas9 RNP complexes were electroporated into human CD4⁺ T cells isolated from healthy donors.

Electroporation of CXCR4 Cas9 RNPs caused efficient, site-specific editing of genomic DNA. The Cas9 RNP-induced DSBs in the *CXCR4* gene were likely repaired by NHEJ, a predominant DNA repair pathway in cells that gives rise to variable insertions and deletions (indels) and often results in frameshift mutations and loss of gene function (22). Flow cytometry revealed a Cas9 RNP dose-dependent increase in the percentage of T cells expressing low levels of CXCR4, consistent with mutation of the *CXCR4* gene (Figure 7C). The T7 endonuclease I (T7E1) assay is a convenient method to assess editing at specific sites in the genome. Here, T7E1 assay confirmed genomic DNA editing at the *CXCR4* locus in cells treated with CXCR4 Cas9 RNPs, but not in control cells treated with Cas9 protein alone (no sgRNA; CTRL). Cas9 RNP-treated cells were separated based on CXCR4 expression with fluorescence activated cell sorting (FACS). Using the T7E1 assay, an enrichment of editing in the CXCR4^{lo} cells (15-17%) compared to CXCR4^{hi} cells was found (4-12% with varying doses of Cas9 RNP) (Figure 7D). Sanger sequencing of the target *CXCR4* genomic site, performed to directly identify editing events, suggested that the T7E1 assay may have underestimated editing efficiency. The T7E1 assay utilizes denaturation and hybridization of the wild-type and mutant sequences to create a mismatch DNA duplex which is then digested by T7 endonuclease. However, hybridization of the mismatch duplex may be inefficient, especially when the indel mutation is drastically different from the wild-type sequence, making self-hybridization an energetically more favorable product. Other potential reasons for observed underestimation of editing efficiency with endonuclease assays include incomplete duplex melting, inefficient cleavage of single base pair indels, and deviation from the expected 300 and 600 basepair products on the agarose gel as a result of large genome edits (23). Sequencing of the *CXCR4* gene in CXCR4^{lo} cells showed that 5/6 clones had mutations/deletions whereas such mutations/deletions were observed in only 4/10 clones and 0/9 clones in CXCR4^{hi} and CTRL treated CXCR4^{lo} cells, respectively. Importantly, none of the observed edits in the CXCR4^{hi} population terminated the coding sequence (one missense mutation and three in-frame deletions), consistent with the maintenance of protein expression. By contrast, the CXCR4^{lo} population was enriched for cells with a more extensive mutational burden in the locus (Figure 7E). These findings demonstrated successful genomic targeting with Cas9 RNPs and a functional effect on protein expression in human CD4⁺ T cells. FACS was able to enrich the population of edited cells, providing an additional useful tool for Cas9 RNP applications in primary T cells.

**Efficient genetic 'knock-in' with homology-directed repair (HDR).** Exogenous template-mediated HDR is a powerful technique for precise gene modifications that can enable experimental and therapeutic editing of specific variant sequences. Given the high editing efficiency of Cas9 RNPs, we next tested whether exogenous template-mediated HDR in primary T cells could be achieved. A single-stranded oligonucleotide DNA template (HDR template) with 90 nucleotide homology arms was used to recombine with the *CXCR4* locus at the Cas9 RNP cleavage site (16). The CXCR4 HDR template was designed to replace 12 nucleotides from the human reference genome, including the protospacer adjacent motif (PAM) sequence required for CRISPR-mediated DNA cleavage, and introduce a HindIII restriction enzyme cleavage site (Figure 8A). Cas9 RNPs were electroporated into primary CD4⁺ T cells in the presence of four different concentrations of CXCR4 HDR template (0, 50, 100 and 200 pmol; see Supplementary Information Materials and Methods). Cas9 RNP without HDR template again reduced the percentage of CXCR4^{hi} cells. Notably, in this experiment, addition of the CXCR4 HDR template improved the efficacy of CXCR4 ablation, although this effect on cell surface expression was not seen in all experiments (Figure 9A). In the experiment shown here, ∼60% of cells lost high-level cell surface CXCR4 expression with 100 pmol HDR template and Cas9 RNP (1% vs. 60% in control treated cells) (Figure 8B and C).

Highly efficient HDR was observed in cells treated with Cas9 RNP and the single stranded oligonucleotide HDR template (Figure 8D). Up to 33% total editing (defined as the sum of all NHEJ and HDR events that give rise to indels at Cas9 cleavage site) was observed in the presence of 50 pmol CXCR4 HDR template, as estimated by T7E1 assays. At this concentration, 14% HDR was estimated by HindIII digest of the target locus, indicating that a high fraction of editing resulted from HDR (see results below for further quantification). The nearly complete loss of CXCR4 staining with addition of the HDR template suggests that the mutation introduced by HDR (84DLLFV88→ 84ESLDP88) strongly affected the cell surface expression of CXCR4 or its recognition by the antibody (Figure 8B and C). The editing efficiency was reduced with 200 pmol HDR template, perhaps as a result of cellular toxicity.

Both total editing and HDR could be enriched by sorting the CXCR4^{lo} population, although the effect was less pronounced than in Figure 7, consistent with the larger fraction of CXCR4^{lo} cells in the unsorted population. Note that in these experiments a more stringent gate was applied to separate the cells with the highest expression of CXCR4, and in this CXCR4^{hi} population no editing was observed. These studies collectively demonstrated the power of Cas9 RNPs coupled with single-stranded oligonucleotide HDR template to precisely replace targeted DNA sequences in primary human T cells.

**Deep Sequencing of Target Genomic DNA.** Deep sequencing of the targeted *CXCR4* locus allowed more detailed and quantitative analysis of genome editing events. The results highlighted in Figure 10 show the frequency of insertions, deletions and HDR-mediated nucleotide replacement in CXCR4 Cas9 RNP-treated cells with or without CXCR4 HDR template compared to control-treated cells. In CXCR4 Cas9 RNP treated cells, we found 55% of reads overlapping the *CXCR4* target site containing at least one indel within a 200 nucleotide window centered around the expected cut site (Figure 10A, B). As discussed above, the T7E1 assays are useful for identifying edited loci, but may underestimate actual editing efficiency (quantitation of the T7E1 assay in Figure 8D suggested 33% editing compared to the 55% editing efficiency computed by deep-sequencing). We also sequenced the two top predicted 'off-target' sites for the CXCR4 Cas9 RNP (Figure 10B). Rare indels were observed at both off-target sites (∼1-2%), but at a rate comparable to that observed for those sites in the control cells treated with Cas9 protein only (∼1-2%).

The deep sequencing results allowed quantitative analysis of observed indel mutations and their spatial distribution in the target region. Consistent with reports that *S*. *pyogenes* Cas9 cuts ∼3 nucleotides upstream from the PAM sequence, we found the highest frequency of indels at 4 nucleotides upstream of the PAM (Figure 10A). Indels were distributed throughout the sequenced region (Figure 10C and D) with the majority of events near cut sites (>94% within 40 nucleotides). In CXCR4 Cas9 RNP treated cells within +/-100 nucleotides from the cut site, we observed 95% of reads with indels contained a deletion event while 10% contained an insertion event. Interestingly, of the reads with insertion events, ∼50% also contained at least one deletion. We observed a wide range of insertion and deletion sizes, with many reads exhibiting deletions up to -80 nucleotides in length (mean 18 nucleotides, SD 15 nucleotides) and some insertions up to -55 nucleotides in length (mean 4.4 nucleotides, SD 4.8 nucleotides) (Figure 10C, D and 11). This range of indel sizes and locations was consistent with the extensive mutational burden observed in Sanger sequencing of CXCR4^{lo} selected cells in Figure 7.

Deep sequencing verified the successful targeted replacement of 12 nucleotides at the *CXCR4* locus, only in cells treated with both Cas9 RNPs and CXCR4 HDR template. We observed 25% incorporation of HDR template sequence with 50 pmol HDR template and 21% with 100 pmol HDR template (Figure 10A). Of the reads with HDR template sequence incorporated, ∼14% of the detected HDR template reads had additional non-specific indels surrounding the incorporated HindIII site or other imperfect forms of editing within the 200 nucleotide window centered at the predicted cut site. However, the frequency of indels in reads with the HindIII site incorporated was reduced compared to reads where the HindIII site was not detected (Figure 10C, D and 11). Interestingly, there was a consistent pattern of deletion events between CXCR4 Cas9 RNP with and without CXCR4 HDR template with an enrichment of deletions of 2 nucleotides (11%) and 22 nucleotides (5.4%) (Figure 11). Replacement of the PAM sequence likely helped to limit re-cutting of 'knock-in' sequence. Overall, 18-22% of reads (with varying concentrations of HDR template) had correctly replaced nucleotides throughout the sequenced genomic target site, suggesting that this approach could prove useful for generation of experimental and therapeutic nucleotide 'knock-in' primary human T cells.

**Specific 'knock-in' Targeting of Key Cell Surface Receptors.** To confirm that Cas9 RNPs mediate HDR at other genomic sites, we designed a guide RNA and HDR template to target the *PD-1* (*PDCD1*) locus. PD-1 is an 'immune checkpoint' cell surface receptor found on the surface of chronically activated or exhausted T cells that can inhibit effective T cell-mediated clearance of cancers. Monoclonal antibody blockade of PD-1 is approved for treatment of advanced malignancy, and genetic deletion of *PD-1* may prove useful in engineering T cells for cell-based cancer immunotherapies (12). Primary human T cells were electroporated with a PD-1 Cas9 RNP and a PD-1 HDR template designed to generate a frameshift mutation and 'knock-in' a HindIII restriction site in the first exon of *PD-1* thereby replacing the PAM sequence (Figure 12A).

To examine the specificity of Cas9 RNP-mediated targeting, we compared PD-1 cell surface expression following treatment with PD-1 Cas9 RNP versus CXCR4 Cas9 RNP (which should not target the *PD-1* locus) or scrambled guide Cas9 RNP (no predicted cut within the human genome). We performed replicate experiments side-by-side with two different blood donors and with sgRNAs generated with two different *in vitro* transcription protocols (see Supplementary Information Materials and Methods). PD-1 Cas9 RNPs electroporated with PD-1 HDR template significantly reduced the percentage of cells with high PD-1 cell surface expression relative to both CXCR4 Cas9 RNPs and scrambled guide Cas9 RNPs delivered with PD-1 HDR template (Figure 12B). Similarly, CXCR4 Cas9 RNPs and CXCR4 HDR template caused a decrease in the CXCR4^{hi} cell population relative to both PD-1 and scrambled guide Cas9 RNP treatments with CXCR4 HDR template (Figure 12C). Loss of CXCR4 was not a non-specific effect of single-stranded DNA delivered along with CXCR4 Cas9 RNP; we observed a higher percentage of CXCR4 expressing cells after treatment with CXCR4 Cas9 RNP and scrambled HDR template than with CXCR4 Cas9 RNP and CXCR4 HDR template (Figure 9A). These findings confirmed the target-specific modulation of cell surface receptor expression in primary T cells with the programmable Cas9 RNP and HDR template treatments.

We then tested the specificity of HDR templates for nucleotide replacement (Figure 12D; examples of corresponding cell surface expression data are shown in Figure 9B). As expected, we observed efficient *PD-1* editing by PD-1 Cas9 RNPs regardless of whether they were delivered with PD-1 HDR template, CXCR4 HDR template or without any HDR template. In contrast, the HindIII site was only incorporated into *PD-1* in the presence of both PD-1 Cas9 RNP and PD-1 HDR template, but not with CXCR4 HDR template, which should not be recombined at *PD-1* locus due to the lack of sequence homology. Similarly, a HindIII site was only incorporated into *CXCR4* following treatment with CXCR4 Cas9 RNP and CXCR4 HDR template; HDR was not observed at the *CXCR4* locus with PD-1 HDR template, control scrambled HDR template (with a HindIII site) or without HDR template (Figure 12D). Taken together, these studies established that specific pairing of a programmed Cas9 RNP and corresponding HDR template can provide for targeted nucleotide replacement in primary human T cells.

### Materials and Methods

**Human** T **cell isolation and culture.** Human primary T cells were either isolated from fresh whole blood or buffy coats. Peripheral blood mononuclear cells (PBMCs) were isolated by Ficoll gradient centrifugation. CD4⁺ T cells were pre-enriched with Easysep Human CD4⁺ T cell enrichment kit (Stemcell technologies) according to the manufacturer's protocol. Pre-enriched CD4⁺ T cells were stained with following antibodies: αCD4-PerCp (SK3; Becton Dickinson), aCD25-APC (BC96; TONBO Biosciences), aCD127-PE (R34-34; TONBO Biosciences), aCD45RA-violetFluor450 (HI100; TONBO Biosciences) and αCD45RO-FITC (UCHL1; TONBO Biosciences). CD4⁺CD2S^{lo}CD127^{hi} T effectors (Teffs) were isolated using a FACS Aria Illu (Becton Dickinson).

**Cas9 RNP assembly and electroporation.** Cas9 RNPs were prepared immediately before experiments by incubating 20 µM Cas9 with 20 µM sgRNA at 1:1 ratio in 20 µM HEPES (pH 7.5), 150 mM KCl, 1 mM MgCl₂, 10% glycerol and 1 mM TCEP at 37°C for 10 min to a final concentration of 10 µM. T cells were electroporated with a Neon transfection kit and device (Invitrogen).

**Analysis of Genome Editing.** Editing efficiency was estimated by T7 endonuclease I assay. HDR templates were designed to introduce a HindIII restriction site into the targeted gene loci; successful HDR was confirmed with HindIII restriction enzyme digestion. The genomic DNA library, flanking the regions of Cas9 target sites for the CXCR4 on-target and two predicted off-target genes, was assembled by 2-step PCR method and sequenced with the Illumina HiSeq 2500.

### Supplementary Information Materials and Methods

**Human T cell isolation and culture.** Human primary T cells were either isolated from fresh whole blood or buffy coats (Stanford Blood Center). Whole blood was collected from human donors into sodium heparinized vacutainer tubes (Becton Dickinson), with approval by the UCSF Committee on Human Research (CHR), and processed within 12 hrs. Peripheral blood mononuclear cells (PBMCs) were isolated by Ficoll gradient centrifugation. Fresh blood was mixed in a 1:1 ratio with Ca²⁺ and Mg²⁺ free Hank's balanced salt solution (HBSS), Buffy coats were diluted in a 1:10 ratio with HBSS. 30 ml of the respective HBSS/blood solution were transferred to 50 ml Falcon tubes and underlayed with 12 ml Ficoll-Paque PLUS (Amersham/GE healthcare). After density gradient centrifugation (1000g, 20 min, no brakes) the PBMC layer was carefully removed and the cells washed twice with Ca²⁺ and Mg²⁺ free HBSS. CD4⁺ T cells were pre-enriched with Easysep Human CD4⁺ T cell enrichment kit (Stemcell technologies) according to the manufacturer's protocol. Pre-enriched CD4⁺ T cells were stained with following antibodies: aCD4-PerCp (SK3; Becton Dickinson), aCD25-APC (BC96; TONBO Biosciences), αCD127-PE (R34-34; TONBO Biosciences), aCD45RA-violetFluor450 (HI100; TONBO Biosciences) and αCD45RO-FITC (UCHL1; TONBO Biosciences). CD4⁺CD25^{lo}CD127^{hi} T effectors (Teffs) were isolated using a FACS Aria Illu (Becton Dickinson). Teff purity was > 97%.

For Cas9 RNP transfections, the effector CD4⁺ T cells were isolated from whole blood were pre-activated on aCD3 (UCHT1; BD Pharmingen) and aCD28 (CD28.2; BD Pharmingen) coated plates for 48 hrs. Plates were coated with 10 µg/ml aCD3 and aCD28 in PBS for at least 2 hrs at 37°C. Buffy coat derived T cells were activated on plates coated with 10 µg/ml aCD3 (in PBS for at least 2 hrs at 37°C) with 5 µg/ml aCD28 added directly to the RPMI complete medium.

The T cells were activated in RPMI complete (RPMI-1640 (UCSF CCF) supplemented with 5 mmol/14-(2-hydroxyethyl)-1-piperazineethanesulfonic acid (HEPES) (UCSF CCF), 2 mmol/l Glutamax (Gibco), 50 µg/ml penicillin/streptomycin (Coming), 50 µmol/l 2-mercaptoethanol (Sigma-Aldrich), 5 mmol/l nonessential amino acids (Coming), 5 mmol/l sodium pyruvate (UCSF CCF), and 10% (v/v) fetal bovine serum (Atlanta Biologicals)). After electroporation the medium was supplemented with 40 IU/ml IL-2.

**Expression and Purification of Cas9.** The recombinant *S*. *pyogenes* Cas9 used in this study carries at the C-terminus an HA tag and two nuclear localization signal peptides which facilitates transport across nuclear membrane. The protein was expressed with a N-terminal hexahistidine tag and maltose binding protein in *E. coli* Rosetta 2 cells (EMD Millipore) from plasmid pMJ915. The His tag and maltose binding protein were cleaved by TEV protease, and Cas9 was purified by the protocols described in Jinek M, et al. (2012) A programmable dual-RNA-guided DNA endonuclease in adaptive bacterial immunity. Science 337(6096):816-821. Cas9 was stored in 20 mM HEPES at pH 7.5, 150 mM KCl, 10% (v/v) glycerol, 1 mM tris(2-chloroethyl) phosphate (TCEP) at -80°C.

***In vitro* T7 transcription of sgRNA with PAGE purification.** The DNA template encoding for a T7 promoter, a 20 nucleotide (nt) target sequence and the chimeric sgRNA scaffold was assembled from synthetic oligonucleotides by overlapping PCR. Briefly, for the CXCR4 sgRNA template, the PCR reaction contains 20 nM premix of SLKS3 (5'- TAA TAC GAC TCA CTA TAG GAA GCG TGA TGA CAA AGA GGG TTT TAG AGC TAT GCT GGA AAC AGC ATA GCA AGT TAA AAT AAG G -3') and SLKS1 (5'- GCA CCG ACT CGG TGC CAC TTT TTC AAG TTG ATA ACG GAC TAG CCT TAT TTT AAC TTG CTA TGC TGT TTC CAG C -3'), 1 µM premix of T25 (5'-TAA TAC GAC TCA CTA TAG-3') and SLKS1 (5'- GCA CCG ACT CGG TGC CAC TTT TTC AAG -3'), 200 µM dNTP and Phusion Polymerase (NEB) according to manufacturer's protocol. The thermocycler setting consisted of 30 cycles of 95°C for 10 sec, 57°C for 10 sec and 72°C for 10 sec. The PCR product was extracted once with phenol:chloroform:isoamylalcohol and then once with chloroform, before isopropanol precipitation overnight at -20°C. The DNA pellet was washed three times with 70% (v/v) ethanol, dried by vacuum and dissolved in diethylpyrocarbonate (DEPC)-treated water. The PD-1 sgRNA template was assembled from T25, SLKS1, SLKS2 and SLKS11 (5'- TAA TAC GAC TCA CTA TAG CGA CTG GCC AGG GCG CCT GTG TTT TAG AGC TAT GCT GGA AAC AGC ATA GCA AGT TAA AAT AAG G -3') by the same procedure.

A 100-µl T7 *in vitro* transcription reaction consisted of 30 mM Tris-HCl (pH 8), 20 mM MgCl₂, 0.01% (v/v) Triton X-100, 2 mM spermidine, 10 mM fresh dithiothreitol, 5 mM of each ribonucleotide triphosphate, 100 µg/ml T7 Pol and 0.1 µM DNA template. The reaction was incubated at 37°C for 4 h, and 5 units of RNase-free DNaseI (Promega) was added to digest the DNA template 37°C for 1 h. The reaction was quenched with 2xSTOP solution (95% (v/v) deionized formamide, 0.05% (w/v) bromophenol blue and 20 mM EDTA) at 60°C for 5 min. The RNA was purified by electrophoresis in 10% (v/v) polyacrylamide gel containing 6 M urea. The RNA band was excised from the gel, grinded up in a 50 ml tube, and eluted overnight in 25 mls of 300 mM sodium acetate (pH 5) overnight at 4°C with gentle rocking. The solution was then centrifuged at 4000g for 10 minutes and the RNA supernatant was passed through a 0.45 µm filter. One equivalent of isopropanol was added to the filtered supernatant to precipitate the RNA overnight at -20°C. The RNA pellet was collected by centrifugation, washed three times with 70% (v/v) ethanol, and dried by vacuum. To refold the sgRNA, the RNA pellet was first dissolved in 20 mM HEPES (pH 7.5), 150 mM KCl, 10% (v/v) glycerol and 1 mM TCEP. The sgRNA was heated to 70°C for 5 min and cooled to room temperature. MgCl₂ was added to a final concentration of 1 mM. The sgRNA was again heated to 50°C for 5 min, cooled to room temperature and kept on ice. The sgRNA concentration was determined by OD₂₆₀ₙₘ using Nanodrop and adjusted to 100 µM using 20 mM HEPES (pH 7.5), 150 mM KCl, 10% (v/v) glycerol, 1 mM TCEP and 1 mM MgCl₂. The sgRNA was store at -80°C.

***In vitro* T7 transcription of sgRNA with Phenol/chloroform extraction.** DNA templates for *in vitro* T7 transcription were generated by annealing complementing single-stranded ultramers (Ultramer sequences: CXCR4_1: 5'- TAA TAC GAC TCA CTA TAG GAA GCG TGA TGA CAA AGA GGG TTT TAG AGC TAT GCT GGA AAC AGC ATA GCA AGT TAA AAT AA GGC TAG TCC GTT ATC AAC TTG AAA AAG TGG CAC CGA GTC GGT G-3'; CXCR4_2: 5'- CAC CGA CTC GGT GCC ACT TTT TCA AGT TGA TAA CGG ACT AGC CTT ATT TTA ACT TGC TAT GCT GTT TCC AGC ATA GCT CTA AAA CCC TCT TTG TCA TCA CGC TTC CTA TAG TGA GTC GTA TTA-3'; PD-1_1: 5'- TAA TAC GAC TCA CTA TAG CGA CTG GCC AGG GCG CCT GTG TTT TAG AGC TAT GCT GGA AAC AGC ATA GCA AGT TAA AAT AAG GCT AGT CCG TTA TCA ACT TGA AAA AGT GGC ACC GAG TCG GTG C-3'; PD-1_2: 5'- GCA CCG ACT CGG TGC CAC TTT TTC AAG TTG ATA ACG GAC TAG CCT TAT TTT AAC TTG CTA TGC TGT TTC CAG CAT AGC TCT AAA ACA CAG GCG CCC TGG CCA GTC GCT ATA GTG AGT CGT ATT A-3'). Ultramers were mixed in 1:1 ratio in nucleasefree duplex buffer (IDT), heated up to 95°C for 2 min followed by a 30 min incubation at RT.

A 100-µl T7 *in vitro* transcription reaction contained 1x Transcription Optimized buffer (Promega), 10 mM fresh dithiothreitol, 2 mM of each ribonucleotide triphosphate, 400U T7 Pol (Promega), 0.5U pyrophosphatase (Life technologies) and 2 µg DNA template. The reaction was incubated for 4 h at 37°C. 5U of RNase-free DNaseI (Promega) were added to digest the DNA template at 37°C for 30 min. The reaction was stopped with 5 µl 0.5M EDTA.

Given concern for the possibility of nucleic acid exchange between wells during PAGE purification, we tested phenol/chloroform purified sgRNAs side-by-side with PAGE purified sgRNAs as indicated in Figure 12 and 9A. Phenol/chloroform extraction was performed after addition of 190 µl RNAs-free H₂O. sgRNA was precipitated with 80 µl 3M sodium acetate and 420 µl isoproponal and incubation at -20°C for 4 hrs. The RNA pellet was washed twice with 70% (v/v) EtOH and once with 100% (v/v) EtOH. The vacuum dried pellet was reconstituted and the sgRNAs refolded as described in *"In vitro* T7 transcription of sgRNA with PAGE purification".

**Cas9 RNP assembly and electroporation.** Cas9 RNPs were prepared immediately before experiments by incubating 20 µM Cas9 with 20 µM sgRNA at 1:1 ratio in 20 µM HEPES (pH 7.5), 150 mM KCl, 1 mM MgCl₂, 10% (v/v) glycerol and 1 mM TCEP at 37°C for 10 min to a final concentration of 10 µM.

T cells were electroporated with a Neon transfection kit and device (Invitrogen). 2.5 x 10⁵ T cells were washed three times with PBS before resuspension in 8 µl of buffer T (Neon kit, Invitrogen). Cas9 RNP (2 µl of 10 µM Cas9 CTRL without sgRNA or 1 - 2 µl Cas9:sgRNA RNP; final concentration 0.9 - 1.8 µM) as well as HDR template (0 - 200 pmol as indicated) were added to the cell suspension to a final volume of 11 µl (adjusted with Cas9 storage buffer), and mixed. 10 µl of the suspension were electroporated with a Neon electroporation device (Invitrogen; 1600V, 10 msec, 3 pulses). The HDR templates for CXCR4 and PD-1 are a single-stranded oligonucleotide complementary (antisense strand) to the target sequence, and contain a HindIII restriction sequence along with 90-nt homology arms. Upon successful HDR the respective PAM sites are deleted, which should prevent recutting of the edited site by the Cas9 RNPs. The PD-1 HDR template additionally causes a frameshift and nonsense mutation as early as amino acid position 25 by replacing 12 nt with 11 nt (CXCR4 HDR template: 5'-GGG CAA TGG ATT GGT CAT CCT GGT CAT GGG TTA CCA GAA GAA ACT GAG AAG CAT GAC GGA CAA GTA CAG GCT GCA CCT GTC AGT GGC CGA AAG CTT GGA TCC CAT CAC GCT TCC CTT CTG GGC AGT TGA TGC CGT GGC AAA CTG GTA CTT TGG GAA CTT CCT ATG CAA GGC AGT CCA TGT CAT CTA CAC AGT-3'; PD-1 HDR template: 5' -AAC CTG ACC TGG GAC AGT TTC CCT TCC GCT CAC CTC CGC CTG AGC AGT GGA GAA GGC GGC ACT CTG GTG GGG CTG CTC CAG GCA TGC AGA TAA TGA AAG CTT CTG GCC AGT CGT CTG GGC GGT GCT ACA ACT GGG CTG GCG GCC AGG ATG GTT CTT AGG TAG GTG GGG TCG GCG GTC AGG TGT CCC AGA GC-3'). The CXCR4 HDR control donor is a sequence scrambled version on the original CXCR4 HDR template containing a HindIII restriction site (CXCR4 control HDR template: 5'- TTC AAA ACT AGC GTC AGG GGC TCG ATT TAC TCG GGA CTT GCT ACA ACA TCG CAG TCA CGC GCA CGA TCC TTC CAG GAT TGG AGG TGG ACT TAG ATA AAG CTT CCG TGT GCA CCG TAT AGA TTC GTT GAT GCA GGC TAT TCC CGT GAT CCC ACG CGG AGG TGA TGG AGC GTC AAG CAT AGC TAG CAC AGA TGA -3')

Electroporated T cells were transferred to 500 µl of their respective culture medium in a aCD3/CD28 coated 48-well plate. Plates were coated with 10 µg/ml αCD3 (UCHT1; BD Pharmingen) and αCD28 (CD28.2; BD Pharmingen) in PBS for at least 2 hrs at 37°C. 24 hrs after electroporation cells were resuspended and transferred to a non-coated well plate. 3-4 days after electroporation, T cells were analyzed by FACS and T7 endonuclease I assay.

**FACS analysis of edited T cells.** Cell surface staining was performed with αCXCR4-APC (12G5; BD Pharmingen) and αPD-1-PE (EH12.2H7; Biolegend) for 15 min on ice. Cells were kept at 4°C throughout the staining procedure until cell sorting to minimize antibody-mediated internalization and degradation of the antibody. Cells were sorted using a FACS Aria Illu (Becton Dickinson).

**PCR amplification of target region.** 5x10⁴ - 2x10⁵ cells were resuspended in 100 µl of Quick Extraction solution (Epicenter) were added to lyse the cells and extract the genomic DNA. The cell lysate was incubated at 65°C for 20 min and then 95°C for 20 min, and stored at -20°C. The concentration of genomic DNA was determined by NanoDrop (Thermo Scientific).

Genomic regions, containing the *CXCR4* or *PD-1* target sites, were PCR amplified using the following primer sets. For CXCR4: forward 5'- AGA GGA GTT AGC CAA GAT GTG ACT TTG AAA CC -3' and reverse 5'- GGA CAG GAT GAC AAT ACC AGG CAG GAT AAG GCC -3' (938 bp). For PD-1: forward 5'- GGG GCT CAT CCC ATC CTT AG-3' and reverse 5'- GCC ACA GCA GTG AGC AGA GA-3' (905 bp). Both primer sets were designed to avoid amplifying the HDR templates by annealing outside of the homology arms. The PCR reaction contained 200 ng of genomic DNA and Kapa Hot start high-fidelity polymerase (Kapa Biosystems) in high GC buffer according to the manufacturer's protocol. The thermocycler setting consisted of one cycle of 95°C for 5 min, 35 cycles of 98°C for 20 sec, 62°C for *CXCR4* or 68°C for *PD-1* for 15 sec and 72°C for 1 min, and one cycle of 72°C for 1 min. The PCR products were purified on 2% (w/v) agarose gel containing SYBR Safe (Life Technologies). The PCR products were eluted from the agarose gel using QIAquick gel extraction kit (Qiagen). The concentration of PCR DNA was quantitated with a NanoDrop device (Thermo scientific). 200 ng of PCR DNA was used for T7 endonuclease I and HindIII analyses. For Figure 7E, PCR product was cloned with TOPO Zero Blunt PCR Cloning Kit (Invitrogen) and submitted for Sanger sequencing.

**Analysis of Editing Efficiency by T7 endonuclease I assay.** Editing efficiency was estimated by T7 endonuclease I assay. T7 endonuclease I recognizes and cleaves mismatched heteroduplex DNA that arises from hybridization of wild-type and mutant DNA strands. The hybridization reaction contained 200 ng of PCR DNA in KAPA high GC buffer and 50 mM KCl, and was performed on a thermocycler with the following setting: 95°C, 10 min, 95-85°C at -2°C/sec, 85°C for 1 min, 85-75°C at -2°C/sec, 75°C for 1 min, 75-65°C at - 2°C/sec, 65°C for 1 min, 65-55°C at -2°C/sec, 55°C for 1 min, 55-45°C at -2°C/sec, 45°C for 1 min, 45-35°C at -2°C/sec, 35°C for 1 min, 35-25°C at -2°C/sec, 25°C for 1 min, and hold at 4°C. Buffer 2 and 5 units of T7 endonuclease I (NEB) were added to digest the re-annealed DNA. After 1 hr of incubation at 37°C, the reaction was quenched with 6x blue gel loading dye (Thermo Scientific) at 70°C for 10 min. The product was resolved on 2% agarose gel containing SYBR gold (Life technologies). The DNA band intensity was quantitated using Image Lab. The percentage of editing was calculated using the following equation (1 - (1 - (b + c / a + b +c))^{1/2}) x 100, where "a" is the band intensity of DNA substrate and "b" and "c" are the cleavage products. For the quantification of the *PD-1* T7E1 assay (Figure 12D), the intensity of the DNA substrate was calculated as the sum of the two large bands seen in all conditions. Calculation of the % Total Edit based on T7E1 assays allows only an estimate of cleavage efficiency.

**Analysis of HDR by HindIII restriction digestion.** HDR templates were designed to introduce a HindIII restriction site into the targeted gene locus. To test for successful introduction of the HindIII site into the *CXCR4* locus, 938 bp region was PCR amplified using the primers 5'- AGA GGA GTT AGC CAA GAT GTG ACT TTG AAA CC -3' and 5'-GGA CAG GAT GAC AAT ACC AGG CAG GAT AAG GCC -3'. For the *PD-1* locus a 905 bp region was amplified using the primers 5'- GGG GCT CAT CCC ATC CTT AG -3' and 5'-GCC ACA GCA GTG AGC AGA GA-3'. The reaction consisted of 200 ng of PCR DNA and 10 units of HindIII High Fidelity in CutSmart Buffer (NEB). After 2 hr of incubation at 37°C, the reaction was quenched with one volume of gel loading dye at 70°C for 10 min. The product was resolved on 2% (w/v) agarose gel containing SYBR gold (Life technologies). The band intensity was quantitated using Image Lab. The percentage of HDR was calculated using the following equation (b + c / a + b +c) x 100, where "a" is the band intensity of DNA substrate and "b" and "c" are the cleavage products.

**Deep sequencing analysis of on-target and off-target sites.** The genomic regions flanking the Cas9 target site for the CXCR4 on-target and two off-target genes were amplified by 2-step PCR method using primers listed below. CXCR4 on-target (5'- ACA CTC TTT CCC TAC ACG ACG CTC TTC CGA TCT NNN NNC TTC CTG CCC ACC ATC TAC TCC ATC ATC TTC TTA ACT G-3' and 5'- GTG ACT GGA GTT CAG ACG TGT GCT CTT CCG ATC TNN NNN CAG GTA GCG GTC CAG ACT GAT GAA GGC CAG GAT GAG GAC-3'), off-target #1 (*POU domain, class 2, transcription factor 1 isoform 1* [*POU2F1*] locus; 5'- ACA CTC TTT CCC TAC ACG ACG CTC TTC CGA TCT NNN NNG CTA TAA TAG TAC AAG TAT ATG TTA AAT AAG AGT CAT AGC ATG-3' and 5'- GTG ACT GGA GTT CAG ACG TGT GCT CTT CCG ATC TNN NNN CTG GCT TTA TAT ATA TAC ATA GAT AGA CGA TAT AGA TAG C-3') and off-target #2 *(glutamate receptor 1 isoform 1 precursor* [*GRIA1*] locus; 5'- ACA CTC TTT CCC TAC ACG ACG CTC TTC CGA TCT NNN NNC CTG GTC CCA GCC CAG CCC CAG CTA TTC AGC ATC C-3' and 5'- GTG ACT GGA GTT CAG ACG TGT GCT CTT CCG ATC TNN NNN ACT CTG CAC TGG TAT ATC AAT ACA CTT GTT TTT CTC ATC CC-3'). First, 100-150 ng of the genomic DNA from the edited and control samples was PCR amplified using Kapa Hot start high-fidelity polymerase (Kapa Biosystems) according to the manufacturer's protocol. The thermocycler setting consisted of one cycle of 95°C for 5 min and 15-20 cycles of 98°C for 20 sec, 63°C for 15 sec and 72°C for 15 sec, and one cycle of 72°C for 1 min. The resulting amplicons were resolved on 2% (w/v) agarose gel, stained with SYBR Gold and gel extracted using Qiagen gel extraction kit.

Illumina TruSeq Universal adapter (5'- AAT GAT ACG GCG ACC ACC GAG ATC TAC ACT CTT TCC CTA CAC GAC GCT CTT CCG ATC T-3') and modified Illumina RNA PCR barcode primer (5'- CAA GCA GAA GAC GGC ATA CGA GAT-Index- GTG ACT GGA GTT CAG ACG TGT GCT CTT CCG ATC T-3') were attached to the amplicon in the second PCR step using Kapa Hot start high-fidelity polymerase (Kapa Biosystems). The thermocycler setting consisted of one cycle of 98°C for 30 sec, 8-10 cycles of 98°C for 20 sec, 65°C for 15 sec and 72°C for 15 sec, and one cycle of 72°C for 5 min. The resulting amplicons were resolved on 2% (w/v) agarose gel, stained with SYBR Gold and gel extracted using Qiagen gel extraction kit. Barcoded and purified DNA samples were quantified by Qubit 2.0 Fluorometer (Life Technologies), size analyzed by BioAnalyzer (Agilent), quantified by qPCR and pooled in an equimolar ratio. Sequencing libraries were sequenced with the Illumina HiSEq 2500.

**Analysis of deep-sequencing data.** Sequencing reads that contained the unique 12 nt resulting from the HDR template were extracted and analyzed separately from those that did not contain HDR template-derived sequence. All reads that did not contain the replaced 12 nt were aligned to the reference hg19 genome, and all of the reads that contained the replaced 12 nt were aligned to a modified hg19 genome with the expected substitutions using Burrows-Wheeler Aligner (BWA). The samtools mpileup utility was then used to quantify the total number of reads that mapped to each position of the *CXCR4* gene, and a custom script examining the CIGAR string was used to estimate the number and locations of insertions and deletions for each read. Insertion efficiency was estimated for experiment with CXCR4 RNP (without HDR template) as: (number of reads with insertions +/- 100 bp from cut site) / (total number of reads +/- from cut site). For Deletion efficiency for experiment with CXCR4 RNP (without HDR template) was estimated as: (number of reads with deletions +/- 100 bp from cut site) / (total number of reads +/- from cut site). For experiments with CXCR4 RNP + HDR template, Insertion and Deletion efficiencies were calculated based only on reads that that did not contain the 12 nt replacement derived from HDR (these are the fractions shown in Figure 10B). Total editing efficiency was estimated as (number of reads with indels +/- 100 bp from cut site) / (total number of reads +/- from cut site). HDR efficiency was estimated as (number of reads containing HindIII site +/- 100bp from cut site) / (total number of reads +/- 100 bp from cut site). Distribution of insertion and deletion sizes were estimated for a region +/- 20 bp from the cut site. Deep sequencing data is available at the NCBI Sequence Read Archive (SRA, BioProject: SUB996236).

### References

1. Doudna JA & Charpentier E (2014) Genome editing. The new frontier of genome engineering with CRISPR-Cas9. Science 346(6213): 1258096.
2. Hsu PD, Lander ES, & Zhang F (2014) Development and applications of CRISPR-Cas9 for genome engineering. Cell 157(6):1262-1278.
3. Mandal PK, et al. (2014) Efficient Ablation of Genes in Human Hematopoietic Stem and Effector Cells using CRISPR/Cas9. Cell Stem Cell 15(5):643-652.
4. Maus MV, et al. (2014) Adoptive immunotherapy for cancer or viruses. Annual Review of Immunology 32:189-225.
5. Passerini L, et al. (2013) CD4(+) T cells from IPEX patients convert into functional and stable regulatory T cells by FOXP3 gene transfer. Science Translational Medicine 5(215):215ra174.
6. Hutter G, et al. (2009) Long-term control of HIV by CCR5 Delta32/Delta32 stemcell transplantation. The New England Journal of Medicine 360(7):692-698.
7. Didigu CA, et al. (2014) Simultaneous zinc-finger nuclease editing of the HIV coreceptors ccr5 and cxcr4 protects CD4+ T cells from HIV-1 infection. Blood 123(1):61-69.
8. Tebas P, et al. (2014) Gene editing of CCR5 in autologous CD4 T cells of persons infected with HIV. The New England Journal of Medicine 370(10):901-910.
9. Restifo NP, Dudley ME, & Rosenberg SA (201.2) Adoptive immunotherapy for cancer: harnessing the T cell response. Nature Reviews. Immunology 12(4):269-281.
10. Porter DL, Levine BL, Kalos M, Bagg A, & June CH (2011) Chimeric antigen receptor-modified T cells in chronic lymphoid leukemia. The New England Journal of Medicine 365(8):725-733.
11. Moon EK, et al. (2014) Multifactorial T-cell hypofunction that is reversible can limit the efficacy of chimeric antigen receptor-transduced human T cells in solid tumors. Clinical Cancer Research 20(16):4262-4273.
12. Topalian SL, Drake CG, & Pardoll DM (2015) Immune Checkpoint Blockade: A Common Denominator Approach to Cancer Therapy. Cancer Cell 27(4):450-461.
13. John LB, et al. (2013) Anti-PD-1 antibody therapy potently enhances the eradication of established tumors by gene-modified T cells. Clinical Cancer Research 19(20):5636-5646.
14. Genovese P, et al. (2014) Targeted genome editing in human repopulating haematopoietic stem cells. Nature 510(7504):235-240.
15. Kim S, Kim D, Cho SW, Kim J, & Kim JS (2014) Highly efficient RNA-guided genome editing in human cells via delivery of purified Cas9 ribonucleoproteins. Genome Research 24(6): 1012-1019.
16. Lin S, Staahl B, Alla RK, & Doudna JA (2014) Enhanced homology-directed human genome engineering by controlled timing of CRISPR/Cas9 delivery. eLife 3.
17. Zuris JA, et al. (2014) Cationic lipid-mediated delivery of proteins enables efficient protein-based genome editing in vitro and in vivo. Nature Biotechnology*.*
18. Sung YH, et al. (2014) Highly efficient gene knockout in mice and zebrafish with RNA-guided endonucleases. Genome Research 24(1): 125-131.
19. Zou YR, Kottmann AH, Kuroda M, Taniuchi I, & Littman DR (1998) Function of the chemokine receptor CXCR4 in haematopoiesis and in cerebellar development. Nature 393(6685):595-599.
20. Berson JF, et al. (1996) A seven-transmembrane domain receptor involved in fusion and entry of T-cell-tropic human immunodeficiency virus type 1 strains. Journal of Virology 70(9):6288-6295.
21. Feng Y, Broder CC, Kennedy PE, & Berger EA (1996) HIV-1 entry cofactor: functional cDNA cloning of a seven-transmembrane, G protein-coupled receptor. Science 272(5263):872-877.
22. Symington LS & Gautier J (2011) Double-strand break end resection and repair pathway choice. Annual Review of Genetics 45:247-271.
23. Guschin DY, et al. (2010) A rapid and general assay for monitoring endogenous gene modification. Methods in Molecular Biology 649:247-256.
24. Vahedi G, et al. (2013) Helper T-cell identity and evolution of differential transcriptomes and epigenomes. Immunological Reviews 252(1):24-40.
25. Farh KK, et al. (2015) Genetic and epigenetic fine mapping of causal autoimmune disease variants. Nature 518(7539):337-343.

The invention is further defined by the following embodiments:
1. A method of editing the genome of a cell, wherein the cell is a primary hematopoietic cell or a primary hematopoietic stem cell, the method comprising:
   a) providing a reaction mixture comprising a Cas9 ribonucleoprotein complex and the cell, wherein the Cas9 ribonucleoprotein complex comprises a Cas9 nuclease domain and a guide RNA, wherein the guide RNA specifically hybridizes to a target region of the genome of the cell; and
   b) introducing the Cas9 ribonucleoprotein complex inside the cell.
2. The method of embodiment 1, wherein the method provides an efficiency of genome editing of at least about 20%.
3. The method of embodiment 1, wherein the cell does not contain a nucleic acid encoding the Cas9 and/or a DNA nucleic acid encoding a guide RNA.
4. The method of embodiment 1, wherein prior to the providing of a) the cell is not immortalized or transformed.
5. The method of embodiment 4, wherein after the introducing of b) the cell is not immortalized or transformed.
6. The method of embodiment 1, wherein the cell has not been passaged prior to the providing of a).
7. The method of embodiment 6, wherein prior to the providing of a), the cell has been directly isolated from a host organism or tissue and cultured.
8. The method of embodiment 6, wherein prior to the providing of a), the cell has been directly isolated from a host organism or tissue and has not been cultured.
9. The method of embodiment 1, wherein the introducing comprises electroporation.
10. The method of embodiment 1, wherein the introducing comprises:
   coating a nanowire or nanotube with the Cas9 ribonucleoprotein complex;
   contacting the cell with the nanowire or nanotube coated with the Cas9 ribonucleoprotein complex; and
   piercing a cell membrane of the cell with the nanowire or nanotube coated with the Cas9 ribonucleoprotein complex.
11. The method of embodiment 1, wherein the introducing comprises:
   forcing the reaction mixture through a cell deforming constriction that is smaller than the diameter of the cell, wherein the forcing introduces transient pores into a cell membrane of the cell; and
   allowing the Cas9 ribonucleoprotein complex to enter the cell through the transient pores.
12. The method of embodiment 1, wherein the Cas9 ribonucleoprotein complex comprises a ligand for an extracellular receptor on the cell, and the introducing comprises receptor mediated internalization of the Cas9 ribonucleoprotein complex.
13. The method of embodiment 1, wherein the Cas9 ribonucleoprotein complex comprises a cell penetrating peptide, and the introducing comprises contacting the cell penetrating peptide to the cell.
14. The method of embodiment 9, wherein the electroporation comprises positioning the reaction mixture into a chamber between a cathode and an anode, and applying a voltage potential between the cathode and the anode of from about 20 kV/m to about 100 kV/m.
15. The method of embodiment 14, wherein the voltage potential is applied as a pulse having a length of from about 5 ms to about 100 ms.
16. The method of embodiment 15, wherein the method further comprises repeating the application of the voltage potential pulse from 2 to 10 times.
17. The method of embodiment 14, wherein:
   the chamber is a hollow member having a longitudinal length and a horizontal cross sectional area;
   the chamber comprises a first and second distal end separated by the longitudinal length; and
   the chamber has:
      a first electrode at the first distal end; and
      a reservoir containing an electrolytic solution in fluid communication with the second distal end of the chamber, said reservoir having a second electrode.
18. The method of embodiment 17, wherein the chamber has a ratio of longitudinal length to horizontal cross-sectional area in the range of 50 to 10,000.
19. The method of embodiment 1, wherein the Cas9 ribonucleoprotein complex in the reaction mixture is at a concentration of from about 0.25 µM to about 5 µM.
20. The method of embodiment 1, wherein the Cas9 ribonucleoprotein complex in the reaction mixture is at a concentration of from about 0.9 µM to about 1.8 µM.
21. The method of embodiment 1, wherein the reaction mixture contains from about 1 x 10⁵ to about 4 x 10⁵ primary hematopoietic cells or primary hematopoietic stem cells.
22. The method of embodiment 1, wherein the reaction mixture contains from about 2 x 10⁵ to about 2.5 x 10⁵ primary hematopoietic cells or primary hematopoietic stem cells.
23. The method of embodiment 1, wherein the cell is a primary hematopoietic cell.
24. The method of embodiment 23, wherein the primary hematopoietic cell is an immune cell.
25. The method of embodiment 24, wherein the immune cell is a T cell.
26. The method of embodiment 25, wherein the T cell is a regulatory T cell, an effector T cell, or a naive T cell.
27. The method of embodiment 26, wherein the regulatory T cell, effector T cell, or naive T cell is a CD4⁺ T cell.
28. The method of embodiment 25, wherein the T cell is a CD4⁺CD25^{hi}CD127^{lo} regulatory T cell.
29. The method of embodiment 25, wherein the T cell is a FOXP3⁺ T cell.
30. The method of embodiment 25, wherein the T cell is a CD4⁺CD25^{lo}CD127^{hi} effector T cell.
31. The method of embodiment 25, wherein the T cell is a CD4⁺CD25^{lo}CD 127^{hi}CD45RA^{hi}CD45RO⁻ naive T cell.
32. The method of embodiment 25, wherein the T cell is a CD8⁺ T cell.
33. The method of embodiment 25, wherein the T cell is a CD4⁺CD8⁺ T cell.
34. The method of embodiment 25, wherein prior to the providing of a), the T cell is pre-activated.
35. The method of embodiment 25, wherein prior to the providing of a), the T cell is unstimulated.
36. The method of embodiment 25, wherein the T cell comprises a recombinant antigen receptor.
37. The method of embodiment 1, wherein the reaction mixture further comprises a single-stranded oligonucleotide DNA template, and wherein the method comprises introducing the single-stranded oligonucleotide DNA template inside the cell.
38. The method of embodiment 37, wherein the single-stranded oligonucleotide DNA template is at a concentration of from about 9 µM to about 180 µM.
39. The method of embodiment 37, wherein the single-stranded oligonucleotide DNA template is at a concentration of about 45 µM.
40. The method of embodiment any one of 37-39, wherein the method provides an efficiency of primary hematopoietic cell or primary hematopoietic stem cell genome editing of at least about 30%.
41. The method of any one of embodiment 37-39, wherein the method provides an efficiency of primary hematopoietic cell or primary hematopoietic stem cell template directed genome editing of at least about 10%, or at least about 14%.
42. The method of any one of embodiment 37-39, wherein the single stranded oligonucleotide DNA template encodes a recombinant antigen receptor, a portion thereof, or a component thereof.
43. The method of any one of embodiment 1 to 41, wherein the cell is a T cell, and the method further comprises:
   c) after the introducing of b), transferring the reaction mixture to a culture medium containing a CD3 agonist and a CD28 agonist and culturing the cells.
44. The method of embodiment 43, wherein the CD3 agonist and the CD28 agonist are immobilized on a solid surface.
45. The method of embodiment 43, wherein the CD3 agonist is an anti-CD3 antibody.
46. The method of embodiment 43, wherein the CD28 agonist is an anti-CD28 antibody.
47. The method of embodiment 43, wherein the method further comprises:
   c) after the culturing of c), transferring the reaction mixture to a culture medium that does not contain a CD3 agonist or a CD28 agonist and culturing the cells.
48. The method of embodiment 1, wherein the Cas9 ribonucleoprotein complex comprises a Cas9 nuclease.
49. The method of embodiment 1, wherein the Cas9 ribonucleoprotein complex comprises a Cas9 nickase.
50. The method of embodiment 1, wherein the Cas9 ribonucleoprotein complex comprises a Cas9 nuclease domain fused to a restriction endonuclease or nickase.
51. The method of embodiment 1, wherein the Cas9 ribonucleoprotein complex comprises a Cas9 nuclease domain fused to a transcriptional modulator or a chromatin modifier.
52. The method of embodiment 1, wherein the reaction mixture comprises at least two structurally different Cas9 ribnucleoprotein complexes.
53. The method of embodiment 52, wherein the at least two structurally different Cas9 ribonucleoprotein complexes contain structurally different sgRNAs.
54. The method of embodiment 52, wherein the at least two structurally different Cas9 ribonucleoprotein complexes contain structurally different Cas9 domains.
55. A method of editing the genome of a cell, wherein the cell is a primary hematopoietic cell or a primary hematopoietic stem cell, the method comprising:
   a) providing a reaction mixture comprising a Cas9 nuclease domain and the cell; and
   b) introducing the Cas9 nuclease domain inside the cell, wherein the Cas9 nuclease domain forms a complex with a guide RNA inside the cell.
56. The method of embodiment 55, wherein the guide RNA inside the cell is encoded by a guide RNA gene inside the cell, wherein the guide RNA gene comprises DNA.
57. The method of embodiment 55 or 56, wherein the cell does not contain a nucleic acid encoding the Cas9 nuclease domain.
58. The method of embodiment 55, wherein the efficiency of Cas9 delivery is at least about 20% or 30%.
59. A plurality of primary hematopoietic cells or primary hematopoietic stem cells, wherein the plurality of cells do not contain a nucleic acid encoding Cas9 and/or a DNA nucleic acid encoding a guide RNA, and wherein at least 20% of the plurality of cells contains a Cas9 ribonucleoprotein complex.
60. The plurality of cells of embodiment 59, wherein at least 30% of the plurality of cells contains a Cas9 ribonucleoprotein complex.
61. The plurality of cells of embodiment 59, wherein at least 20% of the plurality of cells contains a Cas9 ribonucleoprotein complex and a single stranded oligonucleotide DNA template.
62. The plurality of embodiment cells of 59, wherein the plurality of cells have not enriched for the presence of the Cas9 ribonucleoprotein complex.
63. The plurality of cells of embodiment 59, wherein at least 20% or 30% of the plurality of cells contains a double stranded break, or an NHEJ or HDR repaired double stranded break at a target genomic region.

## Claims

1. A plurality of primary T cells, wherein the plurality of primary T cells do not contain a nucleic acid encoding Cas9 and/or a DNA nucleic acid encoding a guide RNA, and wherein at least 20% of the plurality of cells contains a Cas9 ribonucleoprotein complex.

2. The plurality of cells of claim 1, wherein at least 20% of the plurality of cells contains a Cas9 ribonucleoprotein complex and a single stranded oligonucleotide DNA template.

3. The plurality of cells of claim 1, wherein the plurality of cells have not enriched for the presence of the Cas9 ribonucleoprotein complex.

4. The plurality of cells of claim 1, wherein at least 20% of the plurality of cells contains a double stranded break, or an NHEJ or HDR repaired double stranded break at a target genomic region.

5. The plurality of cells of claim 1, wherein the primary T cells are regulatory T cells, effector T cells, or naïve T cells.

6. The plurality of cells of claim 5, wherein the primary T cells are regulatory T cells, effector T cells, or naïve T cells are CD4+ T cells.

7. The plurality of cells of claim 6, wherein the primary T cells are CD4⁺CD2S^{hi}CD127^{lo} regulatory T cells.

8. The plurality of cells of claim 7, wherein the primary T cells are CD4⁺CD25^{lo}CD127^{hi}CD45RA^{hi}CD45RO⁻ naïve T cells.

9. The plurality of cells of claim 1, wherein the primary T cells are FOXP3⁺ T cells.

10. The plurality of cells of claim 1, wherein the primary T cells are CD8+ cells.

11. The plurality of cells of claim 1, wherein the primary T cells are CD4+CD8+ cells.

12. The plurality of cells of claim 1, wherein the primary T cells express a heterologous protein.

13. The plurality of cells of claim 12, wherein the heterologous protein is a chimeric antigen receptor.
